# EUROPEAN PATENT SPECIFICATION

(11) **EP 1 311 488 B1**
(45) Date of publication and mention of the grant of the patent: **04.11.2009**
(21) Application number: 01954264.6
(22) Date of filing: 08.08.2001
(51) Int. Cl.: C07D 233/64, A61K 31/4172, A61P 7/02

(54) **SUBSTITUTED IMIDAZOLES AS TAFIA INHIBITORS**
SUBSTITUTUIERTE IMIDAZOLE ALS TAFIA INHIBITOREN
IMIDAZOLES SUBSTITUES UTILISES COMME INHIBITEURS TAFIA

(30) Priority: 17.08.2000 GB 0020346; 09.11.2000 GB 0027409; 04.12.2000 GB 0029556
(43) Date of publication of application: 21.05.2003
(73) Proprietor: Pfizer Limited, Sandwich, Kent CT13 9NJ (GB)
(72) Inventor: ALLERTON, Charlotte Moira Norfor, Sandwich, Kent CT13 9NJ (GB); BLAGG, Julian, Sandwich, Kent CT13 9NJ (GB); BUNNAGE, Mark Edward, Sandwich, Kent CT13 9NJ (GB); STEELE, John, Sandwich, Kent CT13 9NJ (GB)
(74) Representative: Ruddock, Keith Stephen
(86) International application number: PCT/IB2001/001425
(87) International publication number: WO 2002/014285

(56) References cited:
- EP-A- 0 175 180
- WO-A-00/66550
- US-A- 5 993 815
- M. B. BOFFA ET AL.: "PLASMA AND RECOMBINANT THROMBIN-ACTIVATABLE FIBRINOLYSIS INHIBITOR (TAFI) AND ACTIVATED TAFI COMPARED WITH RESPECT TO GLYCOSYLATION, THROMBIN/THROMBOMODULIN-DEPENDENT ACTIVATION, THERMAL STABILITY, AND ENZYMATIC PROPERTIES" THE JOURNAL OF BIOLOGICAL CHEMISTRY, vol. 4, no. 273, 1998, pages 2127-2135, XP002183690 cited in the application

## Description

The present invention describes a series of substituted imidazoles as TAFIa inhibitors, useful in the treatment of disease.

Thrombin Activatable Fibrinolysis Inhibitor, TAFI, is a 60kDa glycoprotein found in human plasma. It is also known as procarboxypeptidase B, carboxypeptidase B, plasma carboxypeptidase B, carboxypeptidase U and carboxypeptidase R. It plays an intrinsic part in the blood coagulation process, during which it is transformed into an activated form, TAFIa, whereupon it acts upon the fibrin matrix which comprises a developing blood clot to prevent its dissolution. Imbalances in the blood coagulation process are thought to be the origin of a large and disparate number of disease conditions, which are linked by an unwanted build up of fibrin. The scale of fibrin build up is determined by the delicate equilibrium between two biochemical cascades in the human body; the coagulation and fibrinolysis cascades. These cascades are an integral part of maintaining hemostasis.

To maintain hemostasis in the blood, mammals have developed mechanisms to repair the body in the event of vascular injury. The injured blood vessel will constrict to reduce the blood flow to the area. Platelets will aggregate to reduce the loss of blood from the area, followed by fibrinogen which will polymerise and form a fibrin clot. This clot will cover the area of vascular damage, preventing blood loss. Once the blood vessel has been repaired the clot will then dissolve. The coagulation cascade is responsible for the forming of a clot; the fibrinolysis cascade is responsible for the dissolution of the clot.

Studies have shown that these two processes are intrinsically linked through the generation of α-thrombin. α-Thrombin is the final product of the blood coagulation cascade and is responsible for the conversion of soluble plasma fibrinogen to an insoluble fibrin matrix. Polymerised fibrin provides a haemostatic plug which prevents blood loss from the site of vascular injury and provides a provisional matrix which enhances the subsequent repair process. In addition to mediating coagulation, α-thrombin also reduces the rate at which blood clots are broken down by the serine protease plasmin. The anti-fibrinolytic activity of α-thrombin results from its activation of TAFI. TAFI circulates in normal plasma at a concentration of about 75nM in an inactive form. Thrombin converts the inactive zymogen to the active TAFI (TAFIa), a reaction that is augmented about 1250-fold by thrombomodulin. Once activated, TAFIa cleaves both C-terminal arginine and lysine residues from the developing fibrin clot. The removal of di-basic amino acids from the surface of the fibrin matrix attenuates clot lysis by inhibiting the binding of the key mediators of fibrinolysis: tissue plasminogen activator (tPA) and its substrate, plasminogen, which is the precursor of plasmin. Both tPA and plasminogen contain a structural motif called a kringle domain which binds tightly to C-terminal lysine residues. The removal of these binding sites prevents the formation of a ternary complex between tPA, plasminogen and fibrin and this inhibits the conversion of plasminogen to plasmin, thus protecting the clot from rapid degradation.

It can be seen that if the equilibrium between coagulation and fibrinolysis is in favour of coagulation, then there will be a larger amount of fibrin present than normal. This makes it more likely that the subject will develop one or more of the conditions in which thrombus build up is implicated. By the use of a TAFIa inhibitor, TAFIa will not be able to act upon a developing fibrin clot as described above to inhibit fibrinolysis of the clot. Thus a TAFIa inibitor should serve to enhance the fibrinolysis cascade.

The use of TAFI inhibitors to treat certain conditions is known in the art. Whilst the use of TAFIa inhibitors to treat such conditions is unknown, certain weak, non-specific TAFIa inhibitors have been identified.

USA 5993815 teaches the use of a peptide that binds to the TAFI zymogen, inhibiting activation of the TAFI zymogen, to treat those disorders where a C-terminal lysine or arginine is cleaved from an intact peptide. Suitable disorders are arthritis, sepsis, thrombosis, strokes, deep vein thrombosis and myocardial infarctions. The peptide used is an antibody or a functionally active fragment. The peptide should be used in an amount to promote fibrinolysis *in vivo*.

McKay et al, Biochemistry, 1978, 17, 401, discloses the testing of a number of compounds as competitive inhibitors of bovine carboxypeptidase B of pancreatic origin. Inhibition was measured by the inhibitor's efficiency in protecting the active centre tyrosine and glutamic acid of bovine carboxypeptidase B from irreversible alkylation by bromoacetyl-D-arginine or bromoacetamidobutylguanidine. It is suggested that such inhibitors could act as bradykinin potentiators.

Bovine enzymes of pancreatic origin are very different to those found in human plasma, so one would not expect inhibitors of one to inhibit the other. Moreover, such inhibitors are directed towards a very different utility. Accordingly the above reference contains no teaching of TAFIa inhibitors or their utility.

Redlitz et al, J. Clin. Invest. 1995, 96, 2534, teaches the involvement of plasma carboxypeptidase B (pCPB, or TAFI) in the formation of clots. The lysis of blood clots was followed in the absence and presence of pCPB, whereupon it was found that the presence of pCPB slowed clot lysis. To confirm that pCPB was responsible two control reactions were run; one where the lysis experiment was repeated in the presence of pCPB and a carboxypeptidase inhibitor, PCI, a second where the lysis reaction was conducted in the presence of plasma from which pCPB was removed. In both cases lysis proceeded uninhibited.

Boffa et al, J. Biol. Chem. 1998, 273, 2127, compares plasma and recombinant TAFI and TAFIa with respect to glycosylation, activation, thermal stability and enzymatic properties. Inhibition constants for three competitive inhibitors were determined: ε-aminocaproic acid (ε-ACA), 2-guanidinoethylmercaptosuccinic acid (GEMSA) and potato carboxypeptidase inhibitor (PCI).

There are large numbers of carboxypeptidases, characterised by cleaving the C-terminal amino acid from a peptide. They may be divided into acidic, neutral or basic, depending on the type of amino acid they cleave. Basic carboxypeptidases cleave arginine, lysine and histidine. TAFIa is a specific subset of basic carboxypeptidases. In terms of the present invention the inhibitors disclosed above by Redlitz et al and Boffa et al, are too weak, non-specific or otherwise unsuitable to be considered as suitable TAFIa inhibitors for therapeutic application. Further, whilst the role of TAFIa in clot lysis is explained, there is no suggestion that TAFIa inhibitors can be used to treat disease.

WO00/66550 discusses a broad class of compounds useful as inhibitors of carboxypeptidase U. Inhibitors of carboxypeptidase U are postulated to facilitate fibrinolysis and thus the compounds are taught as useful in the treatment of thrombotic conditions. There is no data to support this assertion, though details of a suitable assay are given.

WO00/66152 discloses formulations containing a carboxypeptidase U inhibitor and a thrombin inhibitor. Suitable carboxypeptidase U inhibitors are those of WO00/66550. The formulations are taught as primarily useful in treating thrombotic conditions.

The present invention discloses a class of TAFIa inhibitors. There are very great advantages in using a TAFIa inhibitor over a TAFI inhibitor. TAFI is activated to TAFIa by reaction with thrombin. A TAFI inhibitor must prevent these two large peptides coming together to react at the appropriate site. To date only large peptides have been described which can interfere with this reaction (USA-5993815). However, it has been discovered that the active site on TAFIa, responsible for reacting with a developing clot, is small, and thus can be blocked by a small molecule, one with a molecular weight of below 1000, preferably below 500. It is a great advantage to have a low molecular weight compound as the 'active' in a medicament. They are associated with oral bioavailability and patients usually prefer oral formulations. Further there is the potential for peptide therapeutics to induce an immune response. This is unlikely to be an issue with a small molecule. Small molecules are also generally more stable in plasma and thus have a greater duration of action. This is unlikely to be the case with large molecules, particularly peptides. For these reasons a TAFIa inhibitor is preferred. The invention provides a potent class of TAFIa inhibitors.

The present invention provides as a preferred set of TAFIa inhibitors, compounds of formula (I) 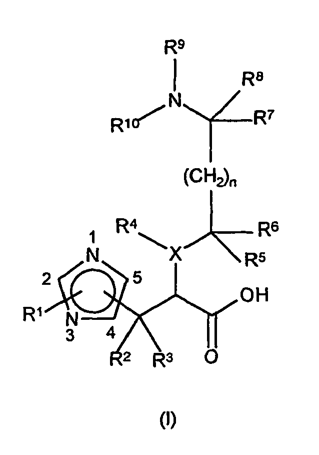 Where:
X is N or CH
n is 0 to 3
R¹ is:
   a) C₁₋₆ alkyl, straight chain or branched chain,
   b) C₁₋₆ alkenyl, straight chain or branched chain,
   c) C₁₋₆ alkynyl, straight chain or branched chain,
   d) Heterocycle,
   e) Aromatic heterocycle,
   f) Aryl;
   g) hydrogen;
   said groups (a), (b) and (c) optionally further substituted by: C₃₋₇ cycloalkyl, aryl, aromatic heterocycle, heterocycle, OR¹¹, NR¹¹R¹², S(O)ₚR¹¹, OC(O)R¹¹, CO₂R¹¹, CONR¹¹R¹², SO₂NR¹¹R¹², halo and NHSO₂R¹¹,
   where R¹ may be attached at any position on the imidazole ring.
R² and R³ are each independently selected from hydrogen, C₁₋₆ alkyl, optionally further substituted by OR¹¹, halo; or
   wherein R² and R³ may be joined to form a link, said link is C₂₋₆ alkylene.
R⁴ is hydrogen, C₁₋₆ alkyl, optionally further substituted by C₃₋₇ cycloalkyl, aryl, OR¹¹, halo and R¹¹; or
   wherein R⁴ and R¹⁰ may be joined to form a link, wherein said link is C₁₋₄ alkylene, optionally further substituted by OR¹¹, halo and R¹¹,
R⁵ and R⁶ are selected from:
   hydrogen, aryl, C₁₋₆ alkyl, said alkyl optionally further substituted by C₃₋₇ cycloalkyl, aromatic heterocycle, heterocycle, aryl, OR¹¹, R¹¹ and halo; or
   wherein R¹⁰ and either of R⁵ or R⁶ may be joined to form a link, wherein said link is a C₁₋₃ alkylene, optionally further substituted by OR¹¹, halo, R¹¹ and aryl; or
   wherein R⁵ and R⁶ may be joined to form a link, wherein said link is C₂₋₆ alkylene.
R⁷ and R⁸ are independently selected from:
   hydrogen, C₁₋₆ alkyl, optionally further substituted by OR¹¹, halo, aryl and R¹¹; or wherein R⁷ and R⁸ may be joined to form a link, wherein said link is C₂₋₆ alkylene.
R⁹ and R¹⁰ are independently selected from:
   Hydrogen, C(NR¹¹)NR¹¹R¹², C₁₋₆ alkyl, said alkyl optionally substituted by OR¹¹, halo, aryl and R¹¹; or
   wherein R⁹ and R¹⁰ may be joined to form a link, wherein said link is C₂₋₆ alkylene.
R¹¹ and R¹² are each independently selected from hydrogen or C₁₋₆ alkyl; or when forming a NR¹¹R¹² moiety, R¹¹ and R¹² may also be joined to form a link wherein said link is C₂₋₆ alkylene.
p is 0, 1 or 2
Wherein:
Aryl is defined as a 6-14 membered aromatic carbocycle, optionally further substituted by R¹¹, halo, OR¹¹, NR¹¹R¹², NR¹¹CO₂R¹², CO₂R¹¹, NR¹¹SO₂R¹², CN, haloalkyl, O(haloalkyl), S(O)ₚR¹¹, OC(O)R¹¹, SO₂NR¹¹R¹², C(O)NR¹¹R¹².

Aromatic heterocycle is defined as a 5 to 7 membered ring, containing from 1 to 3 heteroatoms, each independently selected from O, S and N, said heterocycle group optionally substituted by OR¹¹, NR¹¹R¹², CO₂R¹¹, NR¹¹CO₂R¹² R¹¹, halo, CN, haloalkyl, O(haloalkyl), S(O)ₚR¹¹, OC(O)R¹¹, NR¹¹SO₂R¹², SO₂NR¹¹R¹², C(O)NR¹¹R¹². Heterocycle is defined as a 3-8 membered ring containing from 1-3 heteroatoms, each independently selected from O, S and N, said ring being saturated or partially saturated, said heterocycle group optionally substituted by OR¹¹, NR¹¹R¹², CO₂R¹¹, NR¹¹CO₂R¹², R¹¹, halo, CN, haloalkyl, O(haloalkyl), S(O)ₚR¹¹, OC(O)R¹¹, NR₁₁SO₂R¹², SO₂NR¹¹R¹², C(O)NR¹¹R¹².

Compounds of formula (I) includes zwitterions, pharmaceutically acceptable salts, prodrugs, solvates and polymorphs thereof.

Halo includes fluoro, chloro, bromo and iodo groups.

Alkyl includes straight chain and branched chain.

A 6-14 membered aromatic carbocycle includes phenyl, naphthyl, indenyl, anthryl and phenanthryl.

A pharmaceutically acceptable salt of a compound of the formula (I) may be readily prepared by mixing together solutions of a compound of the formula (I) and the desired acid or base, as appropriate. The salt may precipitate from solution and be collected by filtration or may be recovered by evaporation of the solvent.

The pharmaceutically acceptable salts of the compounds of the formula (I) include the acid addition and the base salts thereof.

Suitable acid addition salts are formed from acids which form non-toxic salts and examples are the hydrochloride, hydrobromide, hydroiodide, sulfate, bisulfate, nitrate, phosphate, hydrogen phosphate, acetate, maleate, fumarate, lactate, tartrate, citrate, gluconate, succinate, saccharate, benzoate, methanesulphonate, ethanesulphonate, benzenesulphonate, p-toluenesulphonate and pamoate salts.

Suitable base salts are formed from bases which form non-toxic salts and examples are the sodium, potassium, aluminium, calcium, magnesium, zinc and diethanolamine salts.

For a review on suitable salts see Berge et al, J. Pharm. Sci., 1977, 66, 1.

The pharmaceutically acceptable solvates of the compounds of the formula (I) include the hydrates thereof.

Also included within the present scope of the compounds of the formula (I) are polymorphs thereof.

It will also be appreciated that the compounds of the invention will include prodrugs thereof: pharmaceutically acceptable derivatives of (I) in which the functional groups explicitly recited above have been derivatised to provide prodrugs which can be converted to the parent compound *in vivo*. Such prodrugs are discussed in Drugs of Today, 1983, 19, 499-538 and Annual Reports in Medicinal Chemistry, 1975, Vol. 10, Ch 31, 306-326. Suitable prodrugs will include compounds of formula (II) and (III). 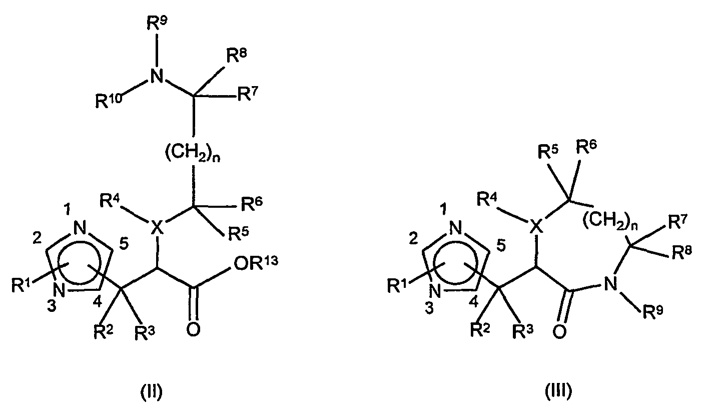 Wherein R¹, R², R³, R⁴, R⁵, R⁶, R⁷, R⁸, X and Z are as described above, R⁹ and R¹⁰ are as described above or in addition one or both groups may be a suitable nitrogen protecting group and R¹³ is an appropriate oxygen protecting group. Suitable nitrogen protecting groups include carbamates, particularly BOC and benzyl groups. Appropriate oxygen protecting groups are known to those skilled in the art and include allyl, aryl and alkyl groups, said alkyl group optionally substituted by aryl or C₃₋₇ cycloalkyl, or more specifically, such groups as benzyl, pivaloyloxymethyl (POM) and C₁₋₆ alkyl. Reference is also made herein to "Protective Groups in Organic Synthesis", 2nd edition, T. W. Greene and P. G. M. Wutz, Wiley-Interscience (1991).

Compounds of formula (I) contain one or more asymmetric carbon atoms and therefore exists in two or more stereoisomeric forms. Where compounds of formula (I) contain an alkenyl or alkenylene group, cis (E) and trans (Z) isomerism may also occur. The present invention includes the individual stereoisomers of the compounds of formula (I) and, where appropriate, the individual tautomeric forms thereof, together with mixtures thereof.

Preferred compounds of formula (I) include those which possess the stereochemistry shown below.

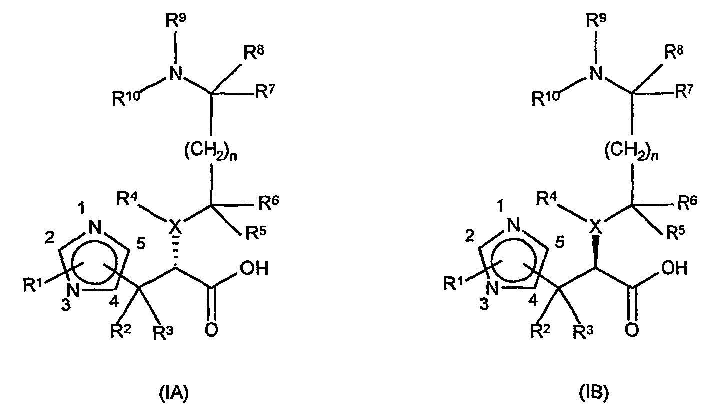

Those compounds of formula (IA) are particularly preferred.

Separation of diastereoisomers or cis and trans isomers may be achieved by conventional techniques, e.g. by fractional crystallisation, chromatography or H.P.L.C. of a stereoisomeric mixture of a compound of the formula (IA) or (IB) or a suitable salt or derivative thereof. An individual enantiomer of a compound of formulae (IA) or (IB) may also be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active acid or base, as appropriate. Reference is made herein to "Enantiomers, Racemates and Resolutions" J. Jacques and A. Collet, published by Wiley, NY, 1981; and "Handbook of Chiral Chemicals" chapter 8, Eds D. Ager and M. Dekker, ISBN:0-8247-1058-4.

Preferred compounds of formula (I) include those where the imidazole is substiuted at any position by R¹ and at the C2 or C4 positions by the amino acid fragment. Particularly preferred are those compounds of formula (I) where R¹ is attached to N1 of the imidazole moiety so as to give the (1,4)-disubstituted imidazole and compounds of formula (I) where R¹ is attached to C4 of the imidazole so as to give the (2,4)-disubstituted imidazole.

Preferably R¹ is an aryl group, a C₃₋₇ cycloalkyl group, a C₁₋₆ alkenyl group or a C₁₋₆ alkyl group, said alkyl or alkenyl groups optionally substituted by one or more groups selected from: a C₃₋₇ cycloalkyl group, heterocycle, aromatic heterocycle, OR¹¹, CO₂R¹¹, NR¹¹SO₂R¹², NR¹¹R¹², C(O)NR¹¹R¹², SO₂NR¹¹R¹², halo, OC(O)R¹¹, aryl or S(O)ₚR¹¹, where p is 0-2.
More preferably R¹ is an aryl group, C₁₋₆ alkenyl group or a C₁₋₆ alkyl group, wherein said alkyl is group is optionaly subsituted by one or more groups selected from CO₂R¹¹, OR¹¹, aryl, C₃₋₇ cycloalkyl, NHSO₂R¹¹, halo, aromatic heterocycle.
Yet more preferably R¹ is a CF₃ group or a C₁₋₆ alkyl group, wherein said alkyl is optionally substituted by a C₃₋₇ cycloalkyl group, aromatic heterocycle, OR¹¹, CO₂R¹¹, NR¹¹SO₂R¹² or aryl.
Even more preferably R¹ is C₁₋₆ alkyl, optionally substituted by a C₃₋₄ cycloalkyl group or aryl group.
Most preferably R¹ is C₁₋₃ alkyl R² and R³ are preferably independently selected from hydrogen and C₁₋₆ alkyl.

Most preferably R² and R³ are hydrogen.

R⁴ is preferably independently selected from hydrogen and C₁₋₆ alkyl, said alkyl optionally substituted by phenyl; or wherein R⁴ and R¹⁰ may be joined to form a link, said link is C₂₋₃ alkylene.
More preferably R⁴ is independently selected from hydrogen and C₁₋₃ alkyl; or wherein R⁴ and R¹⁰ may be joined to form a link, said link is C₂₋₃ alkylene.
Yet more preferably R⁴ is independently selected from hydrogen; or wherein R⁴ and R¹⁰ may be joined to form a link, said link is C₂₋₃ alkylene.
Most preferably R⁴ is hydrogen.

R⁵ and R⁶ are preferably independently selected from hydrogen and C₁₋₆ alkyl, said alkyl group optionally substituted by phenyl; or wherein R⁵ and R¹⁰ may be joined to form a link, said link is C₁₋₃ alkylene.
More preferably R⁵ and R⁶ are independently selected from hydrogen and C₁₋₃ alkyl, said alkyl group optionally substituted by phenyl; or wherein R⁵ and R¹⁰ may be joined to form a link, said link is C₂ alkylene.
Yet more preferably R⁵ and R⁶ are independently selected from hydrogen and C₁₋₃ alkyl.
Most preferably R⁵ and R⁶ are hydrogen.

Preferably R⁷ and R⁸ are independently selected from hydrogen and C₁₋₆ alkyl, said alkyl optionally substituted by phenyl.
More preferably R⁷ and R⁸ are independently selected from hydrogen and C₁₋₆ alkyl.
Yet more preferably R⁷ and R⁸ are independently selected from hydrogen and C₁₋₃ alkyl.
Even more preferably R⁷ and R⁸ are independently selected from hydrogen and CH₃. Most preferably R⁷ and R⁸ are hydrogen.

Preferably R⁹ and R¹⁰ are independently selected from hydrogen, C(NH)NH₂ and C₁₋₆ alkyl; or wherein R¹⁰ and R⁴ may be joined to form a link, said link is C₂₋₃ alkylene.
More preferably R⁹ and R¹⁰ are independently selected from hydrogen and C₁₋₃ alkyl; or wherein R¹⁰ and R⁴ may be joined to form a link, said link is C₂₋₃ alkylene.
Yet more preferably R⁹ and R¹⁰ are independently selected from hydrogen and C₁₋₃ alkyl.
Most preferably R⁹ and R¹⁰ are hydrogen.

Preferably R¹¹ and R¹² are independently selected from hydrogen and C₁₋₃ alkyl. More preferably R¹¹ and R¹² are independently selected from hydrogen and CH₃.

X is preferably CH.

n is preferably 0 or 1.
n is most preferably 0.
aryl is preferably phenyl, optionally substituted by 1-3 groups selected from: R¹¹, halo, OR¹¹, NR¹¹R¹², CO₂R¹¹, NHSO₂R¹¹, CN or haloalkyl.
Most preferably aryl is phenyl.

Preferably Aromatic heterocycle is defined as a 5 to 6 membered ring, containing from 1 to 3 heteroatoms, each independently selected from O, S and N, said heterocycle group optionally substituted by 1-3 groups selected from: OR¹¹, NR¹¹R¹², CO₂R¹¹, NR¹¹CO₂R¹², R¹¹, halo, CN, haloalkyl, O(haloalkyl), S(O)ₚR¹¹, OC(O)R¹¹, NR¹¹SO₂R¹², SO₂NR¹¹R¹², C(O)NR¹¹R¹².

More preferably Aromatic heterocycle is defined as a 5 to 6 membered ring, containing from 1 to 2 heteroatoms, each independently selected from O, S and N, said heterocycle group optionally substituted by 1-3 groups selected from: OR¹¹, NR¹¹R¹², CO₂R¹¹, NR¹¹CO₂R¹², R¹¹, halo, CN, haloalkyl, O(haloalkyl), S(O)ₚR¹¹, OC(O)R¹¹, NR¹¹SO₂R¹², SO₂NR¹¹R¹², C(O)NR¹¹R¹².

Most preferably Aromatic heterocycle is defined as a 5 to 6 membered ring, containing from 1 to 2 heteroatoms, each independently selected from O, S and N.

Preferably, Heterocycle is defined as a 3-8 membered ring containing from 1-2 heteroatoms, each independently selected from O, S and N, said ring being saturated or partially saturated, said heterocycle group optionally substituted by 1-3 groups selected from: OR¹¹, NR¹¹R¹², CO₂R¹¹, NR¹¹CO₂R¹², R¹¹, halo, CN, haloalkyl, O(haloalkyl), S(O)ₚR¹¹, OC(O)R¹¹, NR¹¹SO₂R¹², SO₂NR¹¹R¹², C(O)NR¹¹R¹².

More preferably, Heterocycle is defined as a 5-6 membered ring containing from 1-2 heteroatoms, each independently selected from O, S and N, said ring being saturated or partially saturated, said heterocycle group optionally substituted by 1-3 groups selected from: OR¹¹, NR¹¹R¹², CO₂R¹¹, NR¹¹CO₂R¹², R¹¹, halo, CN, haloalkyl, O(haloalkyl), S(O)ₚR¹¹, OC(O)R¹¹, NR¹¹SO₂R¹², SO₂NR¹¹R¹², C(O)NR¹¹R¹²_{.}

Most preferably, Heterocycle is defined as a 5-6 membered ring containing from 1-2 heteroatoms, each independently selected from O, S and N, said ring being saturated or partially saturated.

Preferred compounds of the present invention include:
(+)-5-Amino-2-[(1-*n*-propyl-1*H*-imidazol-4-yl)methyl]pentanoic acid (Example 2)
(+)-(2*S*)-5-Amino-2-[(1-*n*-butyl-1*H*-imidazol-4-yl)methyl]pentanoic acid (Example 5)
(+)-(2*S*)-5-Amino-2-[(1-*n*-propyl-1*H*-imidazol-4-yl)methyl]pentanoic acid (Example 7)
(+)-(2*S*)-5-Amino-2-(1*H*-imidazol-4-ylmethyl)pentanoic acid (Example 9)
(2*S*)-2-[(2-Aminoethyl)amino]-3-(1-*n*-propyl-1*H*-imidazol-4-yl)propanoic acid (Example 25)
(2*S*)-2-[(2-Aminoethyl)amino]-3-(1-*n*-butyl-1*H*-imidazol-4-yl)propanoic acid (Example 26)
(2*S*)-2-[(2-Aminoethyl)amino]-3-(1-*n*-isobutyl-1*H*-imidazol-4-yl)propanoic acid (Example 29)
(2*S*)-2-[(2-Aminoethyl)amino]-3-(1-*n*-isopentyl-1*H*-imidazol-4-yl)propanoic acid (Example 30)

Particularly preferred is (+)-(2*S*)-5-Amino-2-[(1-*n*-propyl-1*H*-imidazol-4-yl)methyl]pentanoic acid (Example 7)

The present invention also includes compounds of formula (XXIII) and (XXIV)

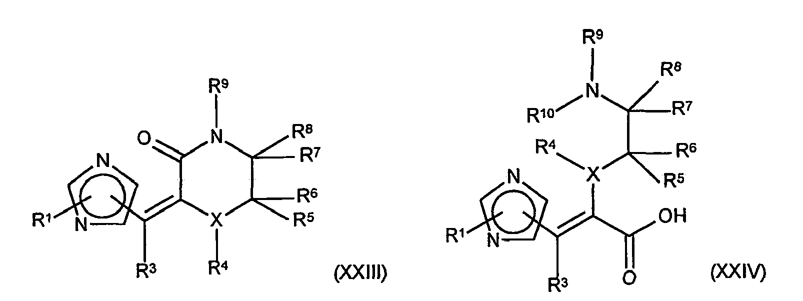

Where R¹, R³, R⁵, R⁶, R⁷, R⁸ and R¹⁰ are as described above, R⁴ is hydrogen, n is 0, X is CH and R⁹ is as described above or is an appropriate nitrogen protecting group. Appropriate nitrogen protecting groups include carbamates, particularly BOC and benzyl groups. These compounds are particularly useful as intermediates in the synthesis of compounds of formula (I)

The invention further provides Methods for the preparation of the compounds of the invention, which are described below and in the Examples and Preparations section. The skilled man will appreciate that the compounds of the invention could be made by methods other than those herein described, by adaptation of the methods herein described and/or adaptation of a plethora of methods known in the art. It is to be understood that the synthetic transformation methods specifically mentioned herein may be carried out in various different sequences in order that the desired substances can be efficiently assembled. The skilled chemist will exercise his judgement and skill as to the most efficient sequence of reactions for synthesis of a given target substance.

It will be apparent to those skilled in the art that sensitive functional groups may need to be protected and deprotected during synthesis of a substance of the invention. This may be achieved by conventional techniques, for example as described in "Protective Groups in Organic Synthesis" by T. W. Greene and P. G. M. Wuts, John Wiley and Sons Inc, 1991.

Compounds of formula (I) may be prepared by reacting a compound of formula (II)

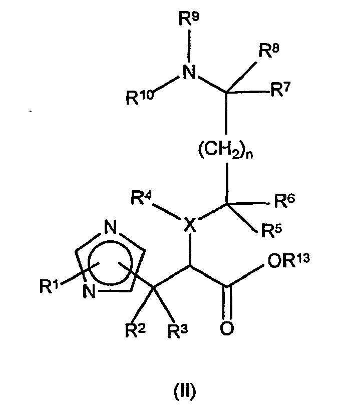

Wherein R¹, R², R³, R⁴, R⁵, R⁶, R⁷, R⁸ and X are as described above, R⁹, R¹⁰ are as described above, Additionally one or both may be a suitable nitrogen protecting group and R¹³ is an appropriate oxygen protecting group,
with a suitable reagent to remove said oxygen protecting group.

Appropriate oxygen protecting groups include allyl groups and alkyl groups, said alkyl groups optionally substituted by aryl groups.

Suitable reagents and conditions to remove said groups are well known to those skilled in the art and may include hydrolysis and hydrogenation.

Where R⁹ and/or R¹⁰ is a nitrogen protecting group, it may be necessary to remove said nitrogen protecting group after reaction of (II) with a suitable reagent to remove said oxygen protecting group. Suitable nitrogen protecting groups are well known to those skilled in the art, as are suitable conditions for their removal.

Compounds of formula (II), where R¹, R³, R⁴, R⁵, R⁶, R⁷, R⁸, R⁹, R¹⁰, R¹³ and X are as described above and R² is hydrogen may be prepared from compounds of formula (V) and (VI) in accordance with the following reaction scheme

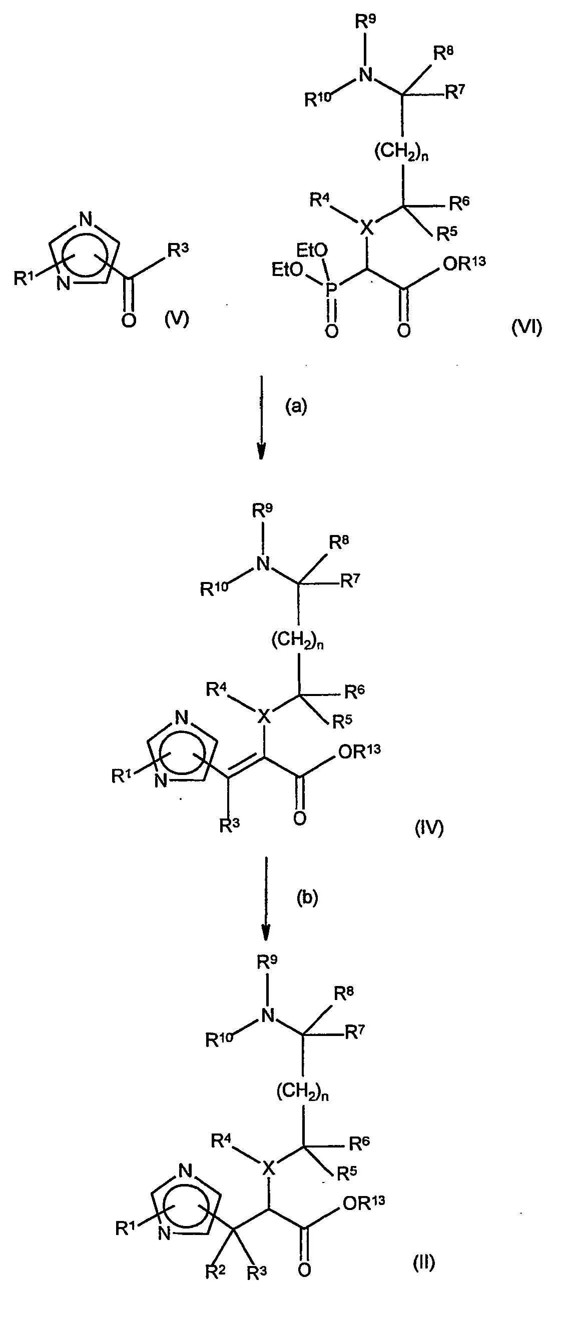

Compounds of formula (IV) may be formed by process step (a), a Wadsworth-Emmons reaction between compounds of formula (V) and (VI). This may be conducted under standard conditions, such as described in Org. Synth. Coll. Vol., 1988, 6, 358 and 1993, 8, 265. Suitable conditions include formation of the phosphonate anion with a suitable base such as NaH at 0°C, then reacting with 1 eq of the appropriate aldehyde at room temperature for 18 hours. A suitable solvent would be tetrahydrofuran.

Compounds of formula (II) may be formed by process step (b), a hydrogenation. This may be carried out by a method such as catalytic hydrogenation, e.g. 10% Pd/C at 4 atmospheres, in an alcoholic solvent (methanol or ethanol) at room temperature to 60°C for between 4 and 72 hours; or by activated metal hydride reduction, e.g. 30 eq NaBH₄, 1.5 to 2.5 eq CuCl, in methanol, at room temperature for 2 hours. The process may also be conducted to give an asymmetric hydrogenation of the alkene bond. Such methods are well known to those skilled in the art and are discussed in "Asymmetric Synthetic Methodology" chapter 9, Eds D. Ager and M. East, CRC Press, 1996, ISBN: 0-8493-8492-9.

Compounds of formula (V) are commercially available or may be prepared by a number of literature methods well known to a man skilled in the art. Reference is made to the preparations described herein and to G. Shapiro et al, Heterocycles, 1995, 41, 215; L. A. Reiter, J. Org. Chem., 1987, 52, 2714; B. H. Lipshutz et al, Tetrahedron Lett. 1986, 27, 4095; F. Aldebbagh et al, Tetrahedron Lett., 1997, 38 7937; and S. M. Abdelaal, J. Het. Chem. 1995, 32, 903.

Compounds of formula (VI) where R⁴, R⁵, R⁶, R⁷, R⁸, R⁹, R¹⁰ and R¹³ are as described above and X is CH, may be prepared in accordance with the following scheme.

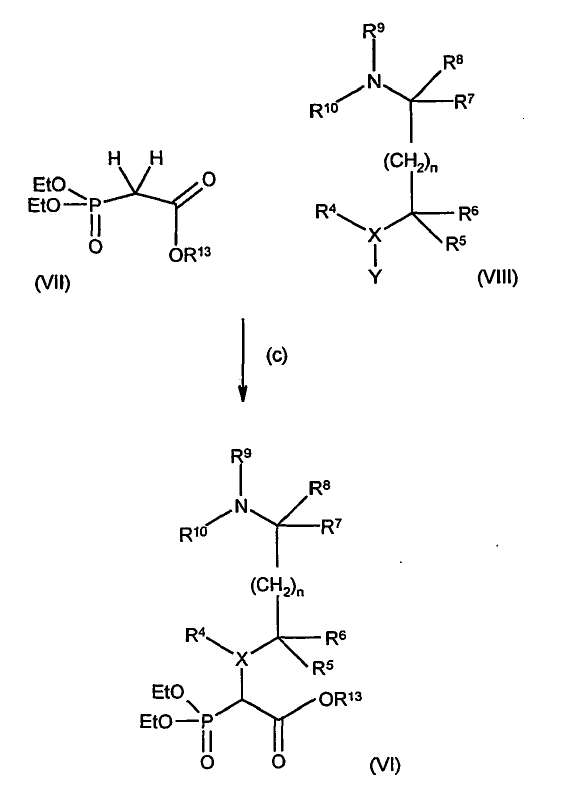

Compounds of formula (VI) may be prepared from the compounds of formula (VII) and (VIII) where Y is halo, under the conditions of process step (c), an alkylation reaction. This may be carried out under standard conditions, typically 1 eq of (VII) is treated with 1.1 eq of NaH, before reaction with (VIII), 18-crown-6 (cat) at reflux for 18 hours.

Compounds of formula (VI) where R⁵, R⁶, R⁷, R⁸, R⁹, R¹⁰ and R¹³ are as described above, R⁴ is a suitable nitrogen protecting group and X is N, may be prepared using the reaction scheme described above.

Compounds of formula (I) may also be prepared by treating a compound of formula (III) under the conditions of a lactam hydrolysis reaction. Suitable conditions include those of process step (d), a lactam hydrolysis. This may be conducted under standard conditions, typically basic conditions, e.g. aqueous LiOH in tetrahydrofuran at room temperature for 4-18 hours.

Compounds of formula (III) where R¹, R³, R⁴, R⁵, R⁶, R⁷, R⁸, R⁹, R¹³, X and Z are as described above and R² is hydrogen, may be prepared by the following process

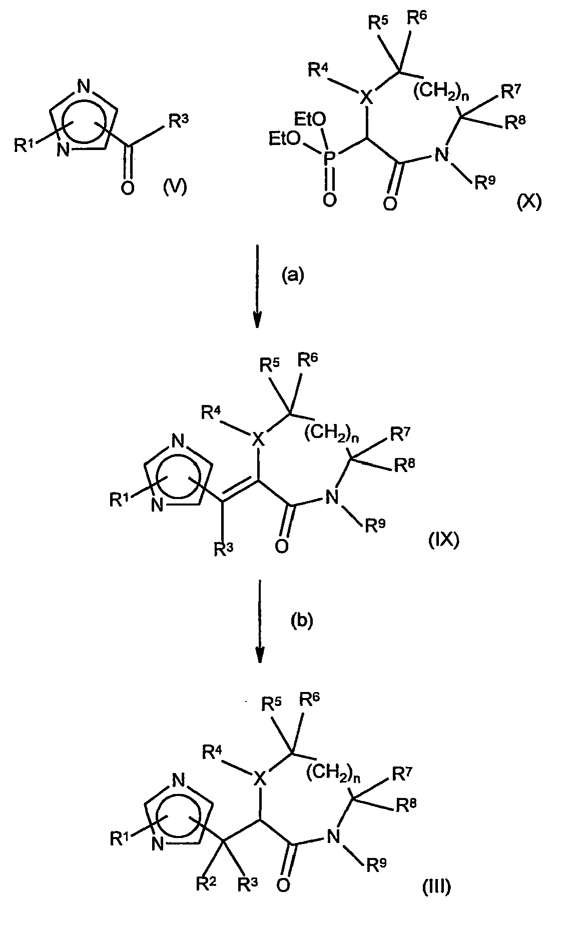

Compounds of formula (IX) may be prepared by reacting compounds of formula (V) and (X) under the conditions of process step (a) described above. Compounds of formula (III) may be prepared by reacting compounds of formula (IX) under the conditions of process step (b) described above.

Compounds of formula (X) where R⁴, R⁵, R⁶, R⁷, R⁸, R⁹, R¹⁰ and R¹³ are as described above, with the proviso R⁹ and R¹⁰ may not be linked and X is CH may be prepared from a compound of formula (XI) where Y is halo, in accordance with the following reaction scheme.

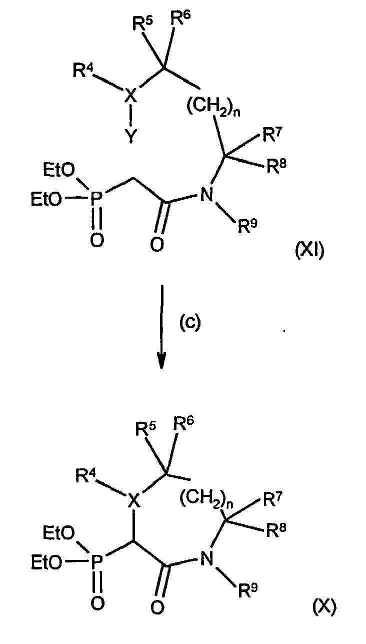

Compounds of formula (X) may be prepared from compounds of formula (XI) under the conditions of process step (c) described above.

Compounds of formula (II) where R¹, R², R³, R⁴, R⁶, R⁷, R⁸, R¹⁰ and R¹³ are as described above, R⁹ is as above or is a suitable nitrogen protecting group, X is N and R⁶ is hydrogen may be prepared from a compounds of formula (XII) and (XIII), in accordance with the following reaction scheme.

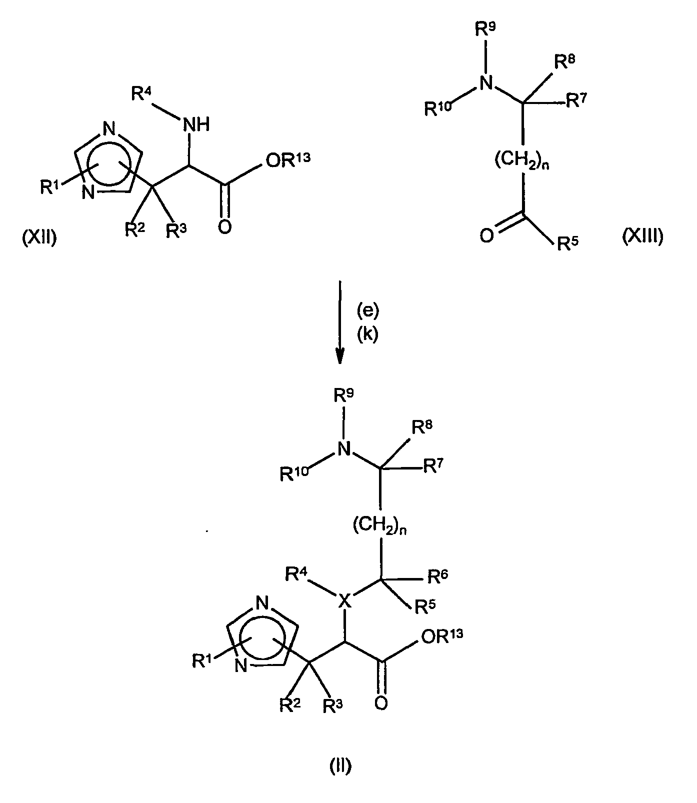

Compounds of formula (II) may be prepared by reacting compounds of formula (XII) and (XIII) under the conditions of process step (e), a reductive alkylation reaction, performed under standard conditions known to those skilled in the art. Suitable conditions would include reacting (XII) and (XIII) in the presence of sodium acetate and sodium cyanoborohydride.

Compounds of formula (II) wherein R⁹ is H may be obtained from compounds of formulae (II) where R⁹ is a suitable nitrogen protecting group by optional process step (k), removal of a nitrogen protecting group; appropriate conditions for the removal of nitrogen protecting groups P¹ are described in "Protective Groups in Organic Synthesis", 2nd edition, T. W. Greene and P. G. M. Wutz, Wiley-Interscience (1991). Appropriate conditions include:
BOC deprotection: 6N aqueous hydrochloric acid at room temperature to reflux temp, for between 1 and 3 hours;
Benzyl deprotection: dissolving metal reduction, e.g. Na, liq NH₃, -78°C.

Compounds of formula (XIII) are commercially available or may be prepared by methods well known to a man skilled in the art.

Compounds of formula (XII) above are commercially available. Alternatively where R¹, R³, R⁴ and R¹³ are as described above, and R² is hydrogen, they may be made by the route disclosed in Helv. Chim. Acta., 1994, 77, 1395 or as disclosed below.

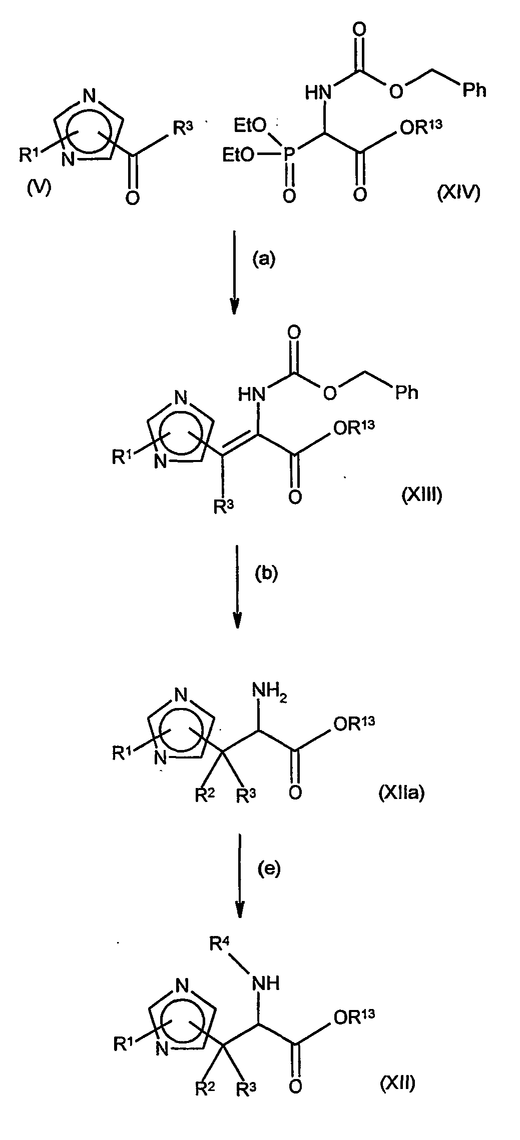

Compounds of formula (XII) may be prepared by reacting compounds of formula (V) and (XIV) under the conditions of process step (a), described above. Compounds of formula (XIIa) may be prepared by reacting compounds of formula (XIII) under the conditions of process step (b) described above. If a compound of formula (XII) is required where R⁴ is not hydrogen, then compounds of formula (XII) may be prepared by reacting compounds of formula (Xlla) under the conditions of process step (e), described above.

Compounds of formula (Xlla) where R¹, R² and R³, are as described above, with the proviso R² and R³ are not linked and R¹³ is methyl, may also be asymmetrically prepared from a compound of formula (XVI), where Y is halo, in accordance with the following reaction scheme.

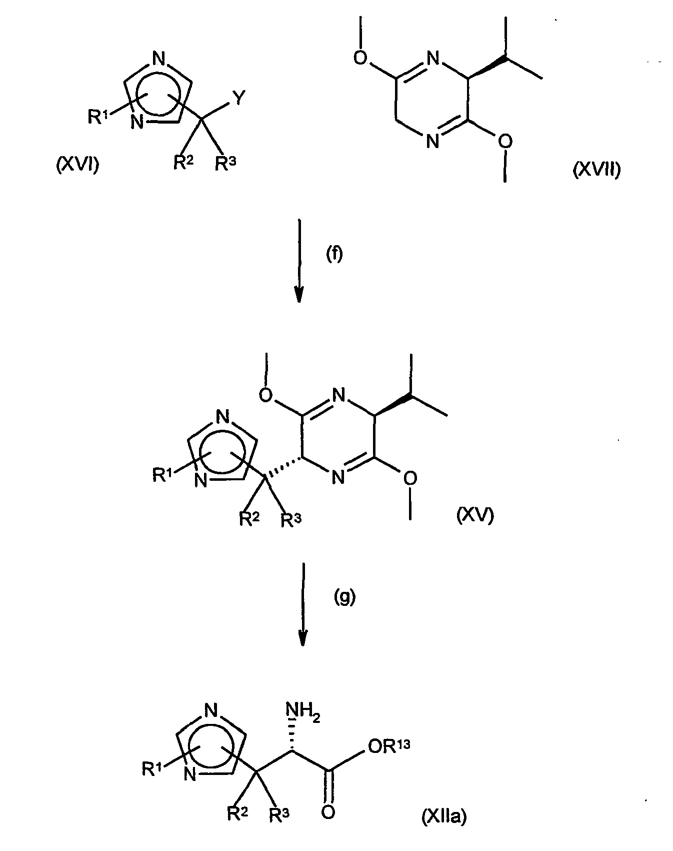

Compounds of formula (XV) may be prepared by reacting compounds of formula (XVII) and (XVI) under the conditions of process step (f) a Schollkopf asymmetric alkylation reaction, comprising reaction of a halide with a suitable deprotonated Schollkopf chiral auxiliary (Angew. Chem. Int. Ed. Engl., 1981, 20, 798). Suitable conditions are treating the Schollkopf auxiliary in tetrahydrofuran at -78°C with BuLi, followed by addition of (XVI) then 24 hours at room temperature. Compounds of formula (XIIa) may be prepared by reacting compounds of formula (XU) under the conditions of process step (g), a hydrolysis reaction, described in Angew. Chem. Int. Ed. Engl., 1981, 20, 798. Suitable conditions are 5eq of 0.25N aqueous hydrochloric acid at room temperature for 2 hours.

Compounds of formula (XII) may be obtained by methods well known to those skilled in the art or as exemplified in the examples. It should be noted that compounds of formula (XII) and intermediates thereto wherein R¹ is not H may be produced by coupling compounds of formula (XII) and intermediates thereto where R1 is H, with an appropriate reagent containing R¹, where R¹ is as disclosed above.

Compounds of formula (II) where R¹, R², R³, R⁴, R⁵, R⁶, R⁷, R⁸, R⁹, R¹⁰ and R¹³ are as described above and X is nitrogen may also be prepared from compounds of formula (XIX) and (XVIII) where Y is halo by the method described in the following reaction scheme.

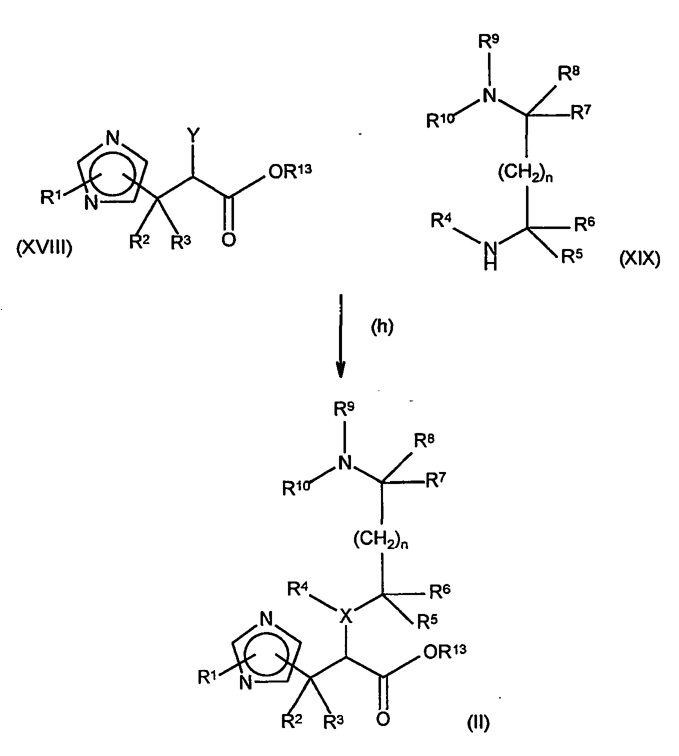

Compounds of formula (II) may be prepared by reacting compounds of formula (XVIII) and (XIX) under the conditions of process step (h) an alkylation reaction, reacting an excess of the amine with the halide. Suitable conditions are 6eq of (XIX) and 1eq of (XVIII) in acetonitrile at room temperature for 2 hours followed by 18 hours at reflux.

Compounds of formula (XIX) may be prepared by a number of literature routes, well known to a man skilled in the art, as well as being commercially available.

Compounds of formula (XX) where R¹, R², R³ and R¹³ are as described above, with the proviso R² and R³ are not linked, may be prepared by the method described in the following reaction scheme.

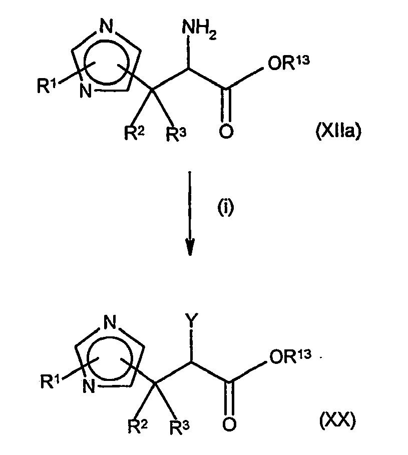

Compounds of formula (XX) may be prepared by reacting compounds of formula (XIIa) under the conditions of process step (i) a diazotisation/halogenation reaction, comprising conversion of the amine group to a diazo group, followed by reaction with a suitable halide, typically *in situ*. Suitable conditions are treating 1 eq of amine with 3.3 eq of NaNO₂ in concentrated hydrochloric acid:water (30:5) at -5°C, then 17 hours at room temperature.

Compounds of formulae (IA) and (IB) where R¹, R³, R⁵, R⁶, R⁷ and R⁸ are as described above, R², R⁴ and R¹⁰ are hydrogen, R⁹ is as described above or is a suitable nitrogen protecting group, n is 0 and X is CH may be prepared from compounds of formula (XXIII), both the E and Z isomers in accordance with the following scheme.

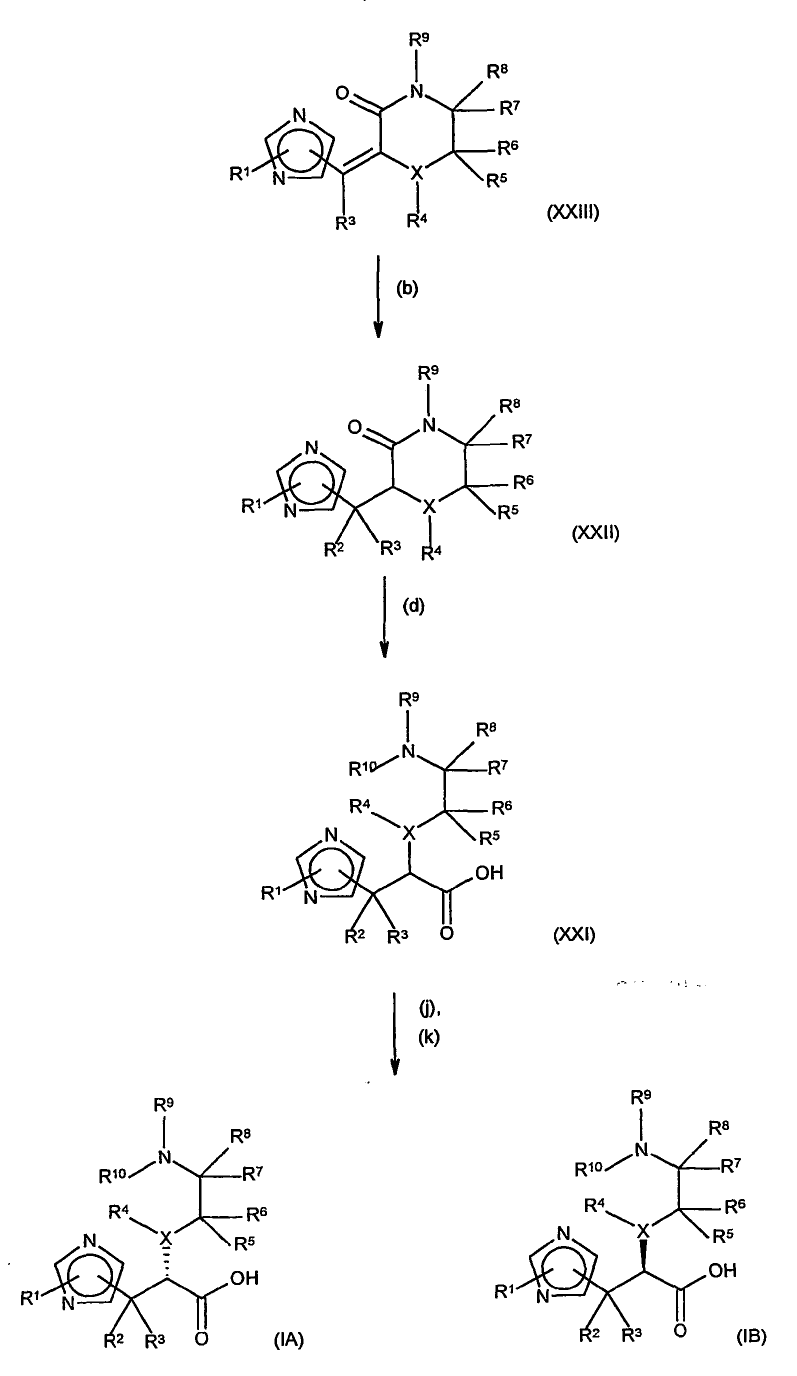

Compounds of formula (XXII) may be prepared from compounds of formula (XXIII), under the conditions of process step (b), as described above. Appropriate nitrogen protecting groups include carbamates, particularly BOC and benzyl groups. Process step (b) may also be conducted asymmetrically, using techniques known to those skilled in the art.

Compounds of formula (XXI) may be prepared from compounds of formula (XXII) under the conditions of process step (d), a lactam hydrolysis reaction which may be conducted under acidic or basic conditions as appropriate.

Compounds of formulae (IA) and (IB) may be prepared from compounds of formula (XXI) under the conditions of process step (j), resolution of the enantiomers, followed by optional process step (k), removal of the nitrogen protecting group when R⁹ is a nitrogen protecting group.

In process step (j), individual enantiomers of a compound of the formulae (IA) or (IB) may be prepared by resolution, such as by H.P.L.C. of the corresponding racemate using a suitable chiral support or by fractional crystallisation of the diastereoisomeric salts formed by reaction of the corresponding racemate with a suitable optically active acid or base, as appropriate. Reference is made herein to "Enantiomers, Racemates and Resolutions" J. Jacques and A. Collet, published by Wiley, NY, 1981; and "Handbook of Chiral Chemicals" chapter 8, Eds D. Ager and M. Dekker, ISBN:0-8247-1058-4.

Compounds of formulae (IA) or (IB) wherein R⁹ is H may be obtained from compounds of formulae (IA) or (IB) where R⁹ is a suitable nitrogen protecting group by optional process step (k), removal of a nitrogen protecting group; appropriate conditions for the removal of nitrogen protecting groups R⁹ are described in "Protective Groups in Organic Synthesis", 2nd edition, T. W. Greene and P. G. M. Wutz, Wiley-Interscience (1991). Appropriate conditions include:
BOC deprotection: 6N aqueous hydrochloric acid at room temperature to reflux temp, for between 1 and 3 hours;
Benzyl deprotection: dissolving metal reduction, e.g. Na, liq NH₃, -78°C.

Compounds of formulae (IA) and (IB) where R¹, R³, R⁵, R⁶, R⁷, R⁸ and X are as described above and R², R⁴, and R¹⁰ are hydrogen and R⁹ is as described above or is an appropriate nitrogen protecting group may also be prepared asymmetrically from compounds of formula (XXIII), where (XXIII) is either the E or Z isomer, in accordance with the reaction scheme shown below.

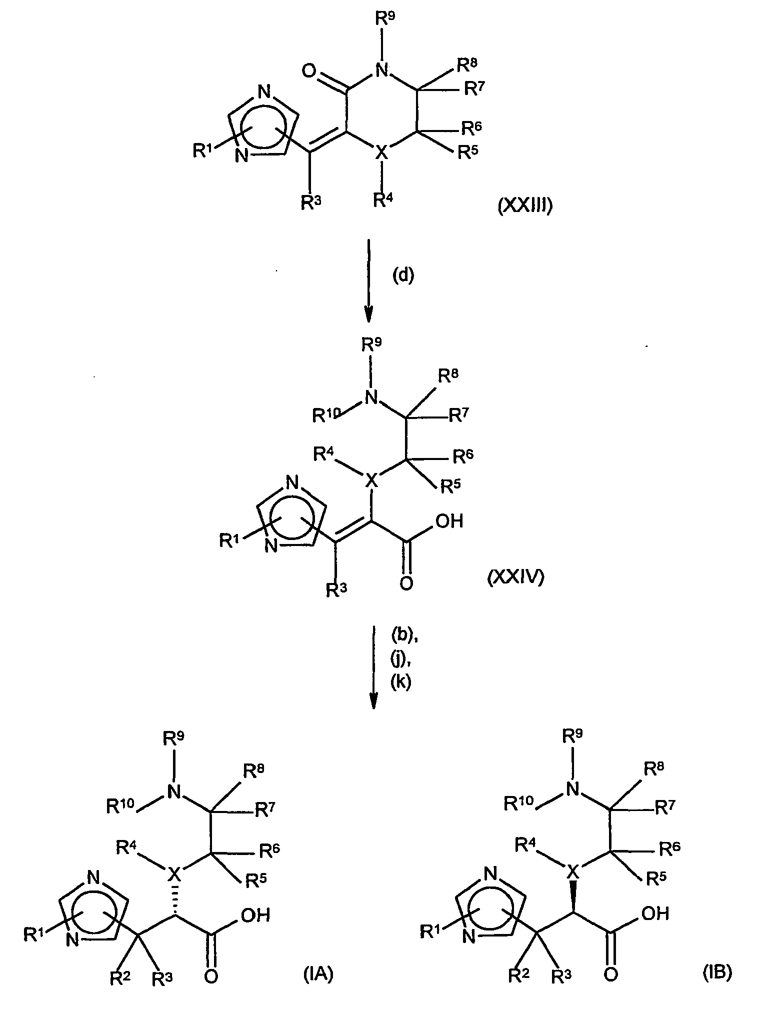

Compounds of formula (XXIV) may be prepared from compounds of formula (XXIII) under the conditions of process step (d), as described above.

Compounds of formula (IA) or (IB) may be prepared from compounds of formula (XXIV) under the conditions of process steps (b), a hydrogenation, (j), resolution of enantiomers and optionally, (k), removal of the nitrogen protecting group P¹ when R⁹ is a nitrogen protecting group. Process steps (b), (j) and (k) are described above.

In an alternative embodiment, compounds of formula (IA) where R¹, R³, R⁵, R⁶, R⁷, R⁸, R¹⁰ and X are as described above, R² and R⁴ are hydrogen and R⁹ is as described above or may be an appropriate nitrogen protecting group, may also be prepared asymmetrically from compounds of formula (XXIV), where (XXIV) is either the E or Z isomer, in accordance with the reaction scheme shown below.

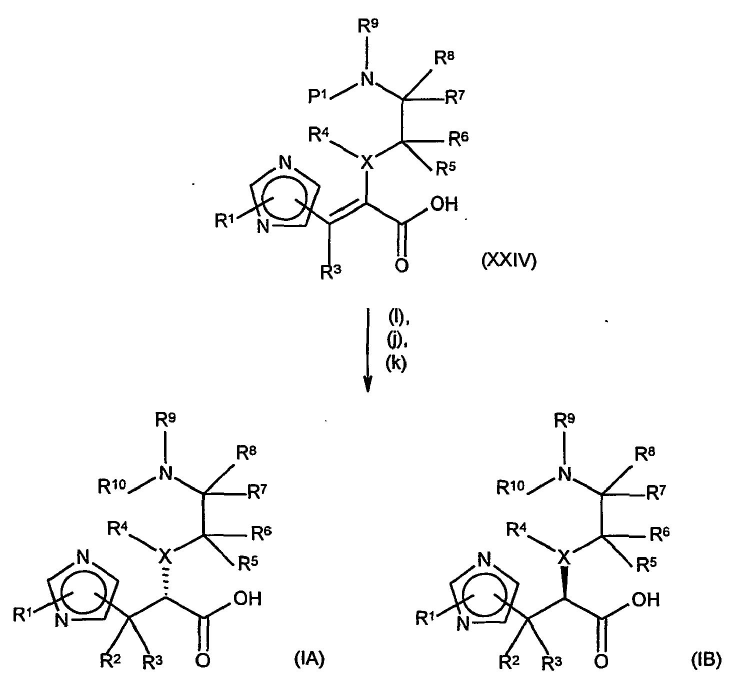

Compounds of formulae (IA) or (IB) may be prepared from compounds of formula (XXIV) under the conditions of process steps (I), an asymmetric hydrogenation, (j), resolution of the enantiomers and optionally (k), removal of the nitrogen protecting group when R⁹ is a nitrogen protecting group. Process step (j) is optional and is dependent upon the degree of enantiomeric selectivity obtained in step (I). Process step (j) may also be conducted *in situ* during process step (I). Process steps (j) and (k) are described above and are further exemplified in the examples.

The methods used to conduct process step (I) are well known to those skilled in the art and are discussed in "Asymmetric Synthetic Methodology" chapter 9, Eds D. Ager and M. East, CRC Press, 1996, ISBN: 0-8493-8492-9, as well as being further exemplified in the examples.

Compounds of formula (XXIII) where R¹, R³, R⁵, R⁶, R⁷, R⁸ and X are as described above and R⁹ is as described above or a suitable nitrogen protecting group may be prepared from compounds of formula (V) and (XXVI) in accordance with the reaction scheme below.

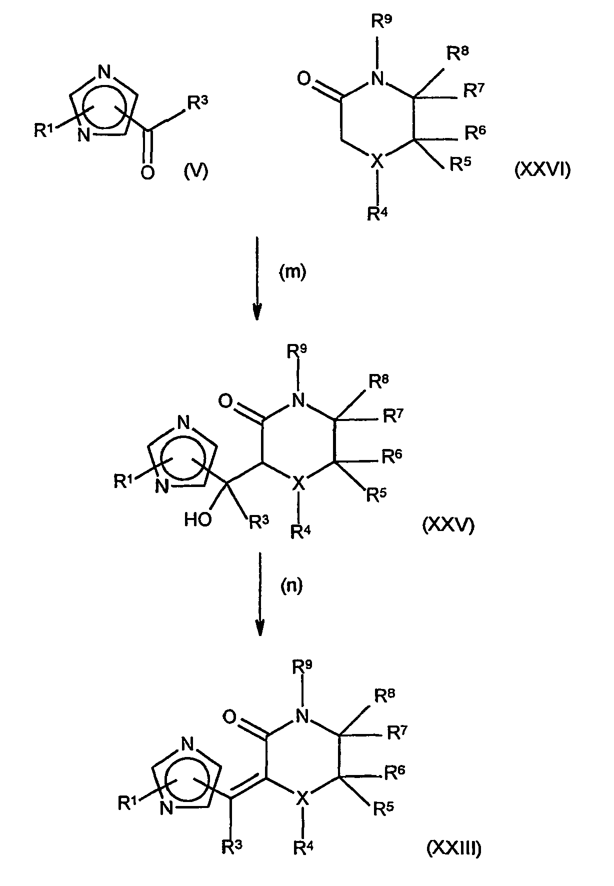

Compounds of formula (XXV) may be prepared from compounds of formula (V) and (XXVI) under the conditions of process step (m), an Aldol type reaction. Suitable conditions for such a reaction are well known to a man skilled in the art. Reference is also made herein to "Advanced Organic Chemistry" (4th Edition) by Jerry March, John Wiley and Sons Inc.

Compounds of formula (XXIII) may be prepared from compounds of formula (XXV) under the conditions of process step (n), an elimination reaction. (XXV) may be treated such that the hydroxy group is removed directly in a dehydration reaction, or it may be eliminated having first being transformed into a good leaving group such as a tosylate or mesylate group.

Compounds of formula (XXII) where R¹, R², R³ R⁴ R⁵, R⁶, R⁷, R⁸ and X are as disclosed above, R⁹ is as disclosed above or a nitrogen protecting group and n is 0 may also be prepared from compounds of formula (XXX) and (XXVI) in accordance with the scheme below.

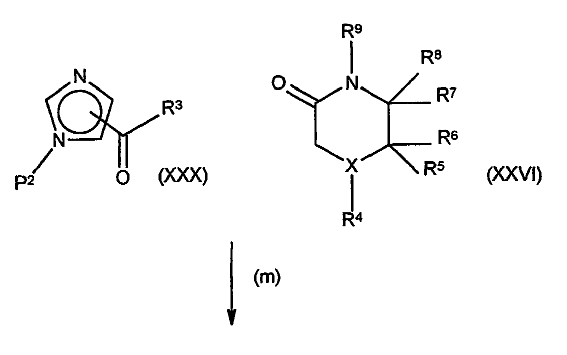

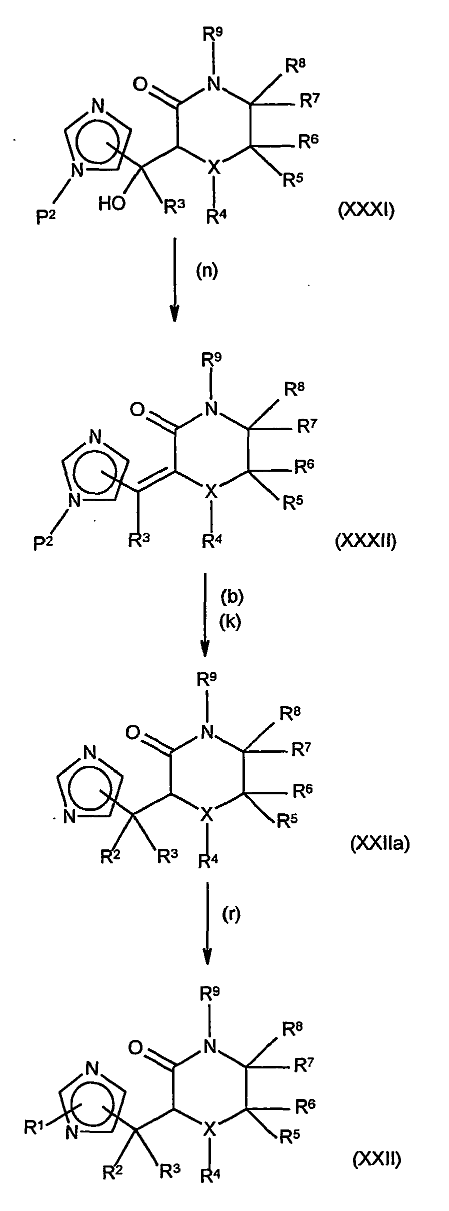

Compounds of formula (XXXI) may be prepared from compounds of formula (XXVI) and formula (XXX), wherein R³ is as described above and P² is a suitable nitrogen protecting group, under the conditions of process step (m) as described above. Compounds of formula (XXXII) may be prepared from compounds of formula (XXXI) under the conditions of process step (n) as described above.

Compounds of formula (XXlla) where R², R³ R⁴ R⁵, R⁶, R⁷, R⁸ and X are as disclosed above, R⁹ is as disclosed above or a nitrogen protecting group, n is 0 and R¹ is hydrogen may be prepared from compounds of formula (XXXII) under the conditions of process step (b), followed by process step (k), both as described above.

Compounds of formula (XXII) where R¹ is not hydrogen may be obtained from compounds of formula (XXlla) under the conditions of process step (r), a coupling reaction. Suitable conditions include those described in process steps (h) or (p) regarding alkylation reactions as well as arylation reactions well known to the skilled man. Suitable alkylation conditions may include:
1.5eq of base (eg Cs₂CO₃) and 1.25eq of alkylating agent, (eg R¹Br), in DMF at 70°C for 3 hours.
Suitable arylation conditions may include:
2eq of R¹-B(OH)₂, 1.5eq of Cu(II)acetate catalyst, 2 eq of pyridine in DCM, for 2 days, under a stream of compressed air. (P.Y.S. Lam et al, Tetrahedron Lett. 39; 2941; 1998)

Compounds of formula (I), where R¹, R², R³, R⁵, R⁶, R⁷, R⁸, R⁹ and R¹⁰ are as described above, R⁴ is hydrogen and X is nitrogen, with the proviso one of R⁹ and R¹⁰ is not hydrogen and R¹ is attached to an imidazole N atom, may be prepared from compounds of formula (XXIX) in accordance with the reaction scheme below.

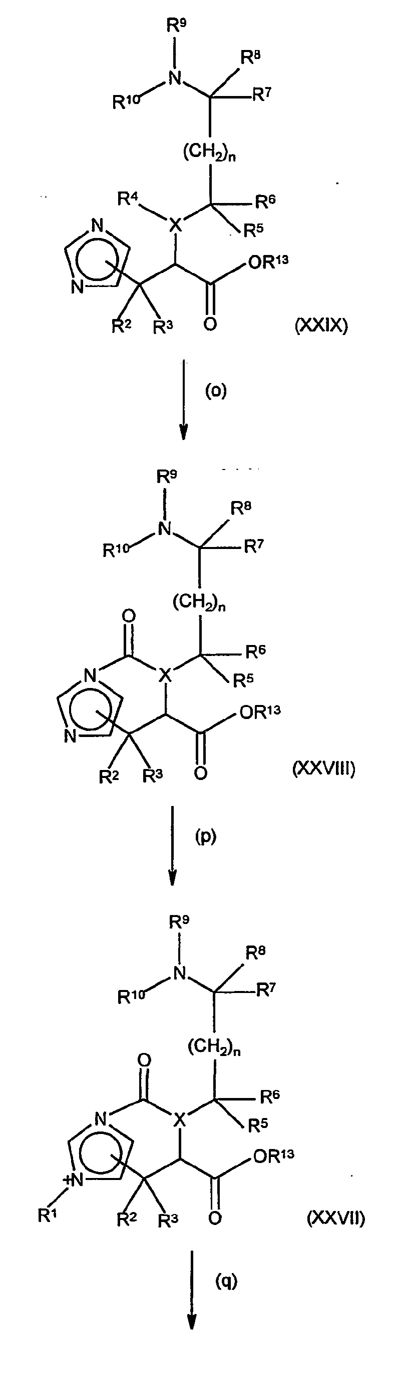

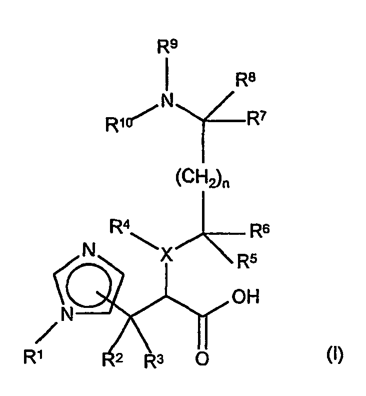

Compounds of formula (XXVIII) may be prepared from compounds of formula (XXIX), where R⁴ is hydrogen and one of R⁹ or R¹⁰ is not hydrogen, by process step (o), a carbonylation reaction. The reaction may be performed under standard conditions, such as described in Tetrahedron 1996, 52, 5363. Appropriate conditions include reacting 1eq of (XXIX) with 1eq of carbonyldiimidazole in *N,N-*dimethylformamide at 60°C for 17 hours.

Compounds of formula (XXVII) may be prepared from compounds of formula (XXVIII) by process step (p), an alkylation reaction. This may be conducted under standard conditions, e.g. reacting (XXVIII) with an alkylating agent such as an alkyl halide, optionally in the presence of a catalyst, in a suitable solvent. Suitable conditions include treating 1eq of (XXVIII) with 2eq of R¹-Cl in acetonitrile at reflux for 18 hours.

Compounds of formula (I) may be prepared from compounds of formula (XXVII) under the conditions of process step (q), a hydrolytic deprotection reaction. The starting material is treated with an aqueous acid, preferably hydrochloric or sulfuric acid.

Compounds of formula (XXIX) may be prepared by the routes disclosed in this document, wherein R¹ is instead hydrogen.

All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the Examples and Preparations hereto.

The present invention provides for the compounds of formula (I) and pharmaceutically acceptable salts, solvates and prodrugs thereof for use as a medicament.

The invention further provides for the use of a TAFIa inhibitor in the preparation of a medicament for the treatment or prevention of a condition selected from thrombosis, atherosclerosis, adhesions, dermal scarring, cancer, fibrotic conditions, inflammatory diseases and those conditions which benefit from maintaining or enhancing bradykinin levels in the body.

Preferably the TAFIa inhibitor is a compound of formula (I) as described herein. Accordingly the present invention provides for the use of a compound of formula (I) or a pharmaceutically acceptable salt, solvate or prodrug thereof in the preparation of a medicament for the treatment or prevention of a condition selected from thrombosis, atherosclerosis, adhesions, dermal scarring, cancer, fibrotic conditions, inflammatory diseases and those conditions which benefit from maintaining or enhancing bradykinin levels in the body.

Additionally the invention provides a method of treating or preventing thrombosis, atherosclerosis, adhesions, dermal scarring, cancer, fibrotic conditions, inflammatory diseases and those conditions which benefit from maintaining or enhancing bradykinin levels in the body which comprises administering a therapeutically effective amount of a TAFIa inhiitor and pharmaceutically acceptable salts, solvates and prodrugs thereof to a patient in need of such treatment.

Preferably the TAFla inhibitor is a compound of formula (I) as described herein. Accordingly the present invention provides a method of treating or preventing thrombosis, atherosclerosis, adhesions, dermal scarring, cancer, fibrotic conditions, inflammatory diseases and those conditions which benefit from maintaining or enhancing bradykinin levels in the body which comprises administering a therapeutically effective amount of a compound of formula (I) and pharmaceutically acceptable salts, solvates and prodrugs thereof to a patient in need of such treatment.

Thrombotic conditions are amongst the most common cause of death in the developed world. There are large numbers of anti-thrombotic agents available to treat these conditions. Most agents work by reducing thrombus formation. All these agents are associated with varying degrees of adverse hemorrhagic side effects. Accordingly, patients being treated in this manner will require regular monitoring in order to avoid adverse bleeding events.

There is a need for an antithrombotic that is efficacious but does not cause bleeding. However this would seem impossible given the inherent contradiction between stopping clot formation to prevent thrombotic disease, and allowing clot formation so as to prevent the patient hemorrhaging.

Surprisingly this has been solved by the compounds of the present invention which are a class of TAFIa inhibitors. Most conventional therapies act to inhibit coagulation or platelet activation. TAFIa inhibitors work by enhancing fibrinolysis and therefore the rate at which the clot is dissolved. This has the effect of shifting the equilibrium between coagulation and fibrinolysis, in favour of fibrinolysis. Most clinically relevant thrombus are sub acute, that is they form slowly over time. The effect of shifting the equilibrium in favour of fibrinolysis is that these clots are dissolved before they become clinically significant.

In the case of vascular injury, the equilibrium moves back in favour of coagulation. The body's first responses of vasoconstriction and platelet agglutination remain unimpaired by the use of TAFIa inhibitors. The body then rapidly activates the coagulation cascade. The effect of this is to temporarily shift the equilibrium towards coagulation and allow formation of a hemostatic plug using fibrin. Once the vascular injury is sealed the body will revert to its pre-injury equilibrium.

The present invention also provides for the use of TAFIa inhibitors in the preparation of a medicament for the treatment or prevention of thrombosis, particularly myocardial infarction, deep vein thrombosis, stroke, young stroke, peripheral vascular disease, angina and other forms of acute coronary syndromes, disseminated intravascular coagulation, sepsis, pulmonary embolism, embolic events secondary to cardiac arrhythmias and the prevention of cardiovascular events following intervention surgery. Preferably said TAFIa inhibitor should have a Ki of less than 20µM, using the assay described below. Preferably said TAFIa inhibitor should have a selectivity for TAFIa over carboxypeptidase N of >50:1, preferably >1000:1, using the assay described below. Preferably said TAFIa inhibitors are non-peptidic.

Preferably the TAFIa inhibitor is a compound of formula (I) as disclosed herein. Accordingly the present invention provides for the use of a compound of formula (I) in the preparation of a medicament for the treatment of a thrombotic condition selected from myocardial infarction, deep vein thrombosis, stroke, young stroke, cerebral infarction, cerebral thrombosis, cerebral embolism, peripheral vascular disease, angina and other forms of acute coronary syndromes, disseminated intravascular coagulation, sepsis, pulmonary embolism, embolic events secondary to cardiac arrhythmias and the prevention of cardiovascular events following surgical revascularisation or intervention.

The invention also provides for a method of treating or preventing thrombosis, particularly myocardial infarction, deep vein thrombosis, stroke, young stroke, cerebral infarction, cerebral thrombosis, cerebral embolism, peripheral vascular disease, angina and other forms of acute coronary syndromes, disseminated intravascular coagulation, sepsis, pulmonary embolism, embolic events secondary to cardiac arrhythmias and the prevention of cardiovascular events following intervention surgery which comprises administering a therapeutically effective amount of a compound of formula (I) and pharmaceutically acceptable salts, solvates and prodrugs thereof to a patient in need of such treatment.

Subjects with thrombotic conditions which are suitable for treatment by the present invention include those having conditions associated with hypercoagulability. These would include though not limited to: factor V mutation, antithrombin III deficiency, protein C and protein S deficiencies, polycythemia vera, heparin cofactor 11 and subjects exhibiting hyperhomocysteinaemia or homocysteinuria.

The present invention also includes as a thrombotic indication the improvement of organ function seen after transplantation, by reducing blood clotting and thus preserving function.

Cardiovascular events following intervention surgery include conditions such as restenosis or reocclusion following interventions such as percutaneous transluminal coronary angioplasty, grafting, stent in-placement, coronary bypass surgery or any other forms of surgical revascularisation or intervention

In the present invention, disseminated intravascular coagulation includes all conditions resulting from intravascular activation of the coagulation process. This might occur acutely through the release of procoagulant substances (eg. obstetric emergencies, snakebite, crush injury malignancy), by abnormal contact of the blood (eg.infections, bums, extracorporeal circulation, grafts) or though generation of procoagulants in the blood (transfusion reactions, leukemia); or chronically, (eg. toxemia, malignant hypertension, severe liver cirrhosis).

Deep vein thrombosis also encompasses what is known as 'economy class syndrome', where clots form in subjects forced to endure cramped conditions for a period of time, such as those sitting in cramped economy class seats on a plane.

The present invention also provides for the use of TAFIa inhibitors and/or TAFI inhibitors as a coating on intravascular devices such as indwelling catheters for dialysis, replacement heart valves or arterial stents; and as a coating on extra corporeal blood circulation devices such as heart, lung and kidney dialysis machines, to prevent thrombosis, particularly myocardial infarction, deep vein thrombosis, stroke, young stroke, cerebral infarction, cerebral thrombosis, cerebral embolism, peripheral vascular disease, angina and other forms of acute coronary syndromes, disseminated intravascular coagulation, sepsis, pulmonary embolism, embolic events secondary to cardiac arrhythmias and the prevention of cardiovascular events such as restenosis following intervention surgery such as percutaneous transluminal coronary angioplasty, grafting, stent in-placement, coronary bypass surgery or any other forms of surgical revascularisation or intervention. Particularly preferred as a coating are compounds of formula (I) and pharmaceutically acceptable salts, solvates and prodrugs thereof.

Accordingly the present invention provides for the use of TAFIa inhibitors and/or TAFI inhibitors as a coating on intravascular devices.

Further the present invention provides for the use of a compound of formula (I) as a coating on intravascular devices.

The invention includes intravascular devices, of which the intravascular portion is coated with a TAFIa inhibitor and/or a TAFI inhibitor; and extra corporeal blood circulation devices such as heart, lung and kidney dialysis machines, where the portion coming into contact with the subjects blood are coated with a TAFIa inhibitor and/or a TAFI inhibitor. Particularly preferred are those intravascular or extra corporeal blood circulation devices coated with compounds of formula (I) and pharmaceutically acceptable salts, solvates and prodrugs thereof. Preferably said TAFIa inhibitor should have a Ki of less than 20µM, using the assay described below. Preferably said TAFIa inhibitor should have a selectivity for TAFIa over carboxypeptidase N of >50:1, preferably >1000:1, using the assay described below. Preferably said TAFIa inhibitors are non-peptidic.

Accordingly the present invention provides an intravascular device coated with a TAFIa inhibitor.

Further, the present invention provides an intravascular device coated with a compound of formula (I).

The compounds of the present invention were tested in a model of coronary artery reperfusion using a method similar to that described by W. E. Rote et al, J. Cardiovasc. Pharmacol., 1994, 23, 203, and were found to be efficacious.

TAFIa inhibitors are also useful in the treatment of atherosclerosis. Atherosclerosis is a common condition in subjects suffering from peripheral vascular disease, insulin resistance and the group of conditions commonly referred to as 'Syndrome X'. Syndrome X is a term often used to group together a number of interrelated diseases. The first stage of syndrome X consists of insulin resistance, abnormal cholesterol and triglyceride levels, obesity and hypertension. Any one of these conditions may be used to diagnose the start of Syndrome X. The disease may then progress with one condition leading to the development of another in the group. For example insulin resistance is associated with high lipid levels, hypertension and obesity. The disease then cascades, with the development of each additional condition increasing the risk of developing more serious diseases. This can progress to the development of diabetes, kidney disease and heart disease. These diseases may lead to stroke, myocardial infarction and organ failure.

Conventional treatment of myocardial ischaemia in clinically stable coronary artery disease is predominately designed to reduce cardiac workload and enhance blood flow. Such approaches clearly reduce myocardial ischaemia thus increasing quality of life. However, these strategies have little effect on the pathogenesis of coronary atherosclerosis which is a chronic process of continuous remodeling of the vascular tree in response to varying degrees of vascular injury.

A role for thrombus formation in the pathophysiology of stable angina pectoris has recently been highlighted by several independent groups. The formation of non-occlusive thrombi not only restrict blood flow, but due to incomplete endogenous lysis may be incorporated by the arterial wall as solidified plaque material enhancing the atherosclerotic process. Long term administration of a TAFIa inhibitor prevents the formation of thrombi and therefore provides a safe and efficacious treatment which alleviates the symptoms of angina pectoris. Without thrombi present, they cannot be incorporated into the arterial wall and thus a TAFIa inhibitor impairs the progression of the disease.

The present invention also provides for the use of compounds of formula (I) and pharmaceutically acceptable salts, solvates and prodrugs thereof in the preparation of a medicament for the treatment or prevention of atherosclerosis.

The invention also provides for a method of treating or preventing atherosclerosis which comprises administering a therapeutically effective amount of a compound of formula (I) and pharmaceutically acceptable salts and prodrugs thereof to a patient in need of treatment.

Further the invention also provides for the use of a TAFIa inhibitor in the preparation of a medicament for the treatment or prevention of atherosclerosis. Preferably said TAFIa inhibitor should have a Ki of less than 20µM, using the assay described below. Preferably said TAFIa inhibitor should have a selectivity for TAFIa over carboxypeptidase N of >50:1, preferably >1000:1, using the assay described below. Preferably said TAFIa inhibitors are non-peptidic.

Atherosclerosis is taken to include both primary and secondary coronary artery disease, in which atherosclerosis restricts the blood supply to the heart. Primary prevention of coronary artery disease means preventing the onset of ischemic complications such as myocardial infarction in patients with no history of coronary artery disease but who have one or more risk factors. Secondary prevention of coronary artery disease means preventing ischemic complications in patients with established coronary artery disease, such as patients who have had a previous myocardial infarction.

TAFIa inhibitors are also effective in inhibiting tumour maturation and progression. Metastasis is a complex and multifactorial process which is not yet fully understood. Accordingly, whilst not wishing to be bound by any theory, it is believed that the haemostatic system is involved at several levels of cancer pathology, including neovascularisation, shedding of cells from the primary tumour, invasion of the blood supply, adherence to the vessel wall and growth at the metastatic site. It is thought that the efficacy of TAFIa inhibitors stems from an ability to reduce fibrin deposition around solid tumours and thereby inhibit the above processes.

The present invention also provides for the use of compounds of formula (I) and pharmaceutically acceptable salts, solvates and prodrugs thereof in the preparation of a medicament for the treatment or prevention of cancer.

The invention also provides for a method of treating or preventing cancer which comprises administering a therapeutically effective amount of a compound of formula (I) and pharmaceutically acceptable salts, solvates and prodrugs thereof to a patient in need of such treatment.

Further the invention also provides for the use of a TAFIa inhibitor in the preparation of a medicament for the treatment or prevention of cancer. Preferably said TAFIa inhibitor should have a Ki of less than 20µM, using the assay described below. Preferably said TAFIa inhibitor should have a selectivity for TAFIa over carboxypeptidase N of >50:1, preferably >1000:1, using the assay described below. Preferably said TAFIa inhibitors are non-peptidic.

TAFIa inhibitors are also effective in preventing the formation of adhesions in the body. Most surgical procedures and physical trauma result in bleeding into the cavity between tissues. The blood which collects at these sites then clots forming fibrin rich thrombi. These thrombi bridge the gaps between adjacent tissues and act as a foci for the accumulation of inflammatory cells and fibroblasts. Invading fibroblasts lay down a collagen rich extracellular matrix which strengthens the adhesion of the tissues producing a firm bond which may then restrict movement. Adhesions have been characterised according to their location and may result following any surgery e.g. abdominal, orthopaedic, neurological, cardiovascular and ocular. This, inappropriate, adhesion of tissues post surgery or trauma is a major issue which can lead to various outcomes e.g. "aches and pains", "twinges", local inflammation, restriction in mobility, pain, intestinal obstruction and sometimes in the most severe cases death. In the case of gynaecological surgery, infertility may result. Additionally clots forming fibrin rich thrombi are implicated in dermal scarring and restenosis.

Without being bound by any theory, it is believed that adhesion formation may be enhanced due to a deficiency in fibrinolysis resulting in enhanced and maintained clot formation. Treatment with a TAFIa inhibitor peri- and/or post-surgical intervention may enhance fibrinolysis of the fibrin rich thrombi and hence inhibit thrombi formation, accretion, stabilisation and therefore inhibit adhesion formation. A TAFIa inhibitor given either systemically, or locally as a topical application, may be seen to be of benefit in a range of surgical procedures. In addition, administration of a TAFIa inhibitor may be seen to treat adhesions resulting from other forms of non surgical physical trauma where this has caused internal bleeding. Examples of such trauma might include sporting injuries, or anything else resulting in a tear, cut, bruise or induaration of the body.

The present invention also provides for the use of compounds of formula (I) and pharmaceutically acceptable salts, solvates and prodrugs thereof in the preparation of a medicament for the treatment or prevention of adhesions or dermal scarring.

The invention also provides for a method of treating or preventing adhesions or dermal scarring which comprises administering a therapeutically effective amount of a compound of formulae (I) and pharmaceutically acceptable salts, solvates and prodrugs thereof to a patient in need of such treatment.

Further the invention also provides for the use of a TAFIa inhibitor in the preparation of a medicament for the treatment or prevention of adhesions or dermal scarring. Preferably said TAFIa inhibitor should have a Ki of less than 20µM, using the assay described below. Preferably said TAFIa inhibitor should have a selectivity for TAFIa over carboxypeptidase N of >50:1, preferably >1000:1, using the assay described below. Preferably said TAFIa inhibitors are non-peptidic.

TAFIa binds to and breaks down bradykinin (Tan et al. Biochemistry 1995, 34, 5811). There are many conditions which are known to benefit from maintaining or enhancing levels of bradykinin. Accordingly the present invention also provides for the use of compounds of formula (I) and pharmaceutically acceptable salts, solvates and prodrugs thereof in the preparation of a medicament for the treatment or prevention of conditions which benefit from maintaining or enhancing levels of bradykinin.

The invention also provides for a method of treating or preventing conditions which benefit from maintaining or enhancing levels of bradykinin which comprises administering a therapeutically effective amount of a compound of formula (I) and pharmaceutically acceptable salts, solvates and prodrugs thereof to a patient in need of such treatment.

Conditions known to benefit from maintaining or enhancing bradykinin levels include: diseases such as hypertension, angina, heart failure, pulmonary hypertension, renal failure and organ failure.

TAFIa inhibitors are efficacious in treatment of any condition in which fibrosis is a contributing factor. Accordingly the present invention also provides for the use of TAFIa inhibitors in the preparation of a medicament for the treatment or prevention of fibrotic disease. Preferably said TAFIa inhibitor should have a Ki of less than 20µM, using the assay described below. Preferably said TAFIa inhibitor should have a selectivity for TAFIa over carboxypeptidase N of >50:1, preferably >1000:1, using the assay described below. Preferably said TAFIa inhibitors are non-peptidic. Particularly preferred are compounds of formula (I) and pharmaceutically acceptable salts, solvates and prodrugs thereof.

Suitable fibrotic conditions include cystic fibrosis, pulmonary fibrotic diseases eg chronic obstructive pulmonary disease (COPD), adult respiratory distress syndrome (ARDS), fibromuscular dysplasia, fibrotic lung disease and fibrin deposits in the eye during opthalmic surgery.

Accordingly the present invention provides for the use of a compound of formula (I) as disclosed herein in the preparation of a medicament for the treatment or prevention of a fibrotic condition selected from cystic fibrosis, pulmonary fibrotic diseases, chronic obstructive pulmonary disease (COPD), adult respiratory distress syndrome (ARDS), fibromuscular dysplasia, fibrotic lung disease and fibrin deposits in the eye during opthalmic surgery.

The invention also provides for a method of treating or preventing a fibrotic condition selected from cystic fibrosis, pulmonary fibrotic diseases, chronic obstructive pulmonary disease (COPD), adult respiratory distress syndrome (ARDS), fibromuscular dysplasia, fibrotic lung disease and fibrin deposits in the eye during opthalmic surgery which comprises administering a therapeutically effective amount of a compound of formula (I) and pharmaceutically acceptable salts and prodrugs thereof to a patient in need of treatment.

TAFIa inhibitors are efficacious in treatment of inflammation. Accordingly the present invention also provides for the use of TAFIa inhibitors in the preparation of a medicament for the treatment or prevention of inflammation. Preferably said TAFIa inhibitor should have a Ki of less than 20µM, using the assay described below. Preferably said TAFIa inhibitor should have a selectivity for TAFIa over carboxypeptidase N of >50:1, preferably >1000:1, using the assay described below. Preferably said TAFIa inhibitors are non-peptidic. Particularly preferred are compounds of formula (I) and pharmaceutically acceptable salts, solvates and prodrugs thereof.

In particular the invention may be used for the treatment or prevention of inflammatory diseases such as asthma, arthritis, endometriosis, inflammatory bowel diseases, psoriasis and atopic dermatitis and for neurodegenerative diseases such as Alzheimers and Parkinsons.

Accordingly the present invention provides for the use of a compound of formula (I) and pharmaceutically acceptable salts, solvates and prodrugs thereof in the preparation of a medicament for the treatment of an inflammatory disease selected from asthma, arthritis, endometriosis, inflammatory bowel diseases, psoriasis and atopic dermatitis and neurodegenerative diseases, Alzheimers and Parkinsons.

The invention also provides for a method of treating or preventing an inflammatory disease selected from asthma, arthritis, endometriosis, inflammatory bowel diseases, psoriasis and atopic dermatitis and neurodegenerative diseases, Alzheimers and Parkinsons which comprises administering a therapeutically effective amount of a compound of formula (I) and pharmaceutically acceptable salts and prodrugs thereof to a patient in need of treatment.

It is to be appreciated that all references herein to treatment include curative, palliative and prophylactic treatment.

The compounds of the present invention have been tested using the following assay. To determine the degree of TAFIa inhibition, compounds were incubated with activated TAFI, and the amount of inhibition expressed in terms of Ki. This assay is based on that disclosed in Boffa et al, J. Biol. Chem., 1998, 273, 2127.

### Assay for TAFIa inhibition.

### i) TAFI activation

Human TAFI (recombinant or purified) was activated by incubating 20µl of stock solution (360µg/ml) with 10µl of human thrombin (10NIH units/ml), 10µl of rabbit thrombomodulin (30µg/ml), 6µl calcium chloride (50mM) in 50µL of 20mM HEPES (N-[2-hydroxyethyl]piperazine-N-[2-ethanesulfonic acid]) buffer containing 150mM sodium chloride and 0.01% TWEEN 80 (polyoxyethylene-sorbitan monooleate) pH 7.6 for 20 minutes at 22°C. At the end of the incubation period, thrombin was neutralised by the addition of 10µL of PPACK (D-Phe-Pro-Arg chloromethyl ketone)(100nM). TAFIa solution was stored on ice for 5 minutes and finally diluted with 175µl of HEPES buffer.

### ii) Ki Determination (TAFIa)

### Calculated Ki

A number of different dilutions of the test compound in water were made up. To 20µl of each dilution was added 150µl of HEPES buffer and 10µl of TAFIa, which was then pre-incubated for 15 minutes at 24°C. To each dilution was then added 20µl furylacryloyl-alanyl-lysine (FAAL) at a standard concentration. Substrate turn over was measured by reading the absorbance of the reaction mixture at 330nm every 15 seconds for 30 minutes. Reaction was performed at 24°C and samples were mixed for 3 seconds prior to each absorbance reading.

A graph of % inhibition against test compound concentration was then plotted; from which was calculated the IC₅₀ value. The Ki value may then be calculated using the Cheng-Prusoff equation.

Two controls, positive and negative, were used to check the accuracy of the results in each case. For the first control, the assay was performed as above, but with 20µl of water rather than a dilution of the test compound. This showed minimal inhibition. For the second control, the assay was performed as above, but with an effective amount of a non specific carboxypeptidase inhibitor rather than a dilution of the test compound. This showed maximal inhibition.

Should the two controls not demonstrate minimal and maximal inhibition respectively then the results were discounted and the test compound reanalysed.

Using the above assay the compounds of the present invention were found to be potent and selective inhibitors of TAFIa. All compounds had a Ki value less than 20µM. The specific Ki value of certain compounds are detailed below:
(±)-6-Amino-2-[(1-*n*-propyl-1*H*-imidazol-4-yl)methyl]hexanoic acid (Example 3) Ki = 310 nM
(+)-(2*S*)-5-Amino-2-[(1-*n*-propyl-1*H*-imidazol-4-yl)methyl]pentanoic acid (Example 7) Ki = 13 nM
(2*S*)-2-[(2-Aminoethyl)amino]-3-(1*H*-imidazol-4-yl)propanoic acid (Example 11) Ki = 344 nM
(2*S*)-2-[(2-Aminoethyl)amino]-3-[1-(1,3-thiazol-5-ylmethyl)-1*H*-imidazol-4-yl]propanoic acid (Example 45) Ki = 197 nM

The selectivity of the compounds of the present invention for TAFIa over carboxypeptidase N has also been determined. This was done by calculating the Ki of the compounds of the present invention for carboxypeptidase N, then comparing it to the Ki for TAFIa. The Ki was calculated using the assay for the calculation of TAFIa Ki, but substituting 10µl of human carboxypeptidase N for 10µl of TAFIa.

The compounds of the present invention exhibit a strong selectivity for TAFIa over carboxypeptidase N of the order of >50:1.

The compounds of the present invention are TAFIa inhibitors, whose utility is based upon preventing the reaction between a developing thrombus and TAFIa.

It has been found that the compounds of the present invention are also capable of binding to a TAFI molecule, at the site implicated in the reaction between TAFIa and the developing clot. The use of TAFIa inhibitors as described above in terms of scope and utility, includes such TAFIa inhibitors which bind to TAFI.

The compounds of the formula (I) can also be administered together with other antithrombotics, including antiplatelet, anticoagulants and profibrinolytics. Suitable antithrombotics include: aspirin, Plavix^{™}, ticlopidine, warfarin (coumarin^{™}), unfractionated heparin, hirudin (Lepirudin^{™}), streptokinase, urokinase, recombinant tissue plasminogen activator (tPA), dipyridamole, Reopro^{™}, Aggrastat^{™}, and Integrilin^{™}. The compounds of the formula (I) can also be administered together with antihypertensives and with agents to treat dyslipidaemia such as statins eg Lipitor^{™}. Further suitable drug classes for coadministration include Factor X inhibitors and antiarrhythmics such as amiodarone or digoxin.

The present invention provides for the use of a TAFIa inhibitor in the preparation of a medicament in combination with an antithrombotic for the treatment of thrombosis. The present invention provides for the use of a compound of formula (I) as described above in the preparation of a medicament in combination with an antithrombotic for the treatment of thrombosis.

In a preferred embodiment the antithrombotic is an profibrinolytic.
In a more preferred embodiment the antithrombotic is recombinant tissue plasminogen activator (tPA).

The present invention provides a method of treating or preventing thrombosis, which comprises administering a therapeutically effective amount of a TAFIa inhibitor in combination with an antifibrinolytic to a patient in need of such treatment.

The present invention also provides for a method of treating or preventing thrombosis, which comprises administering a therapeutically effective amount of a compound of formula (I) and pharmaceutically acceptable salts, solvates and prodrugs thereof in combination with an profibrinolytic to a patient in need of such treatment.

In a preferred embodiment the antithrombotic is an profibrinolytic.
In a more preferred embodiment the antithrombotic is recombinant tissue plasminogen activator (tPA).

The present invention provides for a kit comprising:
a) a composition comprising a compound of formula (I) as disclosed herein and a pharmaceutically acceptable diluent or carrier;
b) a composition comprising an antithrombotic and a pharmaceutically acceptable diluent or carrier; and
c) a container

The components of this kit may be administered separately, simultaneously or sequentially.

The ability of a TAFIa inhibitor used in conjunction with an antithrombotic to lyse thrombi was investigated using surgical procedures similar to those outlined in J. Cardiovasc. Pharmacol. 1994 Feb; 23(2)194-202 and 203-211.

The study was designed with 4 groups (8 dogs/group):
(i) aspirin pre-treatment/vehicle infusion;
(ii) no pre-treatment/vehicle infusion;
(iii) no pre-treatment/ TAFIa inhibitor; and
(iv) aspirin pre-treatment/ TAFIa inhibitor.

### Method

Aspirin pre-treatment was 325mg daily for 3 days. TAFIa inhibitor (compound of Ex 7) was given as a loading dose followed by a continuous infusion with the aim of achieving a steady state free plasma concentration of 4000nM (220x IC₅₀ for TAFIa, *in vitro*). Thirty minutes after initiating vehicle or compound infusion a continuous electrical current was delivered to the lumen of the left circumflex (LCX) coronary artery to cause endothelial damage and stimulate the production of a thrombus. Thrombi were allowed to mature for 1 hour prior to attempting to lyse the thrombus and cause vessel reperfusion with t-PA. A total of 4 bolus injections of t-PA (each 0.45 mg/kg i.v.) were given sequentially at 15 minute intervals. Blood flow through the coronary artery was then monitored for a further 2 hours so as to assess vessel patency. Time to vessel occlusion, and reperfusion were measured and the quantity and quality of blood flow analysed post-vessel re-perfusion. In addition, the effect of treatment on surgical bleeding, activated clotting time, cutaneous bleeding and platelet aggregation was also assessed.

### Results

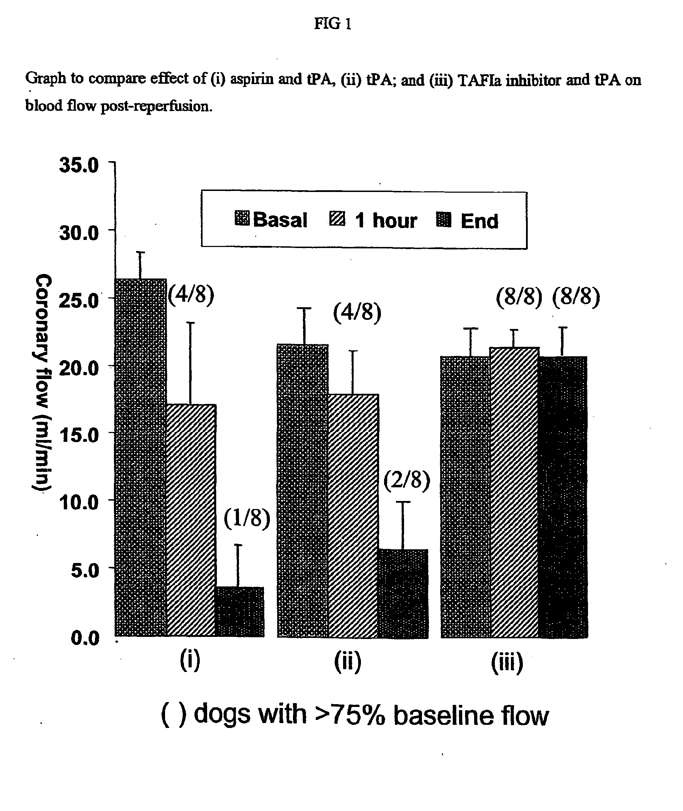

Data is described in Fig 1. From Fig 1 it can be seen that:
1) TPA alone is superior to the combination of tPA and aspirin.
2) The combination of a TAFIa inhibitor and tPA is far superior to tPA alone.
3) The improvement in coronary blood flow caused by TAFIa inhibitor was maintained for the whole of the reperfusion period (165 minutes) with significantly greater flow compared to respective controls. Notably, TAFIa inhibitor significantly increased the proportion of animals in which flow was >75% of baseline at the end of the protocol. At the end of the experiment only 2/8 dogs in the no pre-treatment/vehicle group and 1/8 dogs in the aspirin pre-treatment/vehicle group were patent. In contrast, the injured vessels were patent in 8/8 dogs in the TAFIa inhibitor treatment group.
4) There was no effect of any of the treatments on surgical bleeding, cutaneous bleeding time, activated clotting time or ADP induced platelet aggregation either pre- or post t-PA treatment.

Combination (iv) is not considered here.

The present invention provides for a composition comprising a compound of formula (I) and a pharmaceutically acceptable excipient, diluent or carrier.

The compounds of formula (I) can be administered alone but will generally be administered in admixture with a suitable pharmaceutical excipient, diluent or carrier selected with regard to the intended route of administration and standard pharmaceutical practice.

For example, the compounds of formula (I) can be administered orally, buccally or sublingually in the form of tablets, capsules, ovules, elixirs, solutions or suspensions, which may contain flavouring or colouring agents, for immediate-, delayed-, modified-, sustained-, pulsed- or controlled-release applications.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the compounds of the formula (I) may be combined with various sweetening or flavouring agents, colouring matter or dyes, with emulsifying and/or suspending agents and with diluents such as water, ethanol, propylene glycol and glycerin, and combinations thereof.

The compounds of formula (I) may also be administered in the form of a liquid or suspension filled soft or hard gelatin capsule. Such capsules are generally made of gelatin, glycerin, water and sorbitol. Hard capsules are distinguished from soft capsules by containing less water and thus having a correspondingly stronger shell. Additional excipients suitable for use in such capsules include propylene glycol, ethanol, water, glycerol and edible oils.

The compounds of formula (I) can also be administered parenterally, for example, intravenously, intra-arterially, intraperitoneally, intrathecally, intraventricularly, intraurethrally, intrasternally, intracranially, intramuscularly or subcutaneously, or they may be administered by infusion techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

The compounds of formula (I) can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A^{™}) or 1,1,1,2,3,3,3-heptafluoropropane (HFA 227EA^{™}), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the formula (I) and a suitable powder base such as lactose or starch.

Alternatively, the compounds of the formula (I) can be administered in the form of a suppository or pessary, or they may be applied topically in the form of a gel, hydrogel, lotion, solution, cream, ointment or dusting powder. The compounds of the formula (I) may also be dermally or transdermally administered, for example, by the use of a skin patch. They may also be administered by the pulmonary or rectal routes.

They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For topical application, the compounds of the formula (I) can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the formula (I) may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

The invention is further illustrated by the following, non-limiting examples.

Melting points were determined on a Gallenkamp melting point apparatus using glass capillary tubes and are uncorrected. Unless otherwise indicated all reactions were carried out under a nitrogen atmosphere, using commercially available anhydrous solvents. '0.88 Ammonia' refers to commercially-available aqueous ammonia solution of about 0.88 specific gravity. Thin-layer chromatography was performed on glass-backed pre-coated Merck silica gel (60 F254) plates, and silica gel column chromatography was carried out using 40-63µm silica gel (Merck silica gel 60). Ion exchange chromatography was performed using with the specified ion exchange resin which had been pre-washed with deionised water. Proton NMR spectra were measured on a Varian Inova 300, Varian Inova 400, or Varian Mercury 400 spectrometer in the solvents specified. In the NMR spectra, only exchangeable protons which appeared distinct from the solvent peaks are reported. Low resolution mass spectra were recorded on either a Fisons Trio 1000, using thermospray positive ionisation, or a Finnigan Navigator, using electrospray positive or negative ionisation. High resolution mass spectra were recorded on a Bruker Apex II FT-MS using electrospray positive ionisation. Combustion analyses were conducted by Exeter Analytical UK. Ltd., Uxbridge, Middlesex. Optical rotations were determined at 25°C using a Perkin Elmer 341 polarimeter using the solvents and concentrations specified. Example compounds designated as (+) or (-) optical isomers are assigned based on the sign of optical rotation when determined in deionised water.

### Abbreviations and Definitions

- Arbocel™: Filtration agent, from J. Rettenmaier & Sohne, Germany
- Amberlyst®: Ion exchange resin, available from Aldrich Chemical
- 15: Company
- atm: Pressure in atmospheres (1 atm = 760 Torr)
- Biotage™: Chromatography performed using Flash 75 silica gel cartridge, from Biotage, UK
- BOC: *tert*-Butyloxycarbonyl group
- br: Broad
- *c*: Concentration used for optical rotation measurements in g per 100 ml (1 mg/ml is *c* 0.10)
- cat: Catalytic
- d: Doublet
- dd: Doublet of doublets
- Degussa ®: 10 wt% palladium on activated carbon, Degussa type E101
- 101 Company: available from Aldrich Chemical Company
- DOWEX®: Ion exchange resin, from Aldrich Chemical Company
- ee: Enantiomeric excess
- HRMS: High Resolution Mass Spectrocopy (electrospray ionisation positive scan)
- Hyflo™: Hyflo super cel®, from Aldrich Chemical Company
- liq: liquid
- LRMS: Low Resolution Mass Spectroscopy (electrospray or thermospray ionisation positive scan)
- LRMS (ES⁻): Low Resolution Mass Spectroscopy (electrospray ionisation negative scan)
- m: Multiplet
- m/z: Mass spectrum peak
- MCI™ gel: High porous polymer, CHP20P 75-150µm, from Mitsubishi Chemical Corporation
- q: Quartet
- Rf: Retention factor on TLC
- s: Singlet
- Sep-Pak®: Reverse phase C18 silica gel cartridge, Waters Corporation
- t: Triplet
- TLC: Thin Layer Chromatography
- δ: Chemical shift

### Example 1

### (±)-5-Amino-2-(1H-imidazol-4-ylmethyl)pentanoic acid

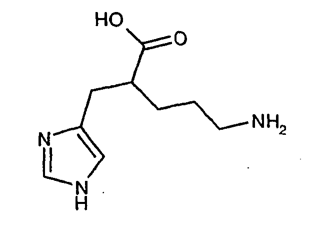

A mixture of the ester from Preparation 1 (150mg, 0.25mmol) in dioxane (2ml) and aqueous sodium hydroxide (2ml, 2N) was stirred at room temperature for 1.5 hours. Aqueous hydrochloric acid (6ml, 6N) was carefully added, and the reaction heated under reflux for 24 hours. The cooled mixture was purified by ion exchange column chromatography (DOWEX® 50WX8-200), using an elution gradient of deionised water : 0.88 ammonia (100:0 to 97:3). The product was triturated with methanol to give the title compound as a white solid, 28mg, 57% yield.
¹H-NMR (CD₃OD 300MHz) δ: 1.44-1.75 (m, 4H), 2.48 (m, 1H), 2.62 (dd, 1H), 2.90 (m, 3H), 6.81 (s, 1H), 7.55 (s, 1H).
HRMS : m/z 198.1242 (MH⁺), calcd 198.1237.

### Example 2

### (±)-5-Amino-2-[(1-n-propyl-1H-imidazol-4-yl)methyl]pentanoic acid

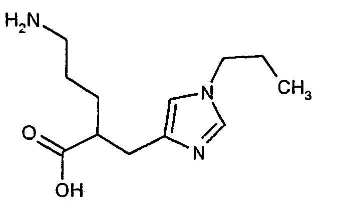

A mixture of the ester from Preparation 2 (85mg, 0.17mmol) in dioxane (1ml) and aqueous sodium hydroxide (1ml, 2N) was stirred at room temperature for 72 hours. TLC analysis showed starting material remaining, so the reaction was heated at 70°C for 3 hours. Aqueous hydrochloric acid (2ml, 6N) was added to the cooled solution and the reaction stirred at room temperature for 18 hours. TLC analysis showed starting material remaining, so the reaction was stirred at 70°C for a further 2 hours. The cooled mixture was extracted with hexane, and the remaining aqueous solution was purified by ion exchange column chromatography (DOWEX® 50WX8-200) eluting with a solvent gradient of deionised water : 0.88 ammonia (100:0 to 97:3). The product was dissolved in a minimum volume of deionised water, and freeze-dried to give the title compound as a gum, 18mg, 43% yield.
¹H-NMR (CD₃OD 300MHz) δ: 0.92 (t, 3H), 1.45-1.70 (m, 4H), 1.79 (m, 2H), 2.43-2.60 (m, 2H), 2.76-2.95 (m, 3H), 3.90 (t, 2H), 6.86 (s, 1H), 7.45 (s, 1H).
HRMS : m/z 240.1713 (MH⁺), calcd 240.1706.

### Example 3

### (±)-6-Amino-2-[(1-n-propyl-1H-imidazol-4-yl)methyl]hexanoic acid

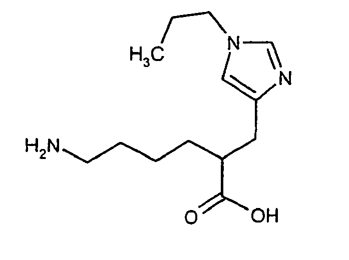

A mixture of the protected amine from Preparation 3 (17mg, 0.05mmol) in aqueous hydrochloric acid (2ml, 6N) was stirred at room temperature for 3 hours. The solution was purified directly by ion exchange chromatography (DOWEX® 50WX8-200), eluting with a solvent gradient of deionised water: 0.88 ammonia (100:0 to 97:3), to give the title compound, 7mg, 55% yield.
¹H-NMR (CD₃OD 300MHz) δ: 0.88 (t, 3H), 1.42 (m, 3H), 1.62 (m, 3H), 1.78 (m, 2H), 2.54 (m, 2H), 2.89 (m, 3H), 3.90 (t, 2H), 6.85 (s, 1H), 7.46 (s, 1H).
HRMS : m/z 254.1870 (MH⁺), calcd 254.1863.

### Example 4

### (-)-(2R)-5-Amino-2-[(1-n-butyl-1H-imidazol-4-yl)methyl]pentanoic acid

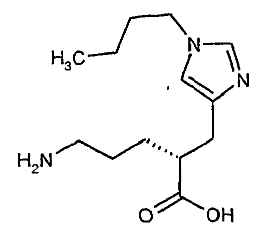

A mixture of the ester from Preparation 6 (185mg, 0.35mmol) in dioxane (6ml) and aqueous sodium hydroxide (6ml, 2N) was stirred at 50°C for 3 hours. Aqueous hydrochloric acid (12ml, 6N) was carefully added, and the reaction stirred at 70°C for a further 18 hours. The cooled mixture was washed with ether, and the aqueous solution purified by ion exchange chromatography (DOWEX® 50WX8-200) eluting with a solvent gradient of deionised water : 0.88 ammonia (100:0 to 95:5). The product was azeotroped well with ether and dried *in vacuo* to give the title compound as an off-white solid, 45mg, 51 % yield.
¹H-NMR (CD₃OD 300MHz) δ: 0.97 (t, 3H), 1.33 (m, 2H), 1.48-1.79 (m, 6H), 2.45-2.61 (m, 2H), 2.79-2.95 (m, 3H), 3.95 (t, 2H), 6.88 (s, 1H), 7.45 (s, 1H).
HRMS : m/z 254.1873 (MH⁺), calcd 254.1863.

### Example 5

### (+)-(2S)-5-Amino-2-[(1-n-butyl-1H-imidazol-4-yl)methyl]pentanoic acid

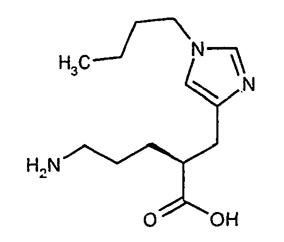

The title compound was obtained in 35% yield, from the ester from Preparation 7, following a similar procedure to that described in Example 4.
¹H-NMR (CD₃OD 300MHz) δ: 0.97 (t, 3H), 1.33 (m, 2H), 1.48-1.79 (m, 6H), 2.45-2.61 (m, 2H), 2.79-2.95 (m, 3H), 3.95 (t, 2H), 6.88 (s, 1H), 7.45 (s, 1H).
HRMS : m/z 254.1874 (M⁺), calcd 254.1863.
[α]_{D} = +3.7 (*c* 0.14, deionised water)
[α]_{D} = -5.2 (*c* 0.15, methanol)

### Example 6

### (-)-(2R)-5-Amino-2-[(1-n-propyl-1H-imidazol-4-yl)methyl]pentanoic acid

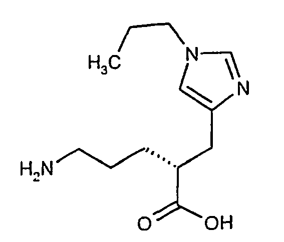

A solution of the protected amine from Preparation 9 (1.01g, 2.97mmol) in aqueous hydrochloric acid (15ml, 6N) was stirred at room temperature for 18 hours. The solution was purified directly by ion exchange chromatography (DOWEX® 50WX8-200), eluting with a solvent gradient of deionised water : 0.88 ammonia (100:0 to 97:3), to give the title compound, 680mg, 94% yield.
¹H-NMR (CD₃OD 400MHz) δ: 0.84 (t, 3H), 1.48 (m, 1H), 1.55-1.68 (m, 3H), 1.76 (m, 2H), 2.42-2.57 (m, 2H), 2.86 (m, 3H), 3.83 (t, 2H), 6.82 (s, 1H), 7.42 (s, 1H).
HRMS : m/z 262.1533 (MNa⁺), calcd 262.1526.
Anal. Found: C, 58.04; H, 8.93; N, 16.92. C₁₂H₂₁N₃O₂•0.5H₂O requires C, 58.04; H, 8.93; N, 16.92%.
[α]_{D} =-2.53(*c* 0.15, deionised water)

### Example 7

### (+)-(2S)-5-Amino-2-[(1-n-propyl-1H-imidazol-4-yl)methyl]pentanoic acid

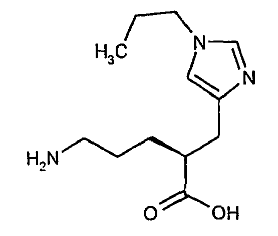

Lithium hydroxide monohydrate (1.1g, 28mmol) and water (28ml) were added to a solution of the lactam from Preparation 11 (3g, 9.33mmol) in tetrahydrofuran (45ml), and the reaction stirred at room temperature for 18 hours. The solution was neutralised using aqueous hydrochloric acid (6N), then further acid (15ml, 6N) was added, and the solution stirred at room temperature for 4 hours. The mixture was purified directly by ion exchange chromatography (DOWEX® 50WX8-200), eluting with a solvent gradient of deionised water: 0.88 ammonia (100:0 to 97:3), to give the title compound as a solid, 2.1g, 94% yield. This was triturated well with acetone, the supernatant removed, and the residual solid dried *in vacuo,* to give the title compound as a white solid.
¹H-NMR (D₂O, 400MHz) δ: 0.60 (t, 3H), 1.30 (m, 2H), 1.40 (m, 2H), 1.55 (m, 2H), 2.26-2.40 (m, 2H), 2.57 (dd, 1H), 2.76 (m, 2H), 3.68 (t, 2H), 6.66 (s, 1H). 7.36 (s, 1H).
HRMS : m/z 240.1699 (MH⁺), calcd 240.1706.
Anal. Found: C, 58.90; H, 8.90; N, 17.17. C₁₂H₂₁N₃O₂•0.3H₂O requires C, 58.88; H, 8.92; N, 16.99%.
[α]_{D} = +2.80 (*c* 0.14, deionised water)
[α]_{D} = -4.9 (*c* 0.16, methanol)
[α]_{D} =-5.0 (*c* 0.10, ethanol)

### Alternative method for Example 7

A slurry of the quinidine salt from preparation 110 (19g, 28.6mmol) in water (95ml) was adjusted to pH 10 using 5N sodium hydroxide solution, and the mixture extracted with dichloromethane (1x40ml, 2x20ml). The remaining aqueous suspension was acidified using 5N hydrochloric acid to pH 0.5, and the solution stirred at room temperature for 18 hours. The solution was purified on a Dowex® HCR-S ion-exchange resin column (40g), using an elution gradient of water:0.88 ammonia (100:0 to 97:3). The resulting foam was slurried with acetone (20ml), the solid filtered and dried *in vacuo* at 40°C to afford the title compound as a white solid, 4.6g, 68% yield.
¹H-NMR (CD₃OD 400MHz) δ: 0.87 (t, 3H), 1.50 (m, 1H), 1.58-1.72 (m, 3H), 1.78 (m, 2H), 2.44-2.59 (m, 2H), 2.90 (m, 3H), 3.88 (t, 2H), 6.84 (s, 1H), 7.46 (s, 1H).
LRMS : m/z 240 (MH⁺)
HRMS : m/z 240.1705 (MH⁺), calcd 240.1706.
Anal. Found: C, 49.10; H, 9.34; N, 14.31. C₁₂H₂₁N₃O₂•3H₂O requires C, 49.13; H, 9.28; N, 14.32%.

### Example 8

### (-)-(2R)-5-Amino-2-(1H-imidazol-4-ylmethyl)pentanoic acid

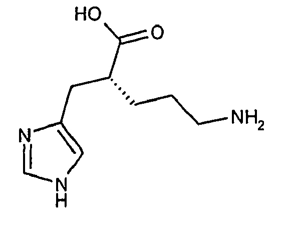

A mixture of the protected amine from Preparation 12 (85mg, 0.14mmol) in aqueous sodium hydroxide (1ml, 2N) and dioxane (1ml) was stirred at room temperature for 3 days. TLC analysis showed starting material remaining, so additional aqueous sodium hydroxide (1ml, 2N) was added, and the reaction stirred at 50°C for 18 hours. The mixture was cooled and treated with aqueous hydrochloric acid (5ml, 6N). The solution was then stirred at 80°C for 18 hours, cooled to room temperature, hexane added and the mixture stirred for an hour. The layers were separated, and the aqueous phase purified directly by ion exchange chromatography (DOWEX® 50WX8-200), eluting with a solvent gradient of deionised water : 0.88 ammonia (100:0 to 97:3), to give the title compound, 20mg, 73% yield.
¹H-NMR (CD₃OD 300MHz) δ: 1.40-1.68 (m, 4H), 2.45 (m, 1H), 2.62 (dd, 1H), 2.78 (m, 2H), 2.90 (m, 1H), 6.78 (s, 1H), 7.50 (s, 1H).
HRMS : m/z 198.1243 (MH⁺), calcd 198.1237.
[α]_{D} = -6.0 (c 0.1 mg/ml, deionised water)

### Example 9

### (+)-(2S)-5-Amino-2-(1H-imidazol-4-ylmethyl)pentanoic acid

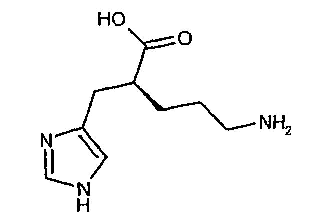

The title compound was obtained in 96% yield from the protected amine from Preparation 13, following the procedure described in Example 8.
¹H-NMR (CD₃OD 300MHz) δ: 1.45 (m, 1H), 1.59 (m, 3H), 2.47 (m, 1H), 2.62 (dd, 1H), 2.78 (m, 2H), 2.90 (dd, 1H), 6.80 (s, 1H), 7.50 (s, 1H).
HRMS : m/z 220.1064 (MNa⁺), calcd 220.1056.

### Example 10

### (±)-5-Amino-2-[(4-n-propyl-1H-imidazol-2-yl)methyl]pentanoic acid

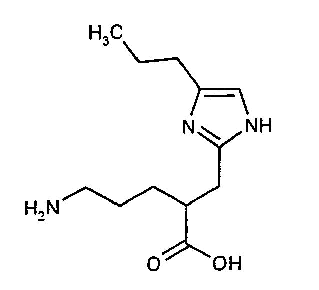

A mixture of the protected amine from Preparation 14 (108mg, 0.23mmol) in aqueous hydrochloric acid (1.5ml, 6N) was stirred under reflux for 1.5 hours. The cooled solution was purified directly by ion exchange chromatography (DOWEX® 50WX8-200), eluting with a solvent gradient of deionised water : 0.88 ammonia (100:0 to 96:4), to give the title compound as a white solid, 30mg, 55% yield.
¹H-NMR (CD₃OD 400MHz) δ: 0.95 (t, 3H), 1.45 (m, 1H), 1.62 (m, 5H), 2.48 (t, 2H), 2.58 (m, 1H), 2.76 (dd, 1H), 2.86 (m, 2H), 2.98 (dd, 1H), 6.60 (s, 1H).
HRMS : m/z 240.1718 (MH⁺), calcd 240.1707.
Anal. Found: C, 54.04; H, 8.97; N, 15.68. C₁₂H₂₁N₃O₂•1.5H₂O requires C, 54.12; H, 9.08; N, 15.78%.

### Example 11

### (2S)-2-[(2-Aminoethyl)amino]-3-(1H-imidazol-4-yl)propanoic acid

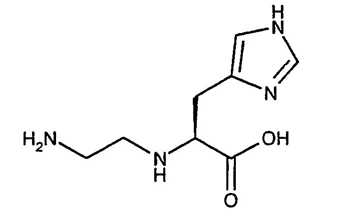

Trifluoroacetic acid (17ml) was added dropwise to a stirred solution of the product from Preparation 16 (2.58g, 8.2mmol) in methanol : water (27ml : 14ml). The reaction was slightly exothermic with evolution of carbon dioxide gas. The mixture was stirred at room temperature for 4 hours and the solvent was removed by evaporation under reduced pressure to give a colourless oil which was dried *in vacuo* overnight. The resultant oil was treated with aqueous sodium hydroxide solution (1N) until solution was at pH=8. A further portion of aqueous sodium hydroxide solution (1N, 30ml) was added and the solution was stirred at room temperature for 72 hours. The solution was concentrated under reduced pressure to 10ml and purified by ion exchange chromatography (DOWEX® 50WX8-200) eluting with a solvent gradient of deionised water : 0.88 ammonia solution (100:0 to 97:3). The solvent was removed by evaporation under reduced pressure to afford a yellow oil which was dissolved in deionised water (15ml) and freeze-dried overnight to afford a foam. This material was dissolved in deionised water : methanol (95:5) and further purified using MCI^{™} gel (55g) chromatography, eluting with a solvent gradient of deionised water: methanol (95:5) to afford the title compound, 1.13g, 69% yield.
¹H-NMR (D₂O, 300 MHz) δ: 2.61-2.87 (m, 4H), 2.92 (m, 2H), 3.25 (t, 1H), 6.81 (s, 1H), 7.59 (s, 1H).
LRMS : m/z 199.2 (MH⁺)
Anal. Found: C, 43.36; H, 7.51; N, 25.12. C₈H₁₄N₄O₂•1.3H₂O requires C, 43.35; H, 7.54; N, 25.28%.
[α]_{D} = +1.74 (c 0.12, deionised water)

### Example 12

### (2R)-2-[(2-Aminoethyl)amino]-3-(1H-imidazol-4-yl)propanoic acid

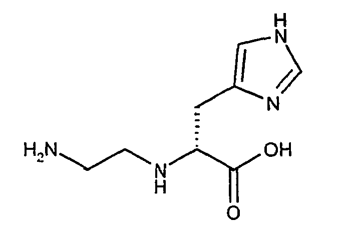

The title compound was prepared from the product of Preparation 17 using the procedure described for Example 11.
¹H-NMR (D₂O, 300 MHz) δ: 2.57-2.82 (m, 4H), 2.89 (m, 2H), 3.22 (t, 1H), 6.77 (s, 1H), 7.55 (s, 1H).
[α]_{D} = -1.0(c 0.10, deionised water)

### Example 13

### (±)-2-[(2-Aminoethyl)amino]-3-(1H-imidazol-2-yl)propanoic acid

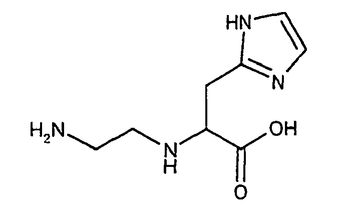

Trifluoroacetic acid (0.5ml) was added dropwise to a stirred solution the product from Preparation 18 (105mg, 0.34mmol) in methanol : water (2ml : 1ml) and the mixture was stirred at room temperature for 4 hours. The solvent was then removed by evaporation under reduced pressure and the residue was treated with aqueous sodium hydroxide solution (1 N) until solution was at pH=7. A further portion of aqueous sodium hydroxide solution (1N, 5ml) was added and the solution was stirred at room temperature for 72 hours. The reaction solution was then submitted to ion exchange chromatography (DOWEX® 50WX8-200) eluting with deionised water : 0.88 ammonia (97:3). The solvent was removed by evaporation under reduced pressure to afford a white solid residue. This material was dissolved in deionised water : methanol (95:5) and was further purified using MCI^{™} gel chromatography, eluting with deionised water:methanol (95:5) to afford the title compound, 4mg, 6% yield.
¹H-NMR (CD₃OD, 300 MHz) δ: 2.74-2.98 (m, 4H), 3.13 (m, 1H), 3.35 (m, 2H), 6.95 (s, 2H).

### Example 14

### (2S)-2-[(2-Aminoethyl)amino]-3-(1H-imidazol-2-yl)propanoic acid

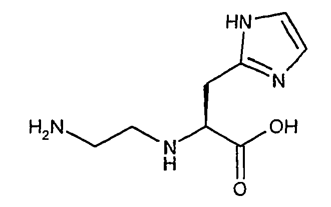

The product from Preparation 19 (200mg, 0.45mmol) was treated with aqueous hydrochloric acid (6N, 4ml) and heated at reflux for 3 hours. The solvent was then removed by evaporation under reduced pressure and the residue was purified by ion exchange chromatography (DOWEX® 50WX8-200) eluting with an elution gradient of deionised water: 0.88 ammonia (100:0 to 97:3). The isolated material was then freeze-dried to afford the title compound as a foam, 62mg, 69% yield.
¹H-NMR (CD₃OD 300 MHz) δ: 2.71-2.98 (m, 4H), 3.13 (m, 1H), 3.34 (m, 2H), 6.92 (s, 2H).
HRMS: m/z 199.1184 (MH⁺), calcd 199.1190.

### Example 15

### (2S)-2-{[(1R or S)-1-(Aminomethyl)propyl]amino}-3-(1H-imidazol-4-yl)propanoic acid

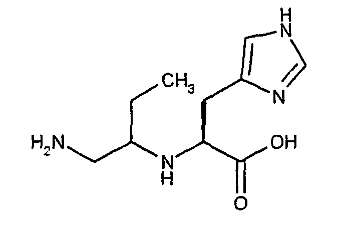

Trifluoroacetic acid was added dropwise to a stirred solution of the product from Preparation 21 (91mg, 0.26mmol) in dichloromethane (1ml) and the mixture was stirred at room temperature for 17 hours under a nitrogen atmosphere. The solvent was then removed by evaporation under reduced pressure and the residue was azeotroped with toluene. The resultant material was dissolved in aqueous sodium hydroxide solution (5ml, 2N) and stirred at room temperature for 72 hours. Solution was then purified by ion exchange chromatography (DOWEX® 50WX8-200), eluting with a solvent gradient of deionised water : 0.88 ammonia (100:0 to 95:5), to afford the title compound, 37.3mg, 62% yield.
¹H-NMR (CD₃OD 400 MHz) δ: 0.81 (t, 3H), 1.37 (m, 1H), 1.50 (m, 1H), 2.62 (m, 1H), 2.67 (m, 1H), 2.78(m, 1H), 2.90 (dd, 1H), 2.98 (dd, 1H), 3.33 (dd, 1H), 6.87 (s, 1H). 7.57 (s, 1H).
HRMS : m/z 227.1511 (MH⁺), calcd 227.1503.

### Example 16

### (2S)-2-{[(1S or R)-1-(Aminomethyl)propyl]amino]-3-(1H-imidazol-4-yl)propanoic acid

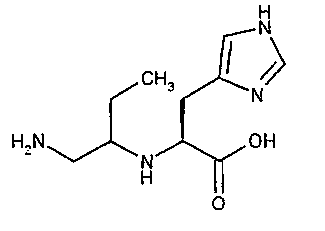

Trifluoroacetic acid was added dropwise to a stirred solution the product from Preparation 22 (167mg, 0.49mmol) in dichloromethane (1ml) and the mixture was stirred at room temperature for 17 hours under a nitrogen atmosphere. Solvent was removed by evaporation under reduced pressure and residue azeotroped with toluene. The resultant material was dissolved in aqueous sodium hydroxide solution (5ml, 2N) and stirred at room temperature for 72 hours. Solution was then purified by ion exchange chromatography (DOWEX® 50WX8-200) eluting with a solvent gradient of deionised water : 0.88 ammonia (100:0 to 95:5) to afford the title compound, 38.7mg, 35% yield.
¹H-NMR (CD₃OD 400 MHz) δ: 0.73 (t, 3H), 1.35 (m, 2H), 2.43 (m, 1H), 2.53 (t, 1H), 2.70 (m, 1H), 2.95 (dd, 1H), 3.10 (dd, 1H), 3.40 (dd, 1H), 6.90 (s, 1H), 7.60 (s, 1H).
HRMS : m/z 227.1500 (MH⁺), calcd 227.1502.

### Example 17

### (2S)-2-{[(1RS)-1-(Aminomethyl)-2-methylpropyl]amino}-3-(1H-imidazol-4-yl)propanoic acid

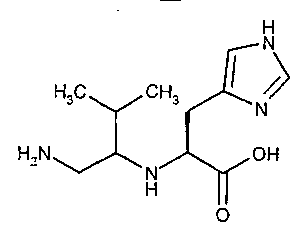

Trifluoroacetic acid (2ml) was added to a stirred solution of the product from Preparation 23 (100mg, 0.28mmol) in dichloromethane (1ml) and the mixture was stirred at room temperature for 17 hours. The solvent was then removed by evaporation under reduced pressure and the residue azeotroped with toluene. The residue was then dissolved in aqueous sodium hydroxide solution (2M, 2ml) and stirred at room temperature for 72 hours. The solution was then purified by ion exchange chromatography (DOWEX® 50WX8-200) eluting with a solvent gradient of deionised water : 0.88 ammonia (100:0 to 97:3). The isolated material (35mg) was further purified by chromatography on reverse phase silica gel (C18 Sep-Pak®), elating with deionised water, and then freeze-dried to afford the title compound (mixture of diastereoisomers), 20mg, 30% yield.
¹H-NMR (CD₃OD 300 MHz), mixture of diastereoisomers, δ: 0.67-0.90 (4x d, 6H), 2.40-3.40 (m, 7H), 6.85-6.95 (2x s, 1H), 7.72-7.62 (2x s, 1H).
HRMS : m/z 241.1661 (MH⁺), calcd 241.1659.
TLC : methanol : ethyl acetate : 0.88 ammonia : acetic acid : water (60 : 12 : 4 : 4 :8) Rf = 0.52 and 0.44.

### Example 18

### (2S)-2-{[(1RS)-2-Amino-1-benzylethyl]amino}-3-(1H-imidazol-4-yl)propanoic acid

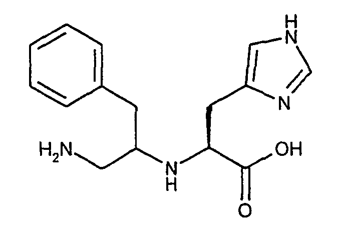

Trifluoroacetic acid (2ml) was added to a stirred solution of the product from Preparation 24 (100mg, 0.25mmol) in dichloromethane (1ml) and stirred at room temperature for 17 hours. The solvent was then removed by evaporation under reduced pressure and the residue azeotroped with toluene. The residue was dissolved in aqueous sodium hydroxide solution (2N, 2ml) and stirred at room temperature for 17 hours. The solution was then purified by ion exchange chromatography (DOWEX® 50WX8-200) eluting with a solvent gradient of deionised water: 0.88 ammonia (100:0 to 97:3) and isolated material was freeze-dried to afford the title compound, 41 mg, 58% yield.
¹H-NMR (CD₃OD, 300 MHz) δ: 2.48-2.72 (m, 2H), 2.77-3.10 (m, 3H), 3.25-3.47 (2x m, 1H), 3.31 (d, 2H), 6.80 (2x s, 1H), 6.91 (d, 1H), 7.10-7.30 (m, 4H), 7.55-7.63 (2x s, 1H).
HRMS : m/z 289.1662 (MH⁺), calcd 289.1659.

### Example 19

### (2S)-3-(1H-Imidazol-4-yl)-2-[(3RS)-pyrrolidinylamino)]propanoic acid

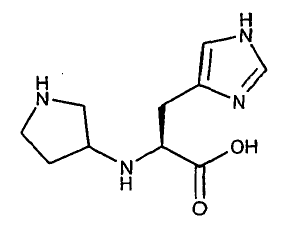

Aqueous sodium hydroxide solution (1.7ml, 5N) was added dropwise to a stirred solution of the product from Preparation 20 (200mg, 0.8mmol) in deionised water (20ml) and the solution was stirred at room temperature overnight. The solution was then purified by ion exchange chromatography (DOWEX® 50WX8-200) eluting with a solvent gradient of deionised water : 0.88 ammonia (100:0 to 95:5) to afford the title compound as a pink foam, 90mg, 50% yield.
¹H-NMR (D₂O, 300 MHz), mixture of diastereoisomers, δ: 1.67 (m, 1H), 2.05 (m, 1H), 2.70 (m, 2H), 2.90 (m, 1H). 3.05-3.38 (m, 5H), 6.69 (s, 1H). 7.59 (s, 1H).
LRMS : m/z 225.3 (MH⁺)
[α]_{D} = +1.57 (*c* 0.076, deionised water)

### Example 20

### (2S)-2-{[(1R,2S)-2-Amino-1-methylpropyl]amino}-3-(1H-imidazol-4-yl)propanoic acid

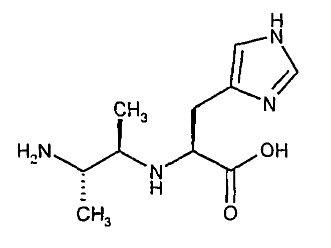

Aqueous sodium hydroxide solution (2ml, 2N) was added to a stirred solution of the product from Preparation 26 (260mg, 7.64mmol) in dioxane (2ml) and the mixture was stirred for 2.5 hours at room temperature. Aqueous hydrochloric acid (50% by volume, 4ml) was added and the mixture was stirred at room temperature for 17 hours. The solution was then purified by ion exchange chromatography (DOWEX® 50WX8-200) eluting with a solvent gradient of deionised water : 0.88 ammonia (100:0 to 97:3) to afford a white solid which was dissolved in deionised water and further purified by chromatography on reverse phase silica gel (C18 Sep-Pak®), eluting with deionised water, to afford the title compound, 15mg, 9% yield.
¹H-NMR (CD₃OD 300 MHz) δ: 0.93 (d, 3H), 1.17 (d, 3H), 2.62-2.80 (m, 2H), 3.08 (m, 1H), 3.20 (m, 1H), 3.37 (m, 1H), 6.92 (s, 1H), 7.61 (s, 1H).
HRMS : m/z 227.1506 (MH⁺), calcd 227.1502.

### Example 21

### (2S)-2-[(2-Aminoethyl)(methyl)amino]-3-(1H-imidazol-4-yl)propanoic acid

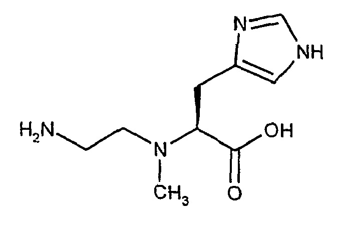

Trifluoroacetic acid (10ml) was added to a stirred solution of the product from Preparation 27 (900mg, 2.8 mmol) in methanol : deionised water (10ml : 8ml) and the mixture was stirred for 2 hours. The solvent was removed by evaporation under reduced pressure to afford a light brown oil which was dissolved in excess aqueous sodium hydroxide solution (1N) and stirred for 17 hours. The solution was concentrated under reduced pressure and purified by ion exchange chromatography (DOWEX® 50WX8-200) eluting with a solvent gradient of deionised water : 0.88 ammonia (100:0 to 96:4) to afford the title compound as a white foam, 381 mg, 60% yield.
¹H-NMR (D₂O, 300 MHz) δ: 2.25 (s, 3H), 2.50 (m, 1H), 2.60-3.37 (m, 6H), 6.78 (s, 1H), 7.58 (s, 1H).

### Example 22

### (2S)-3-(1H-imidazol-4-yl)-2-(1-piperazinyl)propanoic acid

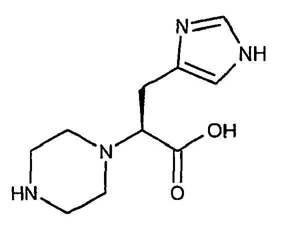

Aqueous sodium hydroxide solution (5N, 170µl) was added to a stirred solution of the product from Preparation 28 (50mg, 0.012mmol) in water (a few drops) and the solution was stirred at room temperature for 18 hours. The solution was then submitted to ion exchange chromatography (DOWEX® 50WX8-200), eluting with a solvent gradient of deionised water : 0.88 ammonia (100:0 to 95:5), and the solvent then removed by evaporation under reduced pressure. The residue was suspended in diethyl ether and then re-evaporated to afford the title compound as a white solid, 17mg, 73% yield.
¹H-NMR (D₂O, 300 MHz) δ: 2.62-2.98 (m, 6H), 3.05-3.30 (m, 5H), 6.80 (s, 1H), 7.60 (s, 1H).
HRMS : m/z 225.1338 (MH⁺), calcd 225.1346.
[α]_{D} = +14.84 (*c* 0.062, deionised water)
TLC : methanol : ethyl acetate : 0.88 ammonia : acetic acid : water (60 : 12 : 4 : 4 : 8) Rf = 0.20.

### Example 23

### (2S)-2-(1,4-Diazepan-1-yl)-3-(1H-imidazol-4-yl)propanoic acid

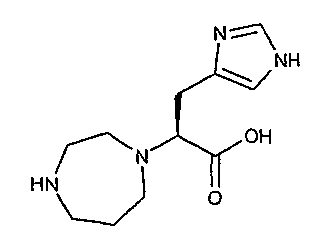

Homopiperazine (1.86g, 18.6mmol) was added to a stirred solution of the product from Preparation 61 (350mg, 1.86mmol) in acetonitrile (40ml) and the solution was stirred for 2 hours at room temperature then heated at reflux for 18 hours. The solvent was removed under reduced pressure and the residue was dissolved in dichloromethane and washed with water (3x20ml). The organic phase was concentrated under reduced pressure and the resultant oil was dissolved in deionised water and purified by ion exchange chromatography (DOWEX® 50WX8-200) eluting with a solvent gradient of deionised water : 0.88 ammonia (100:0 to 95:5) to afford the title compound as a beige solid, 300mg, 68% yield.
¹H-NMR (D₂O, 300 MHz) δ: 1.83 (m, 2H), 2.70-3.23 (m, 10H), 3.40 (t, 1H), 6.80 (s, 1H), 7.60 (s, 1H).
LRMS : m/z 239.2 (MH⁺)
Anal. Found: C, 50.79; H, 7.85; N, 21.31. C₁₁H₁₈N₄O₂•1.25H₂O requires C, 50.66; H, 7.92; N, 21.48%.
[α]_{D} = +2.47 (*c* 0.24, deionised water)

### Example 24

### (2S)-2-[(2-Aminoethyl)amino]-3-(1-ethyl-1H-imidazol-4-yl)propanoic acid

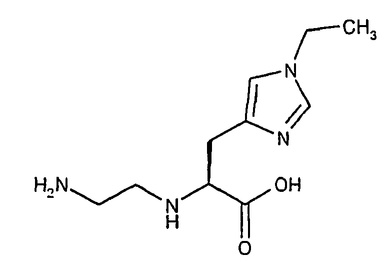

Concentrated hydrochloric acid (5ml) was added to a stirred solution of the product from Preparation 30 (118mg, 0.32mmol) in water (5ml) and the mixture was heated at reflux for 17 hours. The mixture was allowed to cool to room temperature and the solvent was removed by evaporation under reduced pressure. The residue was purified by ion exchange chromatography (DOWEX® 50WX8-200) eluting with deionised water : 0.88 ammonia (97:3). The isolated material was freeze-dried to afford the title compound, 34mg, 47% yield.
¹H-NMR (CD₃OD, 300 MHz) δ: 1.40 (t, 3H), 2.75-3.02 (m, 6H), 3.33 (m, 1H), 3.98 (q, 2H), 6.95 (s, 1H), 7.53 (s, 1H).
HRMS : m/z 227.1492 (MH⁺), calcd 227.1503.

### Examples 25 - 40

The compounds of the following tabulated Examples of the general formula: 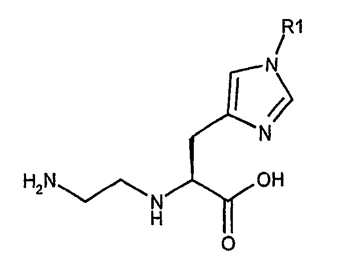 were prepared by a similar method to that of Example 24 using the corresponding products from Preparations 31-46.

| Example No. | R1 | Yield (%) | Analytical Data |
|---|---|---|---|
| 25 ¹ | 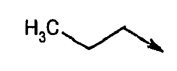 | 55 | ¹H-NMR (CD₃OD, 300 MHz) δ: 0.90 (t, 3H), 1.78 (q, 2H), 2.77-3.01 (m, 6H), 3.31 (m, 1H), 3.90 (t, 2H), 6.90 (s, 1H), 7.52 (s, 1H). |
| | | | LRMS : m/z 241.1 (MH⁺) |
| 26 ¹ | 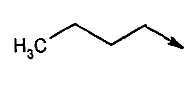 | 82 | ¹H-NMR (CD₃OD, 300 MHz) δ 0.70 (t, 3H), 1.05 (q, 2H), 1.57 (m, 2H), 2.57-2.73 (m, 4H), 2.85 (m, 2H), 3.08 (t, 1H), 3.78 (t, 2H), 6.78 (s, 1H), 7.42 (s, 1H). |
| | | | HRMS : m/z 255.1824 (MH⁺), calcd_255.1816. |
| | | | Anal. Found: C, 55.79; H, 8.65; N, 21.96. C₁₂H₂₂N₄O₂•0.22H₂O requires C, 55.80; H, 8.76; N, 21.69%. |
| 27 ¹ | 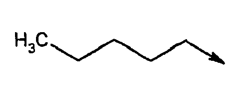 | 38 | ¹H-NMR (CD₃OD, 300 MHz) δ: 0.90 (t, 3H), 1.20-1.40 (m, 4H), 1.77 (m, 2H), 2.78- 3.05 (m, 6H), 3.30 (m, 1H), 3.93 (t, 2H), 6.93 (s, 1H), 7.57 (s, 1H). |
| | | | HRMS : m/z 269.1978 (MH⁺), calcd 269.1972. |
| | | | Anal. Found: C, 54.21; H, 8.83; N, 19.32. C₁₃H₂₄N₄O₂•1.2H₂O requires C, 53.85; H, 9.18; N, 19.32%. |
| 28 ² | 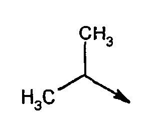 | 96 | ¹H-NMR (CD₃OD 300 MHz) δ: 1.45 (d, 6H), 2.72-3.03 (m, 6H), 3.33 (m, 1H), 4.33 (m, 1H), 7.00 (s, 1H), 7.58 (s, 1H). |
| | | | HRMS : m/z 241.1662 (MH⁺), calcd 241.1659. |
| 29 ² | 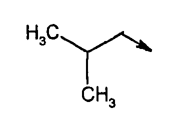 | 54 | ¹H-NMR (CD₃OD, 300 MHz) δ: 0.90 (d, 6H), 2.01 (m, 1H), 2.77-3.03 (m, 6H), 3.33 (m, 1H), 3.77 (d, 2H), 6.90 (s, 1H), 7.50 (s, 1H). |
| | | | HRMS : m/z 255.1825 (MH⁺), calcd 255.1816. |
| 30 ¹ | 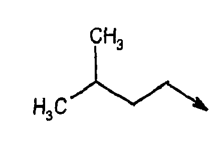 | 65 | ¹H-NMR (CD₃OD 300 MHz) δ: 0.93 (d, 6H), 1.55 (m, 1H),1.67 (m, 2H), 2.73-3.05 (m, 6H), 3.33 (m, 1H), 3.97 (t, 2H), 6.93 (s, 1H), 7.53 (s, 1H). |
| | | | HRMS : m/z 269.1980 (MH⁺), calcd 269.1972. |
| | | | Anal. Found: C, 51.94; H, 8.99; N, 18.53. C₁₃H₂₄N₄O₂•1.80H₂O requires C, 51.60; H, 9.26; N, 18.52%. |
| 31 ^{1,3} | 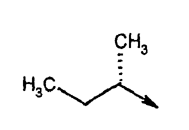 | 3 | ¹H-NMR (D₂O, 400 MHz) δ: 0.66 (t, 3H), 1.34 (d, 3H), 1.66 (m, 2H), 2.66-2.84 (m, 4H), 2.95 (m, 2H), 3.29 (t, 1H), 4.05 (m, 1H), 6.94 (s, 1H), 7.61 (s, 1H). |
| | | | HRMS : m/z 255.1827 (MH⁺), calcd 255.1816. |
| 32 ^{1,4} | 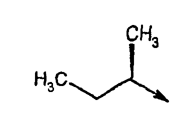 | 27 | ¹H-NMR (D₂O, 400 MHz) δ: 0.66 (t, 3H), 1.35 (d, 3H), 1.66 (m, 2H), 2.68-2.82 (m, 4H), 2.94 (m, 2H), 3.29 (t, 1H), 4.05 (m, 1H), 6.94 (s, 1H), 7.60 (s, 1H). |
| | | | HRMS : m/z 255.1825 (MH⁺), calcd 255.1816. |
| 33 | 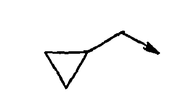 | 10 | ¹H-NMR (CD₃OD, 300 MHz) δ: 0.37 (m, 2H), 0.60 (m, 2H), 1.20 (m, 1H), 2.75-3.03 (m, 6H), 3.33 (m, 1H), 3.80 (d, 2H), 7.00 (s, 1H), 7.58 (s, 1H). |
| | | | HRMS : m/z 253.1661 (MH⁺), calcd 253.1659. |
| 34 ¹ | 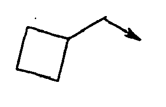 | 53 | ¹H-NMR (CD₃OD, 300 MHz) δ: 1.70-2.10 (m, 6H), 2.60-3.10 (m, 7H), 3.35 (m, 1H), 3.95 (d, 2H), 6.90 (s, 1H), 7.50 (s, 1H). HRMS : m/z 267.1822 (MH⁺), calcd 267.1816. |
| | | | Anal. Found: C, 53.74; H, 8.43; N, 19.30. C₁₃H₂₂N₄O₂•1.3H₂O requires C, 53.92; H, 8.50; N, 19.34%. |
| 35 | 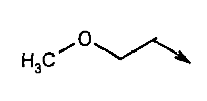 | 2.5 | ¹H-NMR (CD₃OD 300 MHz) δ: 2.75-2.93 (m, 5H), 2.98 (dd, 1H), 3.33 (s+m, 4H), 3.62 (t, 2H), 4.10 (t, 2H), 6.95 (s, 1H), 7.53 (s, 1H). |
| | | | HRMS : m/z 257.1607 (MH⁺), calcd 257.1608. |
| 36 ¹ | 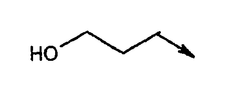 | 55 | ¹H-NMR (D₂O, 300 MHz) δ: 1.90 (m, 2H), 2.64-2.82 (m, 4H), 2.97 (m, 2H), 3.28 (t, 1H), 3.44 (t, 2H), 3.98 (t, 2H), 6.88 (s, 1H), 7.57 (s, 1H). |
| | | | HRMS : m/z 257.1618 (MH⁺), calcd 257.1608. |
| | | | Anal. Found: C, 47.43; H, 7.81; N, 19.98. C₁₁H₂₀N₄O₃•1.3 H₂O requires C, 47.23; H, 8.14; N, 20.03%. |
| 37 ² | 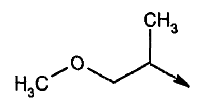 | 28 | ¹H-NMR (CD₃OD 400 MHz) δ: 1.43 (d, 3H), 2.82 (m, 3H), 2.90 (m, 2H), 3.00 (dd, 1H), 3.30 (s, 3H), 3.35 (m, 1H), 3.55 (d, 2H), 4.38 (q, 1H), 7.00 (s, 1H), 7.58 (s, 1H). |
| | | | HRMS : m/z 271.1770 (MH⁺), calcd 271.1765. |
| 38 ¹ | 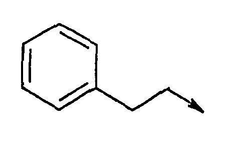 | 42 | ¹H-NMR (CD₃OD 400MHz) δ: 2.61-3.13 (m, 8H), 3.31 (m, 1H), 4.16 (t, 2H), 6.90 (s, 1H), 7.11 (m, 2H), 7.13-7.40 (m, 4H). |
| | | | HRMS : m/z 303.1823 (MH⁺), calcd 303.1816. |
| | | | Anal. Found: C, 58.13; H, 7.51; N, 17.06. C₁₆H₂₂N₄O₂•1.6H₂O requires C, 58.02; H, 7.67; N, 16.92%. |
| 39 | 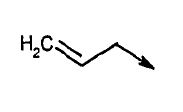 | 64 | ¹H-NMR (CD₃OD 400MHz) δ: 2.78-3.01 (m, 5H), 3.34 (m, 2H), 4.58 (d, 2H), 5.19 (d, 1H), 5.23 (d, 1H), 5.99 (m, 1H), 6.92 (s, 1H), 7.54 (s, 1H). |
| | | | HRMS : m/z 239.1510 (MH⁺), calcd 239.1503. |
| | | | Anal. Found: C, 50.64; H, 7.56; N, 21.03. C₁₁H₁₈N₄O₂•1.3H₂O requires C, 50.48; H, 7.93; N, 21.41%. |
| 40 | 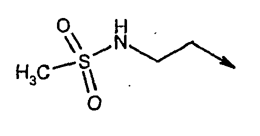 | 14 | ¹H-NMR (CD₃OD, 400MHz) δ: 2.78-3.00 (m, 9H), 3.35 (m, 1H), 3.39 (t, 2H), 4.06 (t, 2H), 6.97 (s, 1H), 7.58 (s, 1H). |
| | | | HRMS m/z 320.1391 (MH⁺), calcd 320.1387. |

| | | | |
|---|---|---|---|
| Footnotes: 1. Concentrated sulfuric acid (4M) used instead of concentrated hydrochloric acid (6M). 2. Sulfuric acid (2M) used instead of concentrated hydrochloric acid (6M). 3. The isolated product was further purified using a 5µm Hypersil Hypercarb™ column, using an elution gradient of water : trifluoroacetic acid : acetonitrile (100 : 0.1 : 0 to 50 : 0.05 : 50), and then re-subjected to ion-exchange chromatography (as in Example 24). 4. The isolated product was further purified as described in note (3) but using an elution gradient of water: trifluoroacetic acid : methanol (100:0.1:0 to 50:0.05:50). | | | |

### Example 41

### (2S)-2-[(2-Aminoethyl)amino]-3-[1-(carboxymethyl)-1H-imidazol-4-yl]propanoic acid

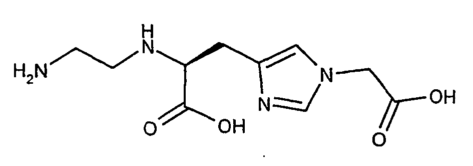

The product from Preparation 47 (145mg, 0.296mmol) was dissolved in concentrated sulfuric acid (4ml) and the solution heated under reflux for 18 hours. The cooled mixture was purified directly by ion exchange chromatography (DOWEX® 50WX8-200), eluting with 0.88 ammonia : water (3:97). The resulting oil was triturated with methanol, to give a solid which was freeze-dried to afford the title compound as a white foam, 61 mg, 77% yield.
¹H-NMR (D₂O, 400MHz) δ: 2.80 (m, 2H), 2.88 (m, 2H), 2.98 (m, 2H), 3.40 (m, 1H), 4.52 (s, 2H), 6.92 (s, 1H), 7.81 (s, 1H).
HRMS : m/z 257.1255 (MH⁺), calcd 257.1245.
Anal. Found: C, 42.66; H, 6.63; N, 20.29. C₁₀H₁₆N₄O₄•1.3H₂O requires C, 42.95; H, 6.70; N, 20.03%.

### Example 42

### (2S)-3-[(1-n-propyl-1H-imidazol-4-yl)methyl]-2-piperidinone

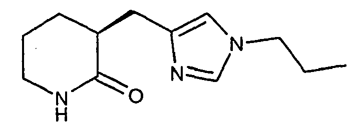

The compound from Preparation 11 (500mg, 1.6mmol) in dichloromethane (15ml) was treated with trifluoroacetic acid (3ml) and the resultant solution was stirred at room temperature for 2 hours. The reaction mixture was then concentrated under reduced pressure and the residue neutralised with saturated aqueous sodium bicarbonate solution. The resultant mixture was then concentrated to dryness under reduced pressure and the residue purified by column chromatography on silica gel using an elution gradient of dichloromethane:methanol : 0.88 ammonia (99.8 : 0 : 0.2 to 94.8 : 5 : 0.2) to give the title compound as an oil, 250mg, 73% yield.
¹H-NMR (CDCl₃, 400MHz) δ: 0.87 (t, 3H), 1.39-1.84 (m, 5H), 1.90 (m, 1H), 2.60 (m, 1H), 2.74 (dd, 1H), 3.13 (dd, 1H), 3.21 (m, 2H), 3.77 (t, 2H), 5.61 (br s, 1H), 6.65 (s, 1H), 7.31 (s, 1H).
LRMS : m/z 222 (MH⁺)
Anal. Found: C, 61.44; H, 8.85; N, 17.86. C₁₂H₁₉N₃O•0.75H₂O requires C, 61.38; H, 8.80; N, 17.89%.
[α]_{D} = -51.6 (*c* 0.095, methanol)

### Example 43

### (2S)-2-[(2-Aminoethyl)amino]-3-(1-methyl-1H-imidazol-4-yl)propanoic acid

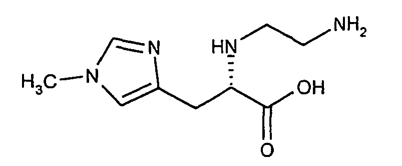

2M Sodium hydroxide solution (0.61ml, 1.22mmol) was added to a solution of the protected amino acid from preparation 90 (200mg, 0.61 mmol) in dioxan (2ml), and the reaction stirred at room temperature for 18 hours. Concentrated hydrochloric acid (2ml) was carefully added, and the solution stirred for a further 24 hours, then concentrated under reduced pressure. The residue was dissolved in water, and purified by column chromatography on Amberlyst® 15 ion-exchange resin, eluting with 5% aqueous ammonia solution. The product was obtained after freeze-drying as a gum, 80mg, 55% yield.
¹H-NMR (D₂O, 400MHz) δ: 2.61-2.79 (m, 4H), 2.90 (m, 2H), 3.22 (m, 1H), 3.54 (s, 3H), 6.79 (s, 1H), 7.42 (s, 1H).
LRMS (ES⁻) : m/z 211 (M-H)-
[α]_{D} = -5.83 (*c* 0.12, methanol)
Anal Found: C, 45.63; H, 7.68; N, 23.15. C₉H₁₆N₄O₂•1.45H₂O requires C, 45.35; H, 7.99; N, 23.50%.

### Examples 44 to 47

The following examples of general structure: 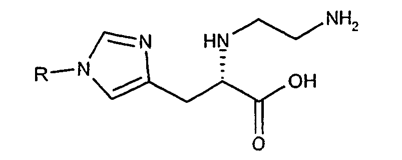 were prepared from the appropriate protected amino acids (Preparations 91-94), following a similar procedure to that described in Example 43.

| Example | R | Yield (%) | Data |
|---|---|---|---|
| 44¹ | 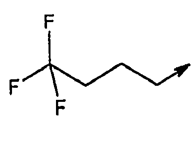 | 48 white solid | ¹H-NMR (D₂O, 400MHz) δ: 1.99 (m, 2H), 2.06 (m, 2H), 2.68-2.83 (m, 4H), 2.98 (t, 2H), 3.32 (t, 1H), 3.98 (t, 2H), 6.90 (s, 1H), 7.58 (s, 1H). |
| | | | LRMS : m/z 309 (MH⁺) |
| | | | [α]_{D} = -0.75 (*c* 0.16, methanol) |
| | | | Anal. Found: C, 45.25; H, 6.31; N, 17.53. C₁₂H₁₉F₃N₄O₂•0.5H₂O requires C, 45.42; H, 6.35; N, 17.66%. |
| 45² | 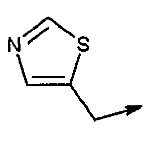 | 49 white solid | ¹H-NMR (D₂O, 400MHz) δ: 2.63-2.81 (m, 4H), 2.95 (m, 2H), 3.24 (m, 1H), 5.21 (s, 2H), 6.85 (s, 1H), 7.41 (s, 1H), 7.62 (s, 1H), 8.84 (s, 1H). |
| | | | LRMS (ES⁻): m/z 294 (M-H)⁻ |
| | | | [α]_{D} = -5.00 (*c* 0.10, methanol) |
| | | | Anal. Found: C, 46.27; H, 5.81; N, 21.91. C₁₂H₁₇N₅O₂S•1.0H₂O requires C, 45.94; H, 6.12; N, 22.32%. |
| 46 | 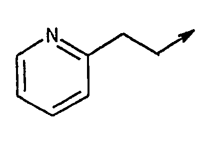 | 52 solid | ¹H-NMR (D₂O, 400MHz) δ: 2.28 (dd, 1H), 2.68 (m, 3H), 2.89 (t, 2H), 3.08 (t, 2H), 3.18 (t, 1H), 4.21 (t, 2H), 6.70 (s, 1H), 6.99 (d, 1H), 7.12 (s, 1H), 7.18 (dd, 1H), 7.60 (dd, 1H), 8.30 (d, 1H). |
| | | | LRMS : m/z 326 (MNa⁺) |
| | | | [α]_{D} = -4.17 (*c* 0.12, methanol) |
| | | | Anal. Found: C, 56.28; H, 7.15; N, 21.68. C₁₅H₂₁N₅O_{2•}1.0H₂O requires C, 56.06 H, 7.21; N, 21.79%. |
| 47 | 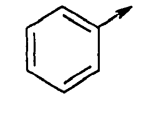 | 65 beige solid | ¹H-NMR (D₂O, 400MHz) δ: 2.70-2.90 (m, 4H), 2.95 (m, 2H), 3.35 (t, 1H), 7.22 (s, 1H), 7.32 (m, 1H), 7.41 (m, 4H), 7.92 (s, 1H). |
| | | | LRMS : m/z 297 (MNa⁺) |
| | | | [α]_{D} = +8.45 (*c* 0.09, methanol) |
| | | | Anal. Found: C, 55.68; H, 6.95; N, 18.29. C₁₄H₁₈N₄O₂•1.5H₂O requires C, 55.80; H, 7.02; N, 18.59%. |

| | | | |
|---|---|---|---|
| 1 = water was used as the column eluant 2 = product purified on DOWEX® 50WX8-200 ion-exchange resin, using water:0.88 ammonia (95:5) as eluant | | | |

### Example 48

### (2S)-2-[(2-Aminoethyl)amino]-3-(1-benzyl-1H-imidazol-4-yl)propanoic acid

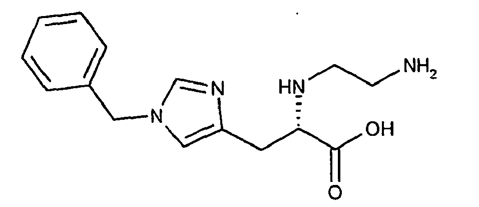

A solution of the compound from preparation 95 (288mg, 0.57mmol) in 4M sulphuric acid (10ml), was heated at 115°C for 36 hours. The cooled solution was neutralised using 1 M sodium hydroxide solution, then passed through an Amberlyst® 15 ion exchange column, eluting with 5% aqueous ammonia. The product was obtained as a gum after freeze-drying, 70mg, 39% yield.
¹H-NMR (D₂O, 400MHz) δ : 2.40 (m, 1H), 2.48 (m, 1H), 2.58 (m, 4H), 3.14 (t, 1H), 5.00 (s, 2H), 6.77 (s, 1H), 7.14 (d, 2H), 7.22 (m, 3H), 7.50 (s, 1H).
LRMS : m/z 289 (MH⁺)
[α]_{D} = +1.00 (*c* 0.14, methanol)
Anal. Found: C, 56.96; H, 7.17; N, 17.63. C₁₅H₂₀N₄O₂•1.5H₂O requires C, 57.13; H, 7.35; N, 17.77%.

### Example 49

### (±)-5-Amino-2-[(1-isopentyl-1H-imidazol-4-yl)methyl]pentanoic acid

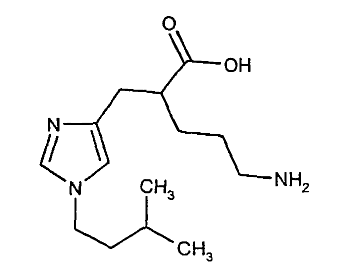

A solution of sodium hydroxide (192mg, 4.80mmol) in water (6ml) was added to a solution of the compound from preparation 105 (420mg, 1.20mmol) in tetrahydrofuran (10ml), and the reaction stirred vigorously for 72 hours. Concentrated hydrochloric acid (6ml) was carefully added, and the mixture stirred at room temperature for 3 hours, then concentrated under reduced pressure. The residue was dissolved in water (50ml), and the solution purified by column chromatography on Amberlyst® 15 ion-exchange resin, using an elution gradient of water:0.88 ammonia (100:0 to 98:2) to afford the title compound, 120mg, 35% yield.
¹H-NMR (D₂O, 400MHz) δ : 0.72 (d, 6H), 1.23-1.40 (m, 3H), 1.46 (m, 4H), 2.30-2.43 (m, 2H), 2.59 (dd, 1H), 2.79 (m, 2H), 3.80 (t, 2H), 6.76 (s, 1H), 7.42 (s, 1H).
LRMS (ES⁻): m/z 266 (M-H)⁻
Anal. Found: C, 58.60; H, 9.62; N, 14.56. C₁₄H₂₅N₃O₂•1.0H₂O requires C, 58.92; H, 9.54; N, 14.72%.

### Example 50

### (±)-2-[(1-Isopentyl-1H-imidazol-4-yl)methyl]-5-(methylamino)pentanoic acid

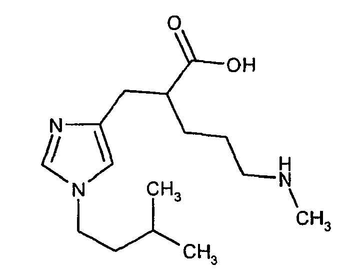

A solution of the compound from preparation 106 (170mg, 0.65mmol) in dioxan (1ml) and concentrated hydrochloric acid (2ml) was heated at reflux for 18 hours. The cooled mixture was concentrated under reduced pressure at room temperature, and the residue dissolved in water (50ml). The solution was purified by column chromatography on Amberlyst® 15 ion-exchange resin, using an elution gradient of water:0.88 ammonia (100:0 to 98:2). Freeze drying afforded the title compound as a brown solid, 120mg, 66% yield.
¹H-NMR (D₂O, 400MHz) δ : 0.75 (d, 6H), 1.25-1.42 (m, 3H), 1.50 (m, 4H), 2.34-2.44 (m, 2H), 2.55 (s, 3H), 2.62 (dd, 1H), 2.86 (m, 2H), 3.82 (t, 2H), 6.78 (s, 1H), 7.43 (s, 1H).
LRMS : m/z 282.2 (MH⁺)
Anal. Found: C, 58.56; H, 9.73; N, 13.61. C₁₅H₂₇N₃O2•1.45H₂O requires C, 58.59; H, 9.80; N, 13.66%.

### Example 51

### (±)-5-Amino-2-[(1-phenyl-1H-imidazol-4-yl)methyl]pentanoic acid

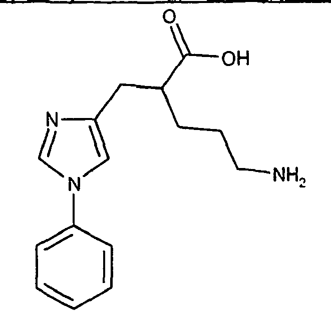

A solution of lithium hydroxide (2ml, 1 M, 2mmol) was added to a solution of the compound from preparation 108 (240mg, 0.68mmol) in tetrahydrofuran (2ml), and the reaction stirred at room temperature for 5 hours. Concentrated hydrochloric acid (2ml) was added carefully, and the reaction stirred at room temperature for 18 hours. The solution was evaporated under reduced pressure, the residue dissolved in water, and the solution purified by column chromatography on Amberlyst® 15 ion-exchange resin using an elution gradient of water:0.88 ammonia (100:0 to 95:5) to afford the title compound as a white foam, 88mg, 45% yield.
¹H-NMR (D₂O, 400MHz) δ : 1.43 (m, 2H), 1.54 (m, 2H), 2.42-2.59 (m, 2H), 2.74 (dd, 1H), 2.83 (m, 2H), 7.18 (s, 1H), 7.32 (m, 1H), 7.40 (m, 4H), 7.88 (s, 1H).
LRMS : m/z 296 (MNa⁺)
Anal. Found: C, 62.21; H, 7.01; N, 14.55. C₁₅H₁₉N₃O₂•1.0H₂O requires C, 61.84; H, 7.27; N, 14.42%.

### Preparation 1

### (±)-Ethyl 2-[(1-{[2-(trimethylsilyl)ethoxy]methyl}-1H-imidazol-4-yl)methyl]-5-(tritylamino)pentanoate

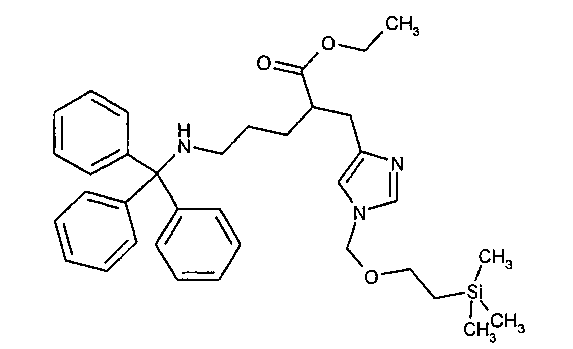

A mixture of the alkenes from Preparation 49 (460mg, 0.77mmol) and 10% palladium on charcoal (100mg) in ethanol (25ml) was hydrogenated at 1.5 atm and room temperature for 72 hours. The reaction mixture was filtered through Arbocel™, washing through with ethanol (200ml), and the filtrate concentrated under reduced pressure. The residual oil was purified by column chromatography on silica gel using ethyl acetate : pentane (50:50) as eluant, to give the title compound, 150mg, 33% yield.
¹H-NMR (CDCl₃, 400MHz) δ: -0.02 (s, 9H), 0.95 (t, 2H), 1.18 (t, 3H), 1.46 (m, 2H), 1.45-1.70 (m, 2H), 2.09 (m, 2H), 2.64-2.79 (m, 2H), 2.90 (dd, 1H), 3.42 (t, 2H), 4.09 (q, 2H), 5.18 (s, 2H), 6.75 (s, 1H), 7.17 (m, 3H), 7.22 (m, 7H), 7.42 (d, 6H).

### Preparation 2

### (±)-Ethyl 2-[(1-n-propyl-1H-imidazol-4-yl)methyl]-5-(tritylamino)pentanoate

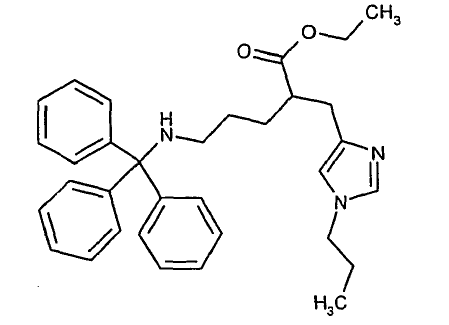

Sodium borohydride (7.2g, 190mmol) was added portionwise over 2 hours to a solution of alkenes from Preparation 50 (3.2g, 6.3mmol) and copper (I) chloride (928mg, 9.5mmol) in methanol (120ml), so as to maintain the reaction temperature at about 45°C, and the reaction stirred at this temperature for 2 hours, (two additional portions of copper (I) chloride (310mg, 3.1mmo)) were added after approx 40 and 80 minutes). The reaction mixture was filtered through Arbocel™ and the filtrate concentrated under reduced pressure. The residue was partitioned between ethyl acetate and water, the layers separated, and the aqueous phase extracted with ethyl acetate (2x). The combined organic extracts were dried (Na₂SO₄) and concentrated under reduced pressure. The crude product was purified by column chromatography on silica gel using an elution gradient of ethyl acetate : pentane (50:50 to 100:0) to give the title compound, 2g, 62% yield.
¹H-NMR (CDCl₃, 300MHz) δ: 0.88 (t, 3H), 1.19 (t, 3H), 1.55 (m, 4H), 1.76 (m, 2H), 2.08 (m, 2H), 2.62-2.80 (m, 2H), 2.86 (dd, 1H), 3.79 (t, 2H), 4.07 (q, 2H), 6.60 (s, 1H), 7.18 (m, 3H), 7.24 (m, 7H), 7.43 (d, 6H).
LRMS : m/z 510 (MH⁺)

### Preparation 3

### (±)-6-[(tert-Butoxycarbonyl)amino]-2-[(1-n-propyl-1H-imidazol-4-yl)methyl]hexanoic acid

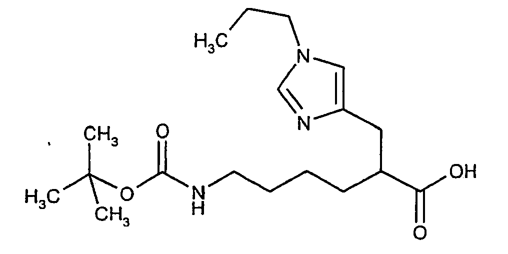

A solution of the compound from Preparation 4 (32mg, 0.07mmol) in tetrahydrofuran (2ml) and ethanol (50µl) was added to a cooled (-78°C) solution of sodium (20mg, 0.87mmol) in 0.88 ammonia (3ml), and the solution stirred for 15 minutes, until the blue colour disappeared. The reaction was allowed to warm to room temperature, the ammonia evaporated off and then the remaining solution was concentrated under reduced pressure. The crude product was purified by ion exchange chromatography on DOWEX® (50WX8-200) resin, eluting with a solvent gradient of water : 0.88 ammonia (100:0 to 97:3), to give the title compound, 17mg, 69% yield.
¹H-NMR (CD₃OD 300MHz) δ: 0.90 (t, 3H), 1.42 (m, 13H), 1.61 (m, 2H), 1.80 (m, 2H), 2.57-2.68 (m, 2H), 2.80-2.95 (m, 2H), 3.00 (m, 1H), 3.95 (t, 2H), 6.98 (s, 1H), 7.76 (s, 1H).
LRMS : m/z 354.3 (MH⁺)

### Preparation 4

### Sodium 6-[benzyl(tert-butoxycarbonyl)amino]-2-[(1-n-propyl-1H-imidazol-4-yl)methyl]hexanoate

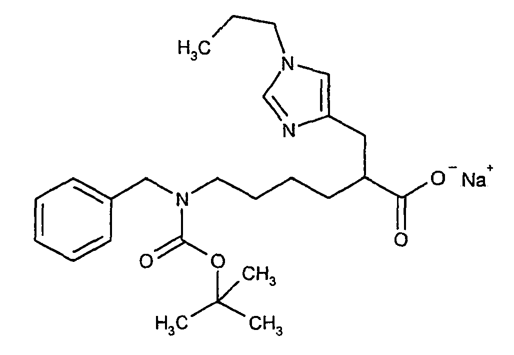

Aqueous sodium hydroxide solution (2ml, 2N) was added to a solution of the ester from Preparation 5 (50mg, 0.106mmol) in dioxane (2ml), and the reaction stirred at room temperature for 18 hours. The mixture was concentrated under reduced pressure and the residue purified by column chromatography on silica gel eluting with dichloromethane : methanol : 0.88 ammonia (90:10:1), to give the title compound, 32mg, 65% yield.
¹H-NMR (CD₃OD 300MHz) δ: 0.88 (t, 3H), 1.15-1.57 (m, 15H), 1.80 (m, 2H), 2.60 (m, 2H), 2.82 (m, 1H), 3.17 (m, 2H), 3.94 (t, 2H), 4.42 (s, 2H), 6.96 (s, 1H), 7.22 (m, 3H), 7.32 (m, 2H), 7.78 (br s, 1H).
LRMS : m/z 444.7 (MH⁺)

### Preparation 5

### (±)-Ethyl 6-[benzyl(tert-butoycarbonyl)amino]-2-[(1-n-propyl-1H-imidazol-4-yl)methyl]hexanoate

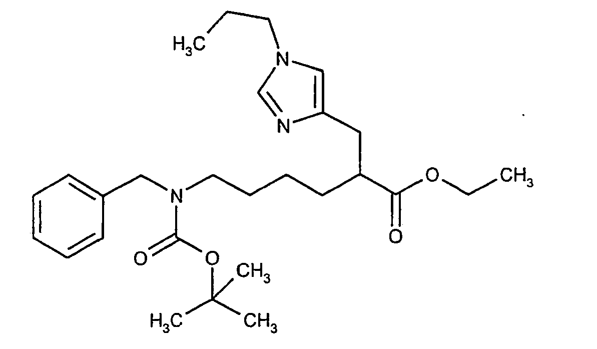

A mixture of the alkenes from Preparation 51 (620mg, 1.32mmol) and 10% palladium on charcoal (70mg) in methanol (50ml) was hydrogenated at 1 atm and room temperature for 4 hours. The reaction mixture was filtered through Arbocel™, and the filtrate concentrated under reduced pressure to give the title compound in quantitative yield as a clear gum, which was used without further purification.
¹H-NMR (CDCl₃, 300MHz) δ: 0.90 (t, 3H), 1.18 (t, 3H), 1.24 (m, 2H), 1.38-1.66 (m, 13H), 1.78 (m, 2H), 2.61-2.80 (m, 2H), 2.86 (dd, 1H), 3.04-3.22 (m, 2H), 3.80 (t, 2H), 4.06 (q, 2H), 4.40 (br s, 2H), 6.61 (s, 1H), 7.18-7.37 (m, 6H).
LRMS : m/z 472.4 (MH⁺)

### Preparation 6

### Ethyl (2R)-2-[(1-n-butyl-1H-imidazol-4-yl)methyl]-5-(tritylamino)pentanoate

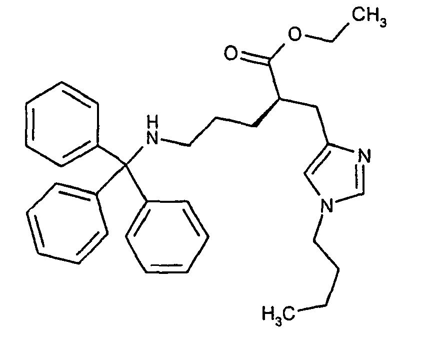

### and Preparation 7

### Ethyl (2S)-2-[(1-n-butyl-1H-imidazol-4-yl)methyl]-5-(tritylamino)pentanoate

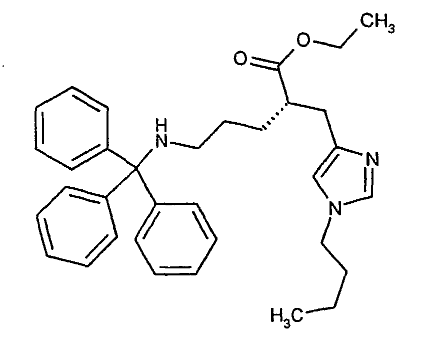

The racemic compound from Preparation 8 was resolved by HPLC using a Chiralcel® OD 250 column (20mm), and hexane : ethanol : diethylamine (85 : 15 : 0.45) as eluant at a rate of 10ml/minute, to afford the title compound of Preparation 6, 98.3%ee,
Retention time: 13.36 minutes,
¹H-NMR (CDCl₃, 300MHz) δ: 0.92 (t, 3H), 1.20 (t, 3H), 1.28 (m, 2H), 1.45-1.78 (m, 6H), 2.10 (m, 2H), 2.62-2.79 (m, 2H), 2.88 (dd, 1H), 3.81 (t, 2H), 4.08 (q, 2H), 6.60 (s, 1H), 7.18 (m, 3H), 7.24 (m, 7H), 7.43 (d, 6H).
and the title compound of Preparation 7, 94.2%ee,
Retention time: 14.91 minutes.
¹H-NMR (CDCl₃, 300MHz) δ: 0.92 (t, 3H), 1.20 (t, 3H), 1.28 (m, 2H), 1.45-1.78 (m, 6H), 2.10 (m, 2H), 2.62-2.79 (m, 2H), 2.88 (dd, 1H), 3.81 (t, 2H), 4.08 (q, 2H), 6.60 (s, 1H), 7.18 (m, 3H), 7.24 (m, 7H), 7.43 (d, 6H).

### Preparation 8

### (±)-Ethyl 2-[(1-n-butyl-1H-imidazol-4-yl)methyl]-5-(tritylamino)pentanoate

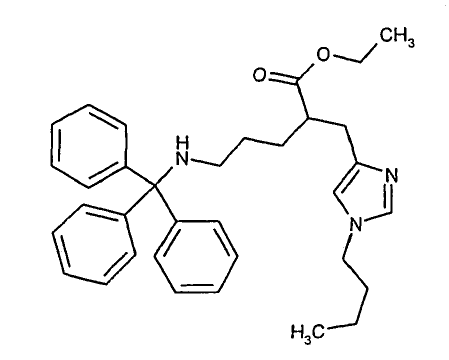

Sodium borohydride (871 mg, 23mmol) was added portionwise over an hour to a solution of the alkene from Preparation 52 (400mg, 0.76mmol) and copper (I) chloride (112mg, 1.15mmol) in methanol (15ml). TLC analysis showed starting material remaining, so additional copper (I) chloride (75mg, 0.76mmol) and sodium borohydride (290mg, 7.7mmol) were added, and the reaction stirred at room temperature for a further 2 hours. The reaction mixture was filtered through Arbocel™, the filtrate concentrated under reduced pressure and the residue partitioned between ethyl acetate and brine. The layers were separated, the aqueous phase extracted with ethyl acetate (2x), and the combined organic extracts dried (Na₂SO₄) and concentrated under reduced pressure, to give the title compound, 185mg, 47% yield.
¹H-NMR (CDCl₃, 400MHz) δ: 0.92 (t, 3H), 1.19 (t, 3H), 1.27 (m, 2H), 1.48-1.77 (m, 6H), 2.10 (m, 2H), 2.62-2.79 (m, 2H), 2.88 (dd, 1H), 3.82 (t, 2H), 4.08 (q, 2H), 6.60 (s, 1H), 7.17 (m, 3H), 7.24 (m, 7H), 7.43 (d, 6H).

### Preparation 9

### Lithium (2R)-5-[(tert-butoxycarbonyl)amino]-2-[(1-n-propyl-1H-imidazol-4-yl)methyl]pentanoate

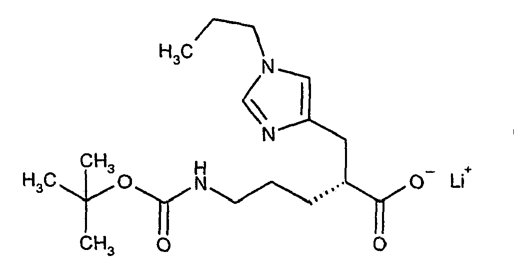

Water (2ml) and lithium hydroxide monohydrate (81mg, 1.93mmol) were added to a solution of the lactam from Preparation 10 (207mg, 0.64mmol) in tetrahydrofuran (3.5ml), and the solution stirred at room temperature for 23 hours. The mixture was concentrated under reduced pressure and the residue purified by column chromatography on silica gel using dichloromethane : methanol : 0.88 ammonia (90:10:0 to 90:10:1) to give the title compound, 200mg, 92% yield.
¹H-NMR (CD₃OD, 300MHz) δ: 0.90 (t, 3H), 1.42 (s, 9H), 1.45-1.62 (m, 4H), 1.80 (m, 2H), 2.57-2.70 (m, 2H), 2.85 (m, 1H), 3.02 (m, 2H), 3.95 (t, 2H), 6.97 (s, 1H), 7.76 (s, 1H).
LRMS (ES⁻): m/z 338 (M-H)⁻

### Preparation 10

### (-)-tert-Butyl (3R)-2-oxo-3-[(1-n-propyl-1H-imidazol-4-yl)methyl]-1-piperidinecarboxylate

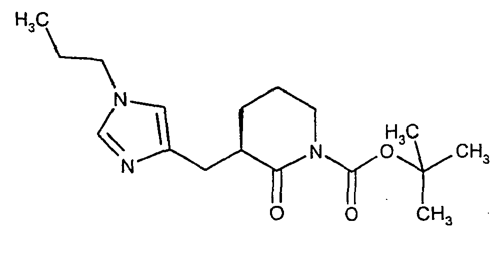

### and Preparation 11

### (+)-tert-Butyl (3S)-2-oxo-3-[(1-n-propyl-1H-imidazol-4-yl)methyl]-1-piperidinecarboxylate

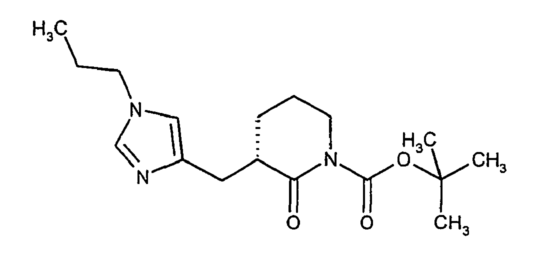

A mixture of the alkene from Preparation 53 (6.6g, 20.6mmol) and palladium black (700mg) in ethanol (120ml) was hydrogenated at 4 atm and 60°C for 18 hours. The cooled mixture was filtered through Arbocel™, washing through with ethyl acetate, and the filtrate concentrated under reduced pressure. The crude product was purified by column chromatography on silica gel, eluting with dichloromethane : methanol (97:3), to afford the racemate of the title compounds as a yellow oil, 4.3g, 65% yield. This racemic compound was resolved by HPLC using a Chiralcel® OG 250 column (20 mm), and hexane : isopropanol (70:30) as eluant at a rate of 10 ml/minute, to give the title compound of Preparation 10, 1.56g, 99.5% ee,
Retention time: 10.10 minutes,
¹H-NMR (CDCl₃, 300MHz) δ: 0.92 (t, 3H), 1.54 (s, 9H), 1.63 (m, 2H), 1.80 (m, 3H), 2.00 (m, 1H), 2.65-2.88 (m, 2H), 3.18 (m, 1H), 3.58 (m, 1H), 3.70-3.90 (m, 3H), 6.72 (s, 1H), 7.38 (s, 1H).
LRMS : m/z 322.5 (MH⁺)
[α]_{D} = -34.34 (*c* 0.12, dichloromethane)
and the title compound of Preparation 11, 1.56g, 98.9% ee,
Retention time: 15.23 minutes,
¹H-NMR (CDCl₃, 300MHz) δ: 0.92 (t, 3H), 1.54 (s, 9H), 1.80 (m, 4H), 2.00 (m, 2H), 2.63-2.85 (m, 2H), 3.19 (m, 1H), 3.58 (m, 1H), 3.90-3.98 (m, 3H), 6.72 (s, 1H), 7.37 (s, 1H).
LRMS : m/z 322.3 (MH⁺)
[α]_{D} = +27.7 (*c* 0.22, dichloromethane)

### Preparation 12

### Ethyl (2R)-2-[(1-{[2-(trimethylsilyl)ethoxy]methyl}-1H-imidazol-4-yl)methyl]-5-(tritylamino)pentanoate

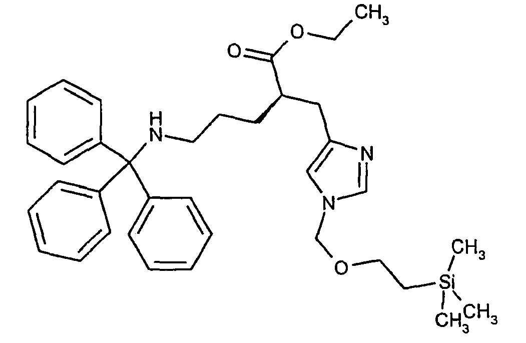

### and Preparation 13

### Ethyl (2S)-2-[(1-{[2-(trimethylsilyl)ethoxy]methyl}-1H-imidazol-4-yl)methyl]-5-(tritylamino)pentanoate

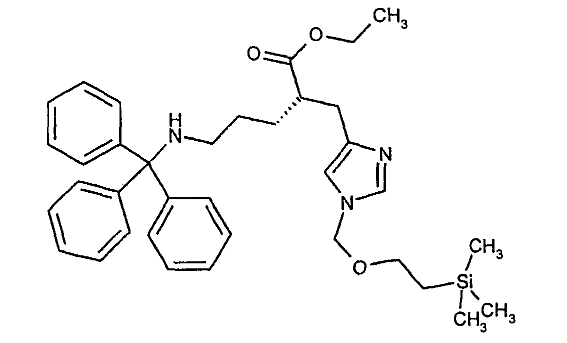

The compound from Preparation 1 was resolved by HPLC using a Chiralcel® OD 250 column (20mm), and hexane : isopropanol : diethylamine (90 : 10 : 0.5) as eluant at 10ml/minute, to give, the title compound of Preparation 12, in 25% yield, 99.4% ee,
Retention time : 16.90 minutes.
¹H-NMR (CDCl₃, 400MHz) δ: -0.02 (s, 9H), 0.95 (t, 2H), 1.20 (t, 3H), 1.44-1.66 (m, 4H), 2.09 (m, 2H), 2.64-2.80 (m, 2H), 2.90 (dd, 1H), 3.42 (t, 2H), 4.09 (q, 2H), 5.18 (s, 2H), 6.75 (s, 1H), 7.17 (m, 3H), 7.22 (m, 7H), 7.42 (d, 6H).
LRMS : m/z 598.7 (MH⁺)
and the title compound of Preparation 13, in 36% yield, 96.5% ee,
Retention time: 22.27 minutes.
¹H-NMR (CDCl₃, 400MHz) δ: -0.02 (s, 9H), 0.95 (t, 2H), 1.20 (t, 3H), 1.44-1.66 (m, 4H), 2.09 (m, 2H), 2.64-2.80 (m, 2H), 2.90 (dd, 1H), 3.42 (t, 2H), 4.09 (q, 2H), 5.18 (s, 2H), 6.75 (s, 1H), 7.17 (m, 3H), 7.22 (m, 7H), 7.42 (d, 6H).

### Preparation 14

### Lithium 5-[(tert-butoxycarbonyl)amino]-2-[(4-propyl-1-{[2-(trimethylsilyl)ethoxy]methyl}-1H-imidazol-2-yl)methyl]pentanoate

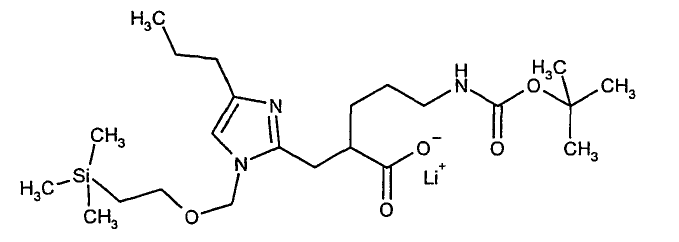

Lithium hydroxide monohydrate (42mg, 0.99mmol) was added to a solution of the lactam from Preparation 15 (150mg, 0.33mmol) in tetrahydrofuran (1ml) and water (1.5ml), and the reaction stirred for 4 hours at room temperature. The mixture was concentrated under reduced pressure and the residue purified by column chromatography on silica gel eluting with dichloromethane : methanol (90:10) as eluant to give the title compound, 108mg, 70% yield.
¹H-NMR (CD₃OD, 300MHz) δ: 0.00 (s, 9H), 0.96 (m, 5H), 1.42 (s, 9H), 1.54 (m, 3H), 1.63 (m, 3H), 2.58 (t, 2H), 2.80 (m, 1H), 2.88-2.98 (m, 1H), 3.02 (m, 2H), 3.16 (dd, 1H), 3.60 (t, 2H), 5.34 (d, 1H), 5.50 (d, 1H), 7.07 (s, 1H).
LRMS : m/z 470.3 (MH⁺)

### Preparation 15

### tert-Butyl 2-oxo-3-[(4-n-propyl-1-{[2-(trimethylsilyl)ethoxy]methyl}-1H-imidazol-2-yl)methyl]-1-piperidinecarboxylate

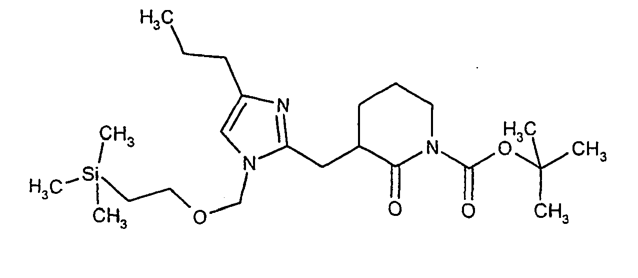

The title compound was obtained in 75% yield from the alkene of Preparation 54, following a similar procedure to that described in Preparation 10/11.
¹H-NMR (CDCl₃, 300MHz) δ: -0.02 (s, 9H), 0.82-0.98 (m, 5H), 1.50 (s, 9H), 1.60 (m, 3H), 1.81 (m, 2H), 2.05 (m, 1H), 2.46 (t, 2H), 2.74 (dd, 1H), 3.03 (m, 1H), 3.35 (dd, 1H), 3.46 (t, 2H), 3.58 (m, 1H), 3.82 (m, 1H), 5.15 (d, 1H), 5.30 (d, 1H), 6.59 (s, 1H).
LRMS : m/z 452.4 (MH⁺)

### Preparation 16

### Methyl (2S)-2-({2-[(tert-butoxycarbonyl)amino]ethyl}amino)-3-(1H-imidazol-4-yl)propanoate

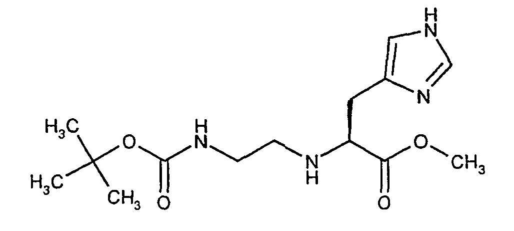

L-Histidine methyl ester (7.93g, 32.8mmol) and sodium acetate (10.75g, 131mmol) were added to a stirred solution of *tert-*butyl *N*-(2-oxoethyl)carbamate (5.22g, 32.8mmol) in methanol (100ml). 4A molecular sieves and sodium cyanoborohydride (4.12g, 65.6mmol) were added and the mixture was stirred at room temperature for 17 hours. Aqueous hydrochloric acid (2N, 4ml) was added and the mixture was then basified with saturated aqueous sodium carbonate solution to pH=10. The mixture was filtered to remove solid which was washed with methanol. Methanol was removed by evaporation under reduced pressure and the residual aqueous solution was extracted with ethyl acetate (2x300ml). The combined organic extracts were then dried (MgSO₄), filtered, and concentrated under reduced pressure. The resultant residue was purified by column chromatography on silica gel, eluting with a solvent gradient of dichloromethane : methanol (96:4 to 92:8), to afford the title compound as a colourless oil, 8.07g, 79% yield.
¹H-NMR (CDCl₃, 300 MHz) δ: 1.42 (s, 9H), 2.65 (m, 1H), 2.90 (m, 2H), 3.07 (m, 1H), 3.19 (m, 1H), 3.30 (m, 1H), 3.58 (m, 1H), 3.73 (s, 3H), 5.22 (br s, 1H), 6.97 (s, 1H), 7.02 (br s, 2H), 7.91 (s, 1H).
LRMS : m/z 313.1 (MH⁺)

### Preparation 17

### Methyl (2R)-2-({2-[(tert-butoxycarbonyl)amino]ethyl}amino)-3-(1H-imidazol-4-yl)propanoate

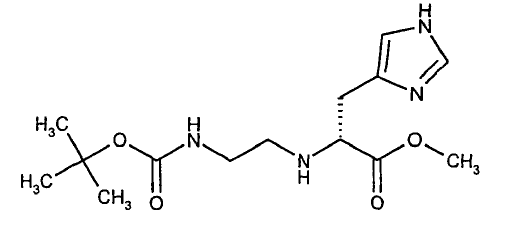

The title compound was prepared from D-histidine methyl ester according to the procedure described in Preparation 16.
¹H-NMR (CDCl₃, 300 MHz) δ: 1.41 (s, 9H), 2.57 (m, 1H), 2.80 (m, 2H), 3.00 (m, 1H), 3.14 (m, 1H), 3.23 (m, 1H), 3.50 (m, 1H), 3.68 (s, 3H), 6.77 (s, 1H), 7.50 (s, 1H).
LRMS : m/z 313 (MH⁺)

### Preparation 18

### (±)-Methyl 2-({2-[(tert-butoxycarbonyl)amino]ethyl}amino)-3-(1H-imidazol-2-yl)propanoate

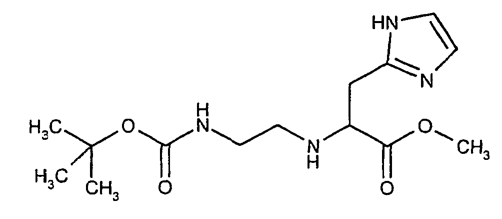

A solution of the amine from Preparation 55 (183mg, 10.8mmol) was dissolved in methanol (7ml) and of *tert*-butyl *N*-(2-oxoethyl)carbamate (172mg, 10.8mmol) was added. Sodium acetate (354mg, 43.2mmol), 4A molecular sieves and then sodium cyanoborohydride (135mg, 21.6mmol) were added, and the resultant mixture was stirred at room temperature for 18 hours. Aqueous hydrochloric acid (2N, 1ml) was then added and the reaction mixture was stirred thoroughly and then basified with saturated aqueous sodium carbonate solution to pH=10. The resultant mixture was then filtered to remove solid and the filtrate was extracted with ethyl acetate (2x). The combined organic extracts were dried (MgSO₄), filtered, and then concentrated under reduced pressure. The residue was purified by column chromatography on silica gel eluting with a solvent gradient of methanol : dichloromethane (1:99 to 5:95) to give the title compound, 105mg, 31% yield.
¹H-NMR (CD₃OD, 400MHz) δ: 1.42 (s, 9H), 2.58 (m, 1H), 2.74 (m, 1H), 3.11 (m, 4H), 3.67 (m, 1H), 3.70 (s, 3H), 7.10 (s, 2H).

### Preparation 19

### Methyl (2S)-2-({2-[(tert-butoxycarbonyl)amino]ethyl}amino)-3-(1-{[2-(trimethylsilyl)ethoxy]methyl}-1H-imidazol-2-yl)propanoate

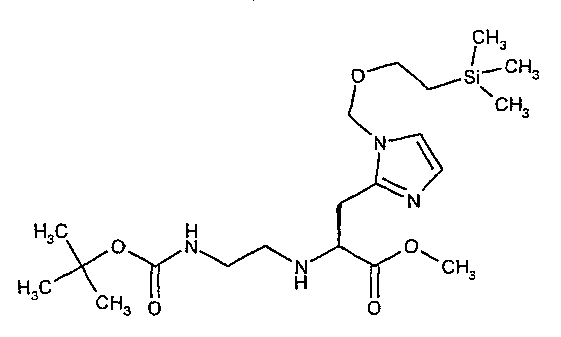

A solution of the amine from Preparation 56 (120mg, 0.40mmol) was dissolved in methanol (3.5ml) and of *tert*-butyl *N*-(2-oxoethyl)carbamate (51 mg, 0.33mmol) was added. Sodium acetate (131mg, 1.60mmol), 4A molecular sieves and then sodium cyanoborohydride (50mg, 0.80mmol) were added, and the resultant mixture was stirred at room temperature for 18 hours. Aqueous hydrochloric acid (1N, 1ml) was then added and the reaction mixture was stirred thoroughly and then basified with saturated aqueous sodium carbonate solution to pH=10. The resultant mixture was extracted with ethyl acetate (2x) and the combined organic extracts were then dried (MgSO₄), filtered, and concentrated under reduced pressure. The residue was purified by column chromatography on silica gel eluting with ethyl acetate : methanol : 0.88 ammonia (55 : 5 : 0.5) to give the title compound, 30mg, 21 % yield.
¹H-NMR (CDCl₃, 300MHz) δ: -0.02 (s, 9H), 0.90 (t, 2H), 1.29 (s, 9H), 2.63 (m, 1H), 2.84 (m, 1H), 3.02 (dd, 1H), 3.13 (dd, 1H), 3.19 (m, 1H), 3.48 (t, 2H), 3.74 (s, 3H), 3.84 (m, 1H), 5.21 (dd, 2H), 5.77 (br s, 1H), 6.90 (s, 1H), 6.97 (s, 1H).
LRMS : m/z 443.3 (MH⁺)

### Preparation 20

### Methyl (2S)-3-(1H-imidazol-5-yl)-2-[(3RS)-pyrrolidinylamino]propanoate

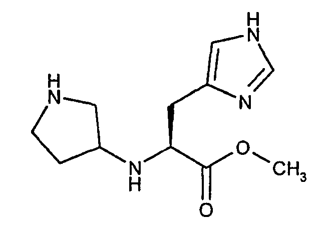

A solution of the product from Preparation 25 (0.4g, 1.22mmol) in acetic acid (30ml) was hydrogenated over palladium catalyst (10% on carbon, 50mg) at 50°C and 3.5 atm for 72 hours. The solution was filtered over Arbocet™/Hyflo™ and the filtrate was concentrated under reduced pressure. The resultant oil was dissolved in dichloromethane and extracted with saturated aqueous sodium bicarbonate solution (3x20ml). The aqueous phase was concentrated under reduced pressure and the resultant white solid was triturated with hot ethyl acetate (2x50ml) then with hot methanol (2x50ml). The methanol extracts were combined and evaporated under reduced pressure. The resultant residue was dissolved in dichloromethane : methanol : 0.88 ammonia (80 : 20 : 2) and purified by column chromatography on silica gel, eluting with dichloromethane : methanol : 0.88 ammonia (80 : 20 : 5), to afford the title compound as an orange oil, 200mg, 70% yield.
¹H-NMR (300 MHz, D₂O), mixture of diastereoisomers, δ: 1.70 (m, 1H), 2.02 (m, 1H), 2.93 (m, 3H), 3.10-3.47 (m, 4H), 3.58 (2x s, 2x1½H), 3.61 (m, 1H), 6.98 (2x s, 2x½H), 8.00 (2x s, 2x½H).
HRMS : m/z 239.1514 (MH⁺), calcd 239.1503.

### Preparations 21 and 22

### Methyl (2S)-2-[((1R or S)-1-{[(tert-butoxycarbonyl)amino]methyl}propyl)amino]-3-(1H-imidazol-4-yl)propanoate and

### Methyl (2S)-2-[((1S or R)-1-{[(tert-butoxycarbonyl)amino]methyl}propyl)amino]-3-(1H-imidazol-4-yl)propanoate

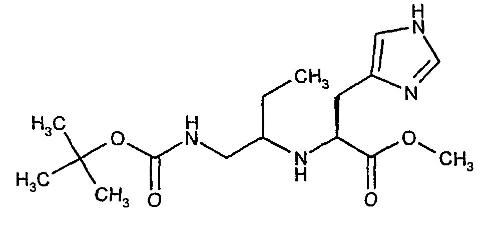

L-Histidine methyl ester dihydrochloride (945mg, 3.9mmol) and sodium acetate (1.28g, 15.6mmol) were added to a stirred solution of the product from Preparation 77 (730mg, 3.9mmol) in methanol (50ml). 4A molecular sieves and sodium cyanoborohydride (491mg, 7.8mmol) were added and the mixture was stirred at room temperature for 17 hours. The mixture was filtered and the filtrate was concentrated to 10ml under reduced pressure. Aqueous hydrochloric acid (2N, 2ml) was added and the mixture was stirred for two minutes. Saturated aqueous sodium hydrogen carbonate solution was added and the mixture was extracted with ethyl acetate (3x150ml). The combined organic extracts were dried (Na₂SO₄), filtered, and concentrated under reduced pressure. The residue was purified by column chromatography on silica gel (Biotage^{™} column), eluting with a solvent gradient of dichloromethane : methanol (95:5 to 90:10), to afford the title compound of Preparation, 21, 178mg, 13% yield:
¹H-NMR (CDCl₃ ,400 MHz) δ: 0.90 (t, 3H), 1.40 (m, 2H), 1.43 (s, 9H), 2.30 (br m, 1H), 2.82 (dd, 1H), 2.97 (dd, 1H), 3.02 (m, 1H), 3.20 (br m, 1H), 3.65 (m, 1H), 3.72 (s, 3H), 5.21 (br s, 1H), 6.80 (s, 1H), 7.57 (s, 1H).
LRMS : m/z 341.2 (MH⁺)
TLC : dichloromethane:methanol (90:10) Rf = 0.48.

And Preparation 22, 271 mg, 20% yield:
¹H-NMR (CDCl₃, 400 MHz) δ: 0.82 (t, 3H), 1.23-1.42 (m, 2H), 1.45 (s, 9H), 2.50 (br m, 1H), 2.80 (dd, 1H), 3.00 (dd, 1H), 3.03-3.18 (m, 2H), 3.60 (m, 1H), 3.73 (s, 3H), 5.30 (br s 1H), 6.82 (s, 1H), 7.53 (s, 1H)
LRMS : m/z 341.3 (MH⁺)
TLC : dichloromethane:methanol (90:10) Rf = 0.41.

### Preparations 23 - 26

The compounds of the following tabulated Preparations of the general formula: 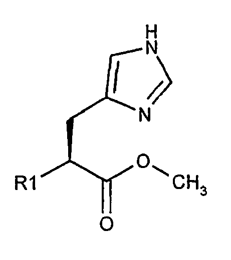 were prepared by a similar method to that of Preparation using L-histidine methyl ester dihydrochloride and the appropriate aldehyde/ketone starting materials (products from Preparations 78-80 or commercially-available 1-benzyl-3-pyrrolidinone).

| Preparation No. | R¹ | Yield (%) | Analytical Data |
|---|---|---|---|
| 23 | 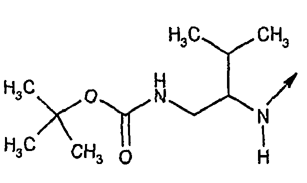 | 48 | ¹H-NMR (CDCl₃, 300 MHz), mixture of diastereoisomers, δ: 0.78-0.98 (4x d, 6H), 1.43 (2x s, 9H), 1.69 (m, 1H), 2.37 (m, 1H), 2.78-3.28 (m, 4H), 3.66 (m, 1H), 3.73 (2x s, 3H), 5.20 (br s, 1H), 6.82 (2x s, 1H), 7.58 (2x s, 1H). |
| | | | TLC : ethyl acetate:methanol (90:10) Rf = 0.27. |
| 24 | 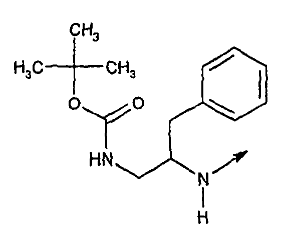 | 45 | ¹H-NMR (CDCl₃, 300 MHz), mixture of diastereoisomers, δ: 1.45 (2x s, 9H), 2.50-3.30 (m, 7H), 3.45-3.70 (2x m, 1H), 3.63-3.73 (2x s, 3H),5.07 (br m, 1H) 6.65-6.78 (2x s, 1H), 7.10-7.58 (m + 2x s, 6H). |
| | | | LRMS : m/z 402.6 (MH⁺) |
| 25 | 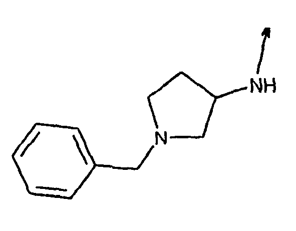 | 37 | ¹H-NMR (CDCl₃, 300 MHz), mixture of diastereoisomers, δ: 1.53 (m, 1H), 2.00 (m, ½ H) 2.13 (m, ½ H), 2.30-2.60 (m, 2H), 2.63-2.81 (m, 2H), 2.90 (m, 1H), 2.99 (m, 1H), 3.30 (m, 1H), 3.45 (m, 1H), 3.59 (m, 2H), 3.67 (s, 1½ H), 3.73 (s, 1½ H) 6.80 (s, 1H), 7.28 (m, 5H), 7.41 (s, ½ H), 7.47 (s, ½ H). |
| | | | LRMS: m/z 329.4 (MH⁺) |
| | | | Anal. Found: C, 65.69; H, 7.41; N, 16.95. C₁₈H₂₄N₄O₂ requires C, 65.83; H, 7.37; N, 17.06%. |
| 26 | 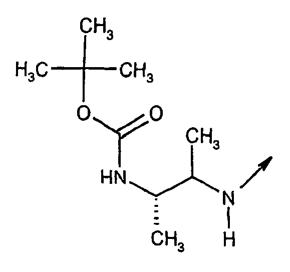 | 67 | ¹H-NMR (CDCl₃, 300 MHz), mixture of diastereoisomers, δ: 0.87-1.13 (m, 6H), 1.45 (2x s, 9H), 2.68 (m, 1H), 2.87 (m, 1H), 3.02 (m, 1H), 3.62 (br m, 1H), 3.70 (s, 3H), 4.45 (br s, 2H), 4.88 (br m, 1H), 6.85 (br s, 1H), 7.60 (br s, 1H). |
| | | | HRMS : m/z 341.2180 (MH⁺), calcd 341.2184. |

### Preparation 27

### Methyl (2S)-2-[{2-[(tert-butoxycarbonyl)amino]ethyl}(methyl)amino]-3-(1H-imidazol-4-yl)propanoate

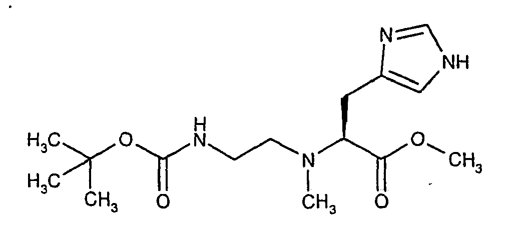

A solution of methyl (2*S*)-3-(4-imidazolidinyl)-2-(methylamino)propanoate (1g, 4.55mmol), of *tert*-butyl *N*-(2-oxoethyl)carbamate (833mg, 5.23mmol), sodium acetate (1.494g, 18.22mmol) and sodium cyanoborohydride (572mg, 9.10mmol) in methanol (30ml) was stirred at 0°C under a nitrogen atmosphere. The mixture was allowed to warm to room temperature then aqueous hydrochloric acid (5ml, 1 N) was added, followed by saturated aqueous sodium hydrogen carbonate solution. The solution was filtered and the aqueous phase was extracted with ethyl acetate. The combined organic extracts were washed with brine, dried (MgSO₄), filtered, and then concentrated under reduced pressure. The residue was purified by column chromatography on silica gel, eluting with dichloromethane : methanol (100:5), to afford the title compound, 900mg, 61 % yield.
¹H-NMR (CDCl₃, 300 MHz) δ: 1.43 (s, 9H), 2.32 (s, 3H), 2.60 (m, 1H), 2.78 (m, 1H), 2.90 (m, 1H), 3.02 (m, 1H), 3.19 (m, 2H), 3.60 (m, 1H), 3.70 (s, 3H), 5.30 (br m, 1H), 6.80 (s, 1H), 7.55 (s, 1H).
LRMS : m/z 327.1 (MH⁺)

### Preparation 28

### Methyl (2S)-3-(1H-imidazol-4-yl)-2-(1-piperazinyl)propanoate

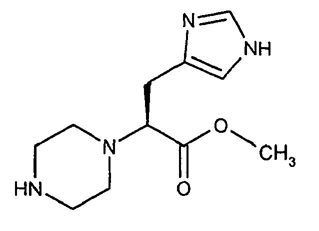

The product from Preparation 29 (200mg, 0.315mmol) was added to a suspension of 4-hydroxybenzoic acid (0.22g, 1.5mmol) in hydrogen bromide solution (45% in acetic acid, 5ml) at 0°C and the mixture was stirred at room temperature for 72 hours. Deionised water (20ml) was added to afford a suspension which was extracted with ethyl acetate (3x20ml). The residual aqueous solution was then concentrated under reduced pressure. The resultant orange foam was crystallised from methanol : ethyl acetate to afford the tri-hydrobromide salt of the title compound as a colourless solid, 82mg, 54% yield. M.p. 211-213°C.
¹H-NMR (D₂O, 300 MHz) δ: 2.80 (m, 2H), 2.97 (m, 2H), 3.15 (m, 6H), 3.65 (s, 3H), 3.73 (t, 1H), 7.23 (s, 1H), 8.53 (s, 1H).
LRMS : m/z 239.2 (MH⁺)
Anal. Found: C, 27.37; H, 4.45; N, 11.36. C₁₁H₁₈N₄O₂•3HBr requires C, 27.47; H, 4.40; N, 11.65%.
[α]_{D} = -32.92 (*c* 0.11, methanol)

### Preparation 29

### Methyl (2S)-2-{4-[(4-methylphenyl)sulfonyl]-1-piperazinyl}-3-(1-trityl-1H-imidazol-4-yl)propanoate

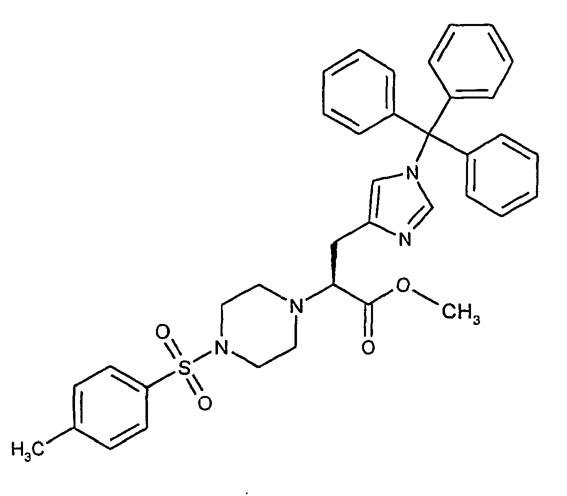

A suspension of methyl (2*S*)-2-amino-3-(1-trityl-1*H*-imidazol-4-yl)propanoate (1g, 2.4mmol) in diisopropylethylamine (5ml), was stirred at room temperature for 20 minutes. *N*,*N*-Bis(2-chloroethyl)-4-methylbenzenesulfonamide (720mg, 2.4mmol) was added and the mixture was stirred at reflux for 3 hours. The mixture was allowed to cool and diluted with acetonitrile. The resultant solution was concentrated under reduced pressure and the residue was suspended in aqueous sodium carbonate solution and extracted with dichloromethane (3x20ml). The combined organic extracts were washed with brine (3x20ml), dried (Na₂SO₄), filtered, and then concentrated under reduced pressure. The residue was purified by column chromatography on silica gel, eluting with a solvent gradient of dichloromethane : methanol (99:1). The isolated material was dissolved in ether and the resultant solution concentrated under reduced pressure to afford the title compound as a colourless foam, 300mg, 19% yield.
¹H-NMR (CDCl₃, 300 MHz) δ: 2.42 (s, 3H), 2.63 (m, 2H), 2.72 (m, 2H), 2.78 (dd, 1H), 2.97 (m, 5H), 3.57 (s, 3H), 3.60 (m, 1H), 6.50 (s, 1H), 7.07 (m, 6H), 7.50 (m, 12H). 7.62 (2x s, 2H).
LRMS : m/z 635.3 (MH⁺)
Anal. Found: C, 69.51; H, 6.06; N, 8.69. C₃₇H₃₈N₄O₄S•0.25H₂O requires C, 69.51; H, 6.07; N, 8.59%.
[α]_{D} = -3.73 (*c* 0.10, dichloromethane)

### Preparation 30

### (7S)-6-{2-[(tert-Butoxycarbonyl)amino]ethyl}-2-ethyl-7-(methoxycarbonyl)-5-oxo-5,6,7,8-tetrahydroimidazo[1,5-c]pyrimidin-2-ium iodide

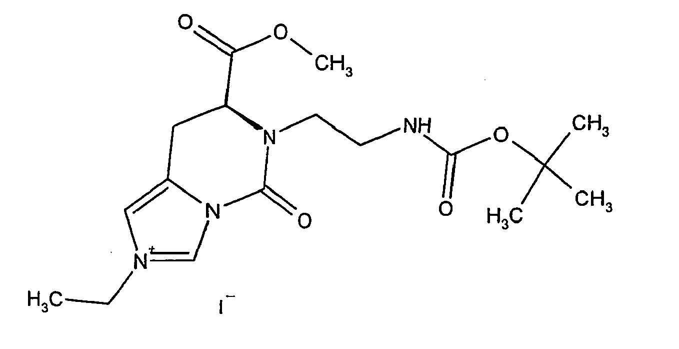

Ethyl iodide (99µl, 1.243mmol) was added to a stirred solution of the product from Preparation 48 (200mg, 0.592mmol) in acetonitrile (5ml) and the mixture was heated at reflux for 17 hours under a nitrogen atmosphere. The mixture was allowed to cool to room temperature and the solvent was removed by evaporation under reduced pressure. The residue was purified by column chromatography on silica gel eluting with dichloromethane : methanol (90:10) to afford the title compound as a white foam, 118mg, 40% yield.
¹H-NMR (D₂O, 300 MHz) δ: 1.27 (s, 9H), 1.42 (t, 3H), 3.22-3.47 (m, 4H), 3.58 (m, 1H), 3.65 (s, 3H), 3.95 (m, 1H), 4.20 (q, 2H), 4.75 (m, 1H), 7.40 (s, 1H).
LRMS : 366.9 (M⁺)
TLC : dichloromethane : methanol : 0.88 ammonia (90 : 10 :1) Rf = 0.26.

### Preparations 31 - 46

The compounds of the following tabulated Preparations of general formula: 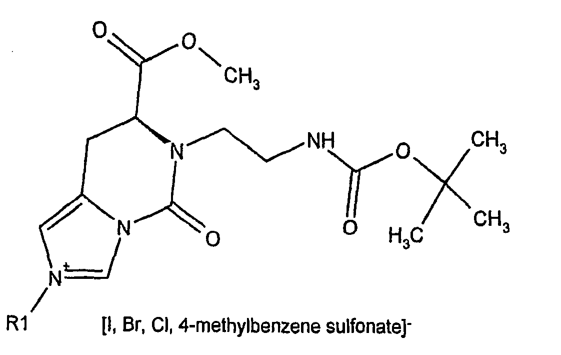 were prepared by a similar method to that of Preparation 30 using the product of Preparation 48 and the appropriate alkylating agent.

| Preparation No. | Alkylating agent | R¹ | Yield (%) | Analytical Data |
|---|---|---|---|---|
| 31 | *n*-Propyl bromide (3eq) | 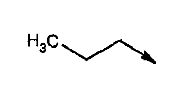 | 44 | ¹H-NMR (D₂O, 400 MHz) δ: 0.75 (t, 3H), 1.20 (s, 9H), 1.75 (q, 2H), 3.20-3.40 (m, 4H), 3.50 (m, 1H), 3.60 (s, 3H), 3.90 (m, 1H), 4.07 (t, 2H), 4.65 (m, 1H), 7.30 (s, 1H). |
| 32 | *n*-Butyl bromide (3eq) | 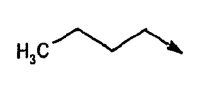 | 46 | ¹H-NMR (D₂O, 300 MHz) δ: 0.82 (t, 3H), 1.22 (q, 2H), 1.30 (s, 9H), 1.80 (m, 2H), 3.27-3.47 (m, 4H), 3.58 (m, 1H), 3.67 (s, 3H), 3.97 (m, 1H), 4.17 (t, 2H), 4.77 (m, 1H), 7.40 (s, 1H). |
| | | | | LRMS : m/z 395.3 (M⁺) |
| 33 | *n*-Pentyl bromide (5eq) | 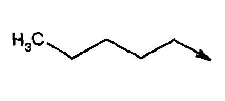 | 55 | ¹H-NMR (D₂O, 300 MHz) δ: 0.72 (t, 3H), 1.03-1.13 (m, 4H), 1.22 (s, 9H), 1.75 (m, 2H), 3.17-3.40 (m, 4H), 3.50 (m, 1H), 3.60 (s, 3H), 3.90 (m, 1H), 4.12 (t, 2H), 4.68 (m, 1H), 7.33 (s, 1H). |
| | | | | LRMS : 409.4 (M⁺) |
| 34 | 2-Bromopropane (5eq) | 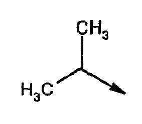 | 9 | ¹H-NMR (D₂O, 300 MHz) δ: 1.28 (s, 9H), 1.47 (d, 6H), 3.20 -3.40 (m, 5H), 3.57 (m, 1H), 3.67 (s, 3H), 3.95 (m, 1H), 4.75 (m, 1H), |
| | | | | 7.45 (s, 1H). LRMS : m/z 381.2 (M⁺) |
| 35 | 1-Iodo-2-methylpropane (5eq) | 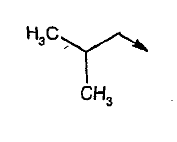 | 32 | ¹H-NMR (D₂O, 400 MHz) δ: 0.82 (d, 6H), 1.27 (s, 9H), 2.07 (m, 1H), 3.25 -3.45 (m, 4H), 3.57 (m, 1H), 3.64 (s, 3H), 3.93 (m, 1H), 4.00 (d, 2H), 4.75 (m, 1H), 7.37 (s, 1H). |
| | | | | LRMS : m/z 394.9 (M⁺) |
| 36 | 1-Bromo-3-methylbutane (5eq) | 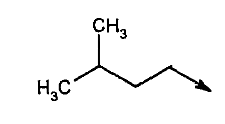 | 51 | ¹H-NMR (D₂O, 300 MHz) δ: 0.83 (d, 6H), 1.28 (s, 9H), 1.45 (m, 1H), 1.70 (m, 2H), 3.25 -3.47 (m, 4H), 3.57 (m, 1H), 3.65 (s, 3H), 3.95 (m, 1H), 4.20 (t, 2H), 4.73 (m, 1H), 7.39 (s, 1H). |
| | | | | LRMS : m/z 409.0 (M⁺) |
| 37 | (1*R*)-1-methylpropyl 4-methylbenzene sulfonate (1.1eq) J. Org. Chem. 1983, 48, 4527. | 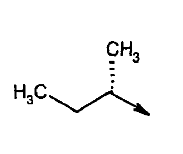 | 19 | ¹H-NMR (D₂O, 300MHz) δ: 0.77 (t, 3H), 1.28 (s, 9H), 1.45 (d, 3H), 1.79 (m, 2H), 2.30 (s, 3H), 3.22-3.42 (m, 4H), 3.50-3.62 (m, 1H), 3.67 (s, 3H), 3.97 (m, 1H), 4.40 (m, 1H), 4.76 (m, 1H), 7.28 (d, 2H), 7.45 (s, 1H), 7.60 (d, 2H). |
| | | | | LRMS : m/z 395.9 (M⁺) |
| 38 | (1*S*)-1-methylpropyl 4-methylbenzene sulfonate (1.1eq) J. Org.Chem. 1974, 39, 1515. | 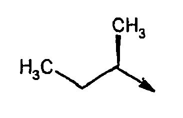 | 20 | ¹H-NMR (D₂O, 300MHz) δ: 0.77 (t, 3H), 1.28 (s, 9H), 1.45 (d, 3H), 1.79 (m, 2H), 2.30 (s, 3H), 3.22-3.42 (m, 4H), 3.50-3.62 (m, 1H), 3.67 (s, 3H), 3.97 (m, 1H), 4.40 (m, 1H), 4.76 (m, 1H), 7.28 (d, 2H), 7.45 (s, 1H), 7.60 (d, 2H). |
| | | | | LRMS : m/z 395.1 (M⁺) |
| 39 | (Bromomethyl) cyclopropane (5eq) | 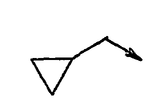 | 57 | ¹H-NMR (D₂O, 300 MHz) δ: 0.40 (m, 2H), 0.67 (m, 2H), 1.25 (s+m, 10H), 3.18 -3.50 (m, 4H), 3.58 (m, 1H), 3.67 (s, 3H), 3.95 (m, 1H), 4.02 (d, 2H), 4.77 (m, 1H), 7.43 (s, 1H). |
| | | | | LRMS : m/z 393.0 (M⁺) |
| 40 | (Bromomethyl) cyclobutane (5eq) | 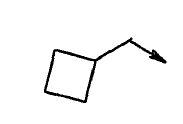 | 35 | ¹H-NMR (D₂O, 300 MHz) δ: 1.27 (s, 9H), 1.62-2.07 (m, 6H), 2.73 (m, 1H), 3.20-3.45 (m, 4H), 3.53 (m, 1H), 3.65 (s, 3H), 3.95 (m, 1H), 4.17 (d, 2H), 4.72 (m, 1H), 7.33 (s, 1H). |
| | | | | LRMS : m/z 407.9 (M⁺) |
| 41 | 1-Bromo-2-methoxyethane (3eq) | 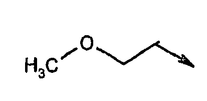 | 73 | ¹H-NMR (D₂O, 400 MHz) δ: 1.27 (s, 9H), 3.20-3.50 (m, 8H), 3.57 (m, 1H), 3.67 (m, 2H), 3.77 (m, 2H), 3.95 (m, 1H), 4.37 (m, 2H), 4.75 (m, 1H), 7.40 (s, 1H). |
| 42 | 1-Bromo propan-3-ol (3eq) | 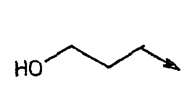 | 73 | ¹H-NMR (D₂O, 300MHz) δ: 1.30 (s, 9H), 2.05 (m, 2H), 3.25-3.48 (m, 4H), 3.58 (m, 3H), 3.67 (s, 3H), 3.97 (m, 1H), 4.30 (t, 2H), 4.78 (m, 1H), 7.41 (s, 1H). |
| | | | | LRMS : m/z 397.3 (M⁺) |
| 43 | (±)-2-Methoxy-1-methylethyl 4-methylbenzene sulfonate¹ (2eq) | 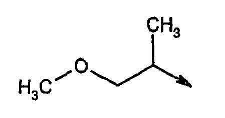 | 30 | ¹H-NMR (D₂O, 400 MHz) δ: 1.27 (s, 9H), 1.43 (d, 3H), 2.27 (s, 3H), 3.20-3.45 (m, 7H), 3.57-3.72 (s+m, 5H), 3.95 (br m, 1H), 4.60-4.80 (m, 3H), 7.28 (d, 2H), 7.47 (s, 1H), 7.59 (d, 2H). |
| 44 | (2-Bromoethyl) benzene | 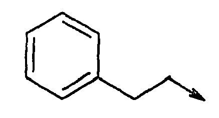 | 33 | ¹H-NMR (D₂O, 300 MHz) δ: 1.27 (s, 9H), 3.06-3.40 (m, 6H), 3.52 (m, 1H), 3.71 (s, 3H), 3.90 (m, 1H), 4.47 (m, 2H), 4.84 (m, 1H), 7.03 (m, 2H), 7.26 (m, 4H). |
| | | | | LRMS: m/z 443.3 (M⁺) |
| 45 | Allyl bromide (5eq) | 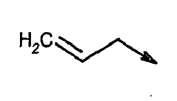 | 76 | ¹H-NMR (D₂O, 300MHz) δ: 1.22 (s, 9H), 3.20-3.38 (m, 4H), 3.44-3.57 (m, 1H), 3.60 (s, 3H), 3.88 (m, 1H), 4.70 (m, 3H), 5.29 (d, 1H), 5.35 (d, 1H), 5.81-5.98 (m, 1H), 7.30 (s, 1H). |
| | | | | LRMS : m/z 379.2 (M⁺) |
| 46 | *N*-(2-bromoethyl) Methane sulfonamide (1.5eq) JACS 1951, *73*, 3100 | 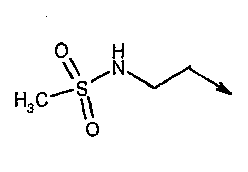 | 29 | ¹H-NMR (D₂O, 300MHz) δ: 1.30 (s, 9H), 2.95 (s, 3H), 3.24-3.56 (m, 7H), 3.64 (s, 3H), 3.98 (m, 1H), 4.32 (t, 2H), 4.77 (m, 1H), 7.42 (s, 1H). |
| | | | | LRMS : m/z 460.7 (M⁺) |

| | | | | |
|---|---|---|---|---|
| Footnote: 1. Product of Preparation 81. | | | | |

### Preparation 47

### (7S)-6-{2-[(tert-butoxycarbonyl)amino]ethyl}-7-(methoxycarbonyl)-2-[2-(methylamino)-2-oxoethyl]-5-oxo-5,6,7,8-tetrahydroimidazo[1,5-c]pyrimidin-2-ium bromide

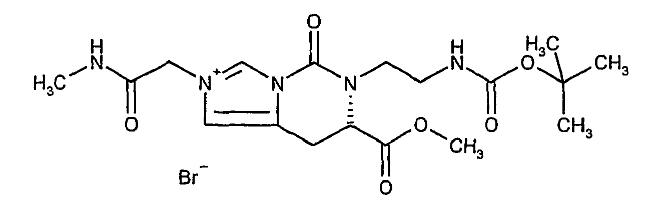

A mixture of the product from Preparation 48 (300mg, 0.89mmol) and 2-bromo-*N-*methylacetamide (Heterocycles 1995, 41, 2427) (270mg, 1.78mmol) in acetonitrile (7ml) was heated at 80°C for 72 hours. The cooled reaction was concentrated under reduced pressure and the residue purified by column chromatography on silica gel using an elution gradient of dichloromethane : methanol (95:5 to 90:10). The product was triturated with ether to afford the title compound as a white solid, 380mg, 87% yield.
¹H-NMR (D₂O, 300MHz) δ: 1.30 (s, 9H), 2.71 (s, 3H), 3.23-3.47 (m, 5H), 3.60 (m, 1H), 3.68 (s, 3H), 3.97 (m, 1H), 4.77 (m, 1H), 5.00 (br s, 2H), 7.38 (s, 1H).
LRMS : m/z 410.4 (M⁺)

### Preparation 48

### Methyl (7S)-6-{2-[(tert-butoxycarbonyl)amino]ethyl}5-oxo-5,6,7,8-tetrahydroimidazo[1,5-c]pyrimidine-7-carboxylate

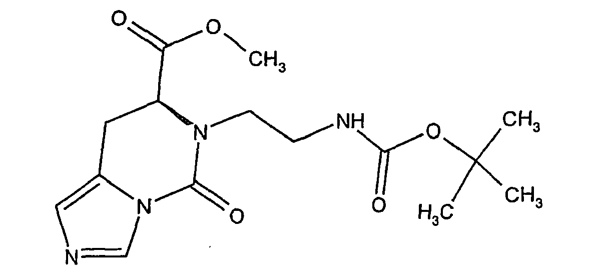

Carbonyldiimidazole (156mg, 0.959mmol) was added to a stirred solution of the product from Preparation 16 (300mg, 0.959mmol) in *N*,*N*-dimethylformamide (5ml) and the mixture was heated at 60-70°C for 17 hours. The solvent was removed by evaporation under reduced pressure, the residue was dissolved in saturated aqueous sodium hydrogen carbonate solution and extracted with dichloromethane. The combined organic extracts were dried (MgSO₄), filtered and then concentrated under reduced pressure. The residue was purified by column chromatography on silica gel, eluting with dichloromethane:methanol (95:5), to afford the title compound as a colourless oil, 210mg, 67% yield.
¹H-NMR (D₂O, 300 MHz) δ: 1.40 (s, 9H), 3.20-3.60 (m, 5H), 3.70 (s, 3H), 4.08 (m, 1H), 4.33 (m, 1H), 4.82 (br m, 1H), 6.80 (s, 1H), 8.13 (s, 1H).
LRMS : m/z 339 (MH⁺)
[α]_{D} = +39.2 (*c* 0.12, dichloromethane)
TLC : ethyl acetate:methanol (95:5) Rf = 0.79

### Preparation 49

### Ethyl (2E and 2Z)-3-(1-{[2-(trimethylsilyl)ethoxy]methyl}-1H-imidazol-4-yl)-2-[3-(tritylamino)propyl]-2-propenoate

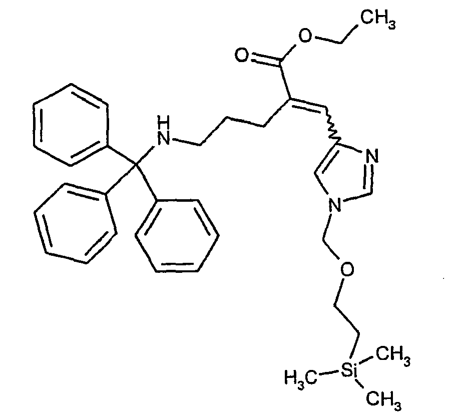

The geometric isomers of the title compound were obtained in 32% and 38% yield respectively, from the compound from Preparation 60, and the aldehyde from Preparation 68, following a similar procedure to that described in Preparation 52. Isomer 1, ¹H-NMR (CDCl₃, 300MHz) δ: -0.02 (s, 9H), 0.90 (t, 2H), 1.28 (t, 3H), 1.78 (m, 2H), 2.18 (t, 2H), 2.40 (br s, 1H), 2.97 (t, 2H), 3.44 (t, 2H), 4.19 (q, 2H), 5.20 (s, 2H), 7.15-7.32 (m, 12H), 7.43 (d, 6H).
LRMS : m/z 596.5 (MH⁺)
and isomer 2, ¹H-NMR (CDCl₃, 300MHz) δ: -0.01 (s, 9H), 0.90 (t, 2H), 1.28 (t, 3H), 1.72 (m, 2H), 2.19 (t, 2H), 2.46 (t, 2H), 3.47 (t, 2H), 4.22 (q, 2H), 5.22 (s, 2H), 6.70 (s, 1H), 7.18 (m, 3H), 7.24 (m, 6H), 7.45 (d, 6H), 7.55 (s, 1H), 7.79 (s, 1H).
LRMS : m/z 596.3 (MH⁺)

### Preparation 50

### Ethyl (2E and 2Z)-3-(1-n-propyl-1H-imidazol-4-yl)-2-[3-(tritylamino)propyl]-2 propenoate

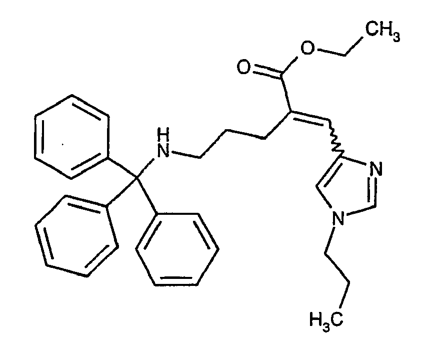

A solution of the compound from Preparation 60 (5.9g, 11.3mmol) in tetrahydrofuran (100ml) was added to an ice-cooled solution of sodium hydride (457mg, 60% dispersion in mineral oil, 11.3mmol) in tetrahydrofuran (100ml), and the mixture stirred for 45 minutes. A solution of the aldehyde from Preparation 66 (1.56g, 11.3mmol) in tetrahydrofuran (100ml) was then added. The reaction was then allowed to warm to room temperature and stirred for 18 hours. The mixture was diluted with aqueous ammonium chloride solution, the layers separated, and the aqueous phase extracted with ethyl acetate (3x). The combined organic extracts were dried (MgSO₄), filtered and concentrated under reduced pressure. The residue was purified by column chromatography on silica gel, eluting with a solvent gradient of ethyl acetate : pentane (40:60 to 60:40), to give the two geometric isomers of the title compound, 1.87g, 33% yield (isomer 1):
¹H-NMR (CDCl₃, 300MHz) δ: 0.92 (t, 3H), 1.27 (t, 3H), 1.78 (m, 4H), 2.18 (t, 2H), 2.52 (br s, 1H), 2.96 (t, 2H), 3.82 (t, 2H), 4.18 (q, 2H), 7.10-7.28 (m, 12H), 7.42 (d, 6H).
LRMS : m/z 508.2 (MH⁺)
and 2.40g, 42% yield (isomer 2):
¹H-NMR (CDCl₃, 300MHz) δ: 0.95 (t, 3H), 1.27 (t, 3H), 1.72 (m, 2H), 1.82 (m, 2H), 2.18 (t, 2H), 2.45 (t, 2H), 3.86 (t, 2H), 4.22 (q, 2H), 6.75 (s, 1H), 7.18 (m, 3H), 7.28 (m, 7H), 7.44 (d, 6H), 7.76 (s, 1H).
LRMS : m/z 508.4 (MH⁺)

### Preparation 51

### Ethyl (2E and 2Z)-2-{4-[benzyl(tert-butoxycarbonyl)amino]butyl}-3-(1-n-propyl-1H-imidazol-4-yl)-2-propenoate

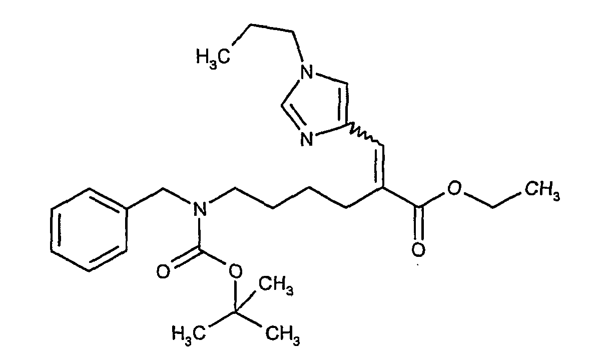

The geometric isomers of the title compound were obtained in 24% and 21% yield respectively, from the compound of Preparation 59, and the aldehyde from Preparation 66, following the procedure described in Preparation 52.
Isomer 1, ¹H-NMR (CDCl₃, 300MHz) δ: 0.96 (t, 3H), 1.27 (t, 3H), 1.37-1.58 (m, 13H), 1.80 (m, 2H), 2.80 (m, 2H), 3.20 (m, 2H), 3.88 (t, 2H), 4.20 (q, 2H), 4.40 (s, 2H), 7.04 (s, 1H), 7.22 (m, 5H), 7.42 (s, 1H), 7.52 (s, 1H).
LRMS : m/z 470.3 (MH⁺)
Isomer 2, ¹H-NMR (CDCl₃, 300MHz) δ: 0.94 (t, 3H), 1.28 (t, 3H), 1.38-1.58 (m, 13H), 1.80 (m, 2H), 2.38 (m, 2H), 3.18 (m, 2H), 3.85 (t, 2H), 4.22 (q, 2H), 4.40 (br s, 2H), 6.70 (s, 1H), 7.23 (m, 5H), 7.40 (s, 1H), 7.75 (s, 1H).
LRMS : m/z 470.3 (MH⁺)

### Preparation 52

### Ethyl (2E and 2Z)-3-(1-n-butyl-1H-imidazol-4-yl)-2-[3-(tritylamino)propyl]-2-propenoate

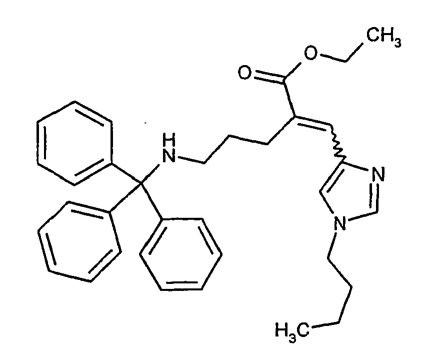

A solution of the compound from Preparation 60 (1g, 2.6mmol) in tetrahydrofuran (20ml) was added to an ice-cooled solution of sodium hydride (106mg, 60% dispersion in mineral oil, 2.6mmol) in tetrahydrofuran (20ml), and the solution stirred for 45 minutes. The aldehyde from Preparation 67 (400mg, 2.6mmol) in tetrahydrofuran (10ml) was then added, and the reaction stirred at room temperature for 18 hours. The reaction was quenched by the addition of aqueous ammonium chloride solution and the mixture extracted with ethyl acetate (2x). The combined organic extracts were dried (Na₂SO₄), filtered, and concentrated under reduced pressure. The residue was dissolved in toluene, adsorbed onto silica, and purified by column chromatography on silica gel, eluting with a solvent gradient of ethyl acetate : pentane (20:80 to 40:60), to give the two geometric isomers of the title compound, 390mg, 29% yield (isomer 1):
¹H-NMR (CDCl₃, 300MHz) δ: 0.94 (t, 3H), 1.28 (m, 5H), 1.76 (m, 4H), 2.18 (t, 2H), 2.55 (br s, 1H), 2.97 (t, 2H), 3.84 (t, 2H), 4.17 (q, 2H), 7.09-7.30 (m, 12H), 7.42 (d, 6H).
LRMS : m/z 522 (MH⁺)
and 400mg, 30% yield (isomer 2):
¹H-NMR (CDCl₃, 300MHz) δ: 0.94 (t, 3H), 1.30 (m, 5H), 1.76 (m, 4H), 2.19 (t, 2H), 2.45 (t, 2H), 3.92 (t, 2H), 4.22 (q, 2H), 6.76 (s, 1H), 7.18 (m, 3H), 7.24 (m, 7H), 7.46 (d, 6H), 7.75 (s, 1H).
LRMS: m/z 523.1 (M+2H)⁺

### Preparation 53

### tert-Butyl (3E)-2-oxo-3-[(1-n-propyl-1H-imidazol-4-yl)methylene]-1-piperidinecarboxylate

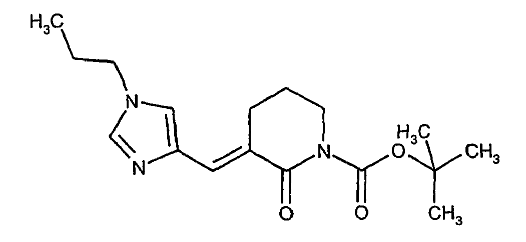

A solution of lithium bis(trimethylsilyl)amide in tetrahydrofuran (43.5ml, 1 M, 43.5mmol) was added dropwise to a cooled (-78°C) solution of *tert*-butyl 2-oxo-1-piperidinecarboxylate (J. Org. Chem. 1983, 48, 2424) (8.7g, 43.5mmol) in tetrahydrofuran (120ml) and, once addition was complete, the solution was allowed to warm to 0°C, and stirred for an hour. The solution was re-cooled to -78°C, a solution of the aldehyde from Preparation 66 (4g, 28.9mmol) in tetrahydrofuran (40ml) was added, and the reaction was then allowed to warm to room temperature. The reaction mixture was stirred for 18 hours and then partitioned between water and ethyl acetate. The phases were separated and the organic phase was dried (MgSO₄), filtered, and concentrated under reduced pressure. The residue was purified by column chromatography on silica gel, eluting with dichloromethane : methanol (95:5), to give the title compound as a single geometric isomer, 4g, 43% yield.
¹H-NMR (CDCl₃, 300MHz) δ: 0.89 (t, 3H), 1.50 (s, 9H), 1.78 (m, 2H), 1.86 (m, 2H), 3.00 (m, 2H), 3.70 (t, 2H), 3.85 (t, 2H), 7.07 (s, 1H), 7.46 (s, 1H), 7.62 (s, 1H).
LRMS : m/z 320.3 (MH⁺)

### Alternative method of synthesis for title compound in Preparation 53

The compound from Preparation 99 (76.5g, 227mmol) was dissolved in dichloromethane (300ml), the solution was cooled to 0°C, and triethylamine (57g, 560mmol) was added. Methanesulphonyl chloride (23.7g, 207mmol) in dichloromethane (15ml) was then added slowly to the stirred solution over 0.5 hours whilst maintaining the reaction temperature between 0-5°C. The reaction was then allowed to warm to room temperature and was stirred for 3 hours. The reaction mixture was then quenched into water (315ml) and the organic phase separated. The aqueous phase was then extracted with dichloromethane (1x50ml) and the combined organic extracts were washed with water (1x100ml), dried and concentrated under reduced pressure to afford the title compound as a solid, 58.0g, 88% yield.

### Preparation 54

### tert-Butyl (3E or 3Z)-2-oxo-3-[(4-n-propyl-1-{[2-(trimethylsilyl)ethoxy]methyl}-1H-imidazol-2-yl)methylene]-1-piperidinecarboxylate or

### tert-Butyl (3E or 3Z)-2-oxo-3-[(5-n-propyl-1-{[2-(trimethylsilyl)ethoxy]methyl}-1H-imidazol-2-yl)methylene]-1-piperidinecarboxylate

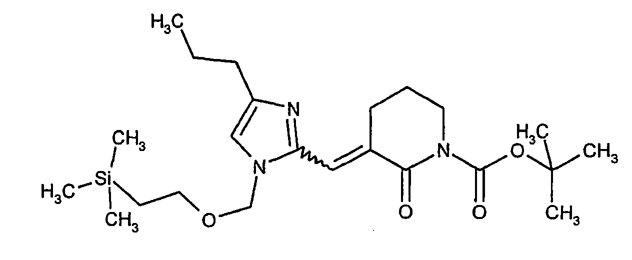

The title compound was obtained as a single stereoisomer in 10% yield from the aldehydes from Preparation 69 and 70, and *tert*-butyl 2-oxo-1-piperidinecarboxylate (J. Org. Chem. 1983, 48, 2424), following a similar procedure to that described in Preparation 53, except hexane : ether (50:50) was used as the column eluant.
¹H-NMR (CDCl₃, 300MHz) δ: -0.03 (s, 9H), 0.88 (t, 2H), 0.98 (t, 3H), 1.56 (s, 9H), 1.66 (m, 2H), 1.92 (m, 2H), 2.58 (t, 2H), 3.22 (m, 2H), 3.48 (t, 2H), 3.77 (m, 2H), 5.30 (s, 2H), 6.80 (s, 1H), 7.73 (s, 1H).
LRMS : m/z 450.6 (MH⁺)

### Preparation 55

### Methyl (2RS)-2-amino-3-(1H-imidazol-2-yl)propanoate

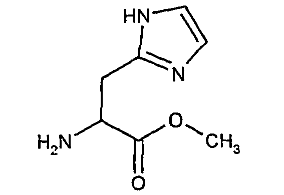

A mixture of the alkene from Preparation 57 (366mg, 12mmol) and 10% palladium on charcoal (50mg) in methanol (8ml) was hydrogenated at 3.5 atm and 50°C for 18 hours. The cooled mixture was filtered through Arbocel™, washing through with methanol, and the filtrate concentrated under reduced pressure to afford the title compound, 200mg, 98% yield.
¹H-NMR (CD₃OD, 400MHz) δ: 3.65 (d, 2H), 3.80 (s, 3H), 4.60 (t, 1H), 7.55 (s, 2H).
LRMS : m/z 170.3 (MH⁺)

### Preparation 56

### Methyl (2S)-2-amino-3-(1-{[2-(trimethylsilyl)ethoxy]methyl}-1H-imidazol-2-yl)propanoate

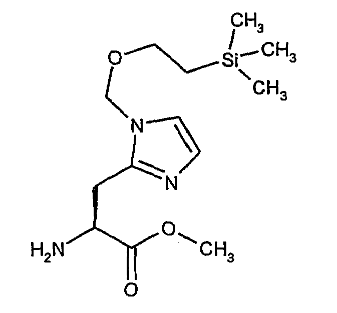

The product from Preparation 58 (950mg, 2.40mmol) was treated with aqueous hydrochloric acid (48ml, 0.25N HCl, 12.0mmol) and the resultant mixture was stirred at room temperature for 2 hours. The reaction was then basified with 0.88 ammonia to pH=9 and extracted with ethyl acetate (2x). The combined organic extracts were dried (Na₂SO₄), filtered, and then concentrated under reduced pressure. The residue was purified by column chromatography on silica gel eluting with ethyl acetate : methanol : 0.88 ammonia (95:5:0.5) to give the title compound, 600mg, 83% yield.
¹H-NMR (CDCl₃, 300MHz) δ: -0.03 (s, 9H), 0.90 (t, 2H), 3.00 (dd, 1H), 3.20 (dd, 1H), 3.48 (t, 2H), 3.71 (s, 3H), 4.05 (m, 1H), 5.23 (dd, 2H), 6.92 (s, 1H), 6.97 (s, 1H).
LRMS : m/z 300.2 (MH⁺)

### Preparation 57

### Methyl (2Z)-2-{[(benzyloxy)carbonyl]amino}-3-(1H-imidazol-2-yl)-2-propenoate

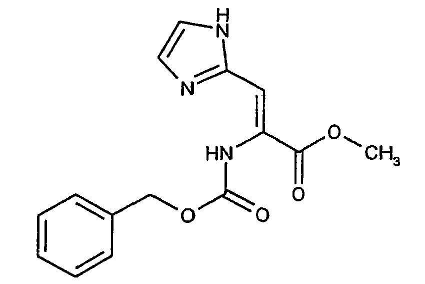

A mixture of methyl 2-{[(benzyloxy)carbonyl]amino}-3-(dimethoxyphosphoryl)-propanoate (1g, 30mmol) in tetrahydrofuran (7ml) was stirred at -40°C and tetramethylguanidine (380mg, 33mmol) was added. The reaction mixture was stirred at -40°C for 20 minutes and then imidazole-2-carboxaldehyde (317mg, 33mmol) was added. The reaction was then allowed to warm to room temperature and was stirred at room temperature for 18h. The solvent was then removed by evaporation under reduced pressure and the residue dissolved in ethyl acetate and washed with water and then brine. The organic phase was then dried (MgSO₄), filtered, and then concentrated under reduced pressure. The residue was purified by column chromatography on silica gel, eluting with a solvent gradient of ethyl acetate : pentane (30:70 to 80:20), to give the title compound, 366mg, 40% yield.
¹H-NMR (CDCl₃, 300MHz) δ: 3.77 (s, 3H), 5.17 (s, 2H), 6.44 (s, 1H), 7.10 (br s, 2H), 7.35 (m, 5H), 10.2 (br s, 1H).
LRMS : m/z 301.9 (MH⁺)

### Preparation 58

### (2R,5R)-2-Isopropyl-3,6-dimethoxy-5-[(1-{[2-(trimethylsilyl)ethoxy]methyl}-1H-imidazol-2-yl)methyl]-2,5-dihydropyrazine

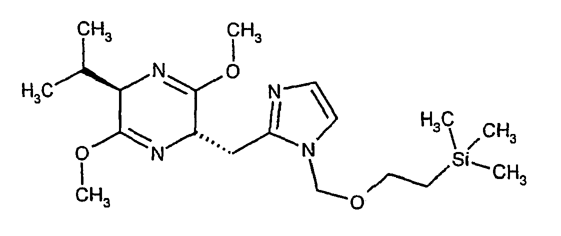

A solution of (2*R*)-2-isopropyl-3,6-dimethoxy-2,5-dihydropyrazine (111mg, 0.60mmol) in tetrahydrofuran (2.5ml) was cooled to -78°C and treated with n-butyl lithium (0.388ml, 1.6M in hexanes, 0.62mmol). The reaction was stirred at -78°C for 45 minutes and the organic solution from Preparation 73 was added. The reaction was then allowed to warm to room temperature and was stirred for a further 18 hours. The reaction was then quenched by the addition of methanol and then the solvent was removed by evaporation under reduced pressure. The residue was diluted with water and ethyl acetate. The layers were separated and the aqueous phase was extracted with further ethyl acetate (2x). The combined organic extracts were then dried (Na₂SO₄), filtered, and then concentrated under reduced pressure. The residue was purified by column chromatography on silica gel, eluting with a solvent gradient of ethyl acetate : hexane (50:50 to 100:0), to give the title compound, 40mg, 17% yield.
¹H-NMR (CDCl₃, 400MHz) δ: -0.03 (s, 9H), 0.65 (d, 3H), 0.84 (t, 2H), 1.00 (d, 3H), 2.16 (m, 1H), 3.03 (dd, 1H), 3.39 (dd, 1H), 3.44 (t, 2H), 3.58 (s, 3H), 3.71 (s, 3H), 3.77 (m, 1H), 4.39 (m, 1H), 5.29 (dd, 2H), 6.90 (s, 1H), 6.95 (s, 1H).
LRMS : m/z 394.8 (MH⁺)

### Preparation 59

### Ethyl (2RS)-6-[benzyl(tert-butoxycarbonyl)amino]-2-(diethoxyphosphoryl)hexanoate

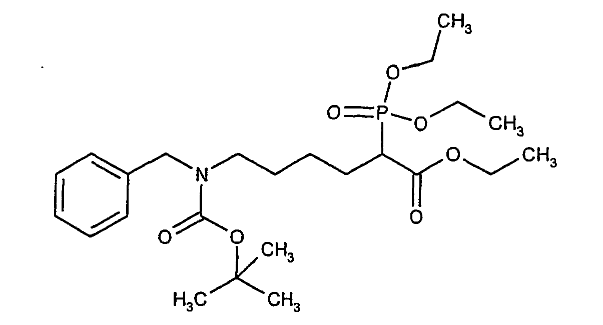

Triethyl phosphonoacetate (2.6ml, 12.9mmol) was added to a solution of sodium hydride (576mg, 14.2mmol) in tetrahydrofuran (75ml), and the solution stirred at room temperature for 30 minutes. A solution of the iodide from Preparation 64 (5.0g, 12.9mmol) in tetrahydrofuran (10ml), and 18-crown-6 (40mg) were added, and the reaction heated under reflux for 18 hours. Aqueous ammonium chloride solution was added to the cooled reaction, and the mixture extracted with ethyl acetate (2x). The combined organic extracts were dried (MgSO₄), filtered, and concentrated under reduced pressure to give a yellow oil. The crude product was purified by column chromatography on silica gel, eluting with a solvent gradient of ethyl acetate : pentane (40:60 to 100:0), to give the title compound, 2.69g, 49% yield.
¹H-NMR (CDCl₃, 300MHz) δ: 1.21-1.37 (m, 9H), 1.38-1.58 (m, 13H), 1.80 (m, 1H), 1.96 (m, 1H), 2.80-2.98 (m, 1H), 3.05-3.25 (m, 2H), 4.16-4.24 (m, 6H), 4.40 (s, 2H), 7.18-7.37 (m, 5H).

### Preparation 60

### Ethyl (2RS)-2-(diethoxyphosphoryl)-5-(tritylamino)pentanoate

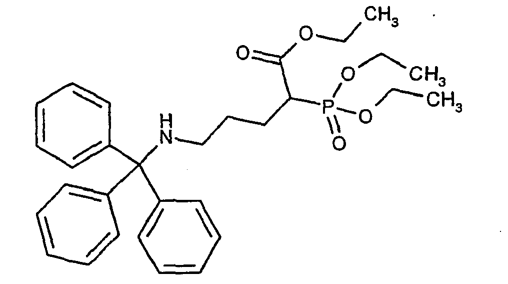

The title compound was prepared in 34% yield from the bromide from Preparation 62, following a similar procedure to that described in Preparation 59.
¹H-NMR (CDCl₃, 400MHz) δ: 1.28 (m, 11H), 1.84-2.02 (m, 2H), 2.15 (t, 2H), 2.93 (m, 1H), 4.17 (m, 6H), 7.18 (m, 3H), 7.24 (m, 6H), 7.44 (d, 6H).
LRMS : m/z 524.4 (MH⁺)

### Preparation 61

### Methyl (2R)-2-chloro-3-(1H-imidazol-4-yl)propanoate

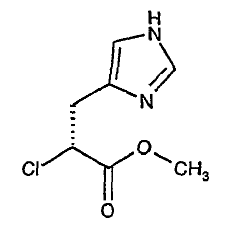

A cold solution of sodium nitrite (2.63g, 38mmol) in water (5ml) was added dropwise to a stirred suspension of D-histidine (2g, 11.5mmol) in concentrated hydrochloric acid (30ml) at -5°C. The mixture was stirred at 0°C for 1 hour and then at room temperature for 17 hours. The mixture was cooled and basified with aqueous ammonium hydroxide solution (2N) until pH = 4-5. The solvent was then removed by evaporation under reduced pressure to afford (2*R*)-2-chloro-3-(1*H*-imidazol-4-yl)propanoic acid.
¹H-NMR (D₂O, 300 MHz) δ: 3.25 (m, 2H), 4.45 (t, 1H), 7.12 (s, 1H), 8.15 (s, 1H).
LRMS : m/z 175.0 (MH⁺)
[α]_{D} = +13.51 (*c* 0.093, methanol)

Hydrogen chloride gas was bubbled through a stirred suspension of (2*R*)-2-chloro-3-(1*H*-imidazol-4-yl)propanoic acid in methanol (60ml) at 0°C for 20 minutes and the suspension was stirred at room temperature for 17 hours. The solvent was then removed by evaporation under reduced pressure and the chilled residue was suspended in cold aqueous saturated sodium bicarbonate solution (20ml) and extracted with dichloromethane (4x20ml). The combined organic extracts were dried (Na₂SO₄), filtered, and concentrated under reduced pressure. The residue was dissolved in diethyl ether and the resultant solution concentrated under reduced pressure to afford the title compound as an oil, 350mg, 14% yield.
¹H-NMR (CDCl₃, 300 MHz) δ: 3.20 (dd, 1H), 3.37 (dd, 1H), 3.75 (s, 3H), 4.59 (m, 1H), 6.90 (s, 1H), 7.57 (s, 1H).
LRMS : m/z 189.0 (MH⁺)
[α]_{D} = +2.13 (*c* 0.16, methanol)

### Preparation 62

### N-(3-bromopropyl)-N-tritylamine

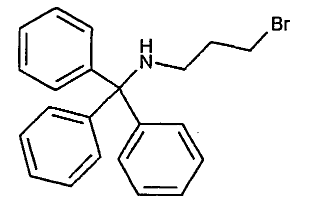

Triphenylphosphine (121g, 0.46mol) was added portionwise to an ice-cooled solution of the alcohol from Preparation 63 (139g, 0.44mol) and carbon tetrabromide (153g, 0.46mol) in dichloromethane (1360ml) and, once addition was complete, the reaction was stirred at room temperature for 48 hours. The reaction was diluted with water, the layers separated, and the aqueous phase extracted with dichloromethane (2x). The combined organic extracts were dried (Na₂SO₄), filtered, and concentrated under reduced pressure. The crude product was purified by column chromatography on silica gel, eluting with a solvent gradient of hexane : ethyl acetate (99:1 to 95:5), to afford the title compound, 81.5g, 49% yield.
¹H-NMR (CDCl₃, 300MHz) δ: 2.02 (m, 2H), 2.28 (m, 2H), 3.58 (t, 2H), 7.19 (m, 3H), 7.27 (m, 6H), 7.46 (d, 6H).

### Preparation 63

### 3-Hydroxy-N-trityl-1-propanamine

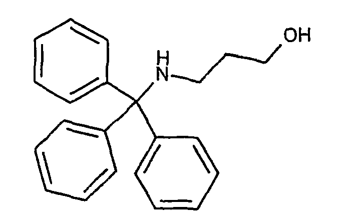

A mixture of 3-amino-1-propanol (51ml, 0.66mol), chlorotriphenylmethane (184g, 0.66mol) and triethylamine (92ml, 0.66mol) in dichloromethane (1000ml) was stirred at room temperature for 18 hours. The reaction mixture was diluted with water and the layers separated. The aqueous phase was extracted with further dichloromethane (2x) and the combined organic extracts were dried (Na₂SO₄), filtered, and concentrated under reduced pressure. The residue was triturated well with diisopropyl ether, and the resulting solid was filtered and dried. This solid was then triturated with methanol, the suspension filtered, and the filtrate concentrated under reduced pressure, to give the title compound as a white solid, 139.1g, 66% yield.
¹H-NMR (CDCl₃, 300MHz) δ: 1.70 (m, 2H), 2.38 (t, 2H), 3.86 (t, 2H), 7.19 (m, 3H), 7.25 (m, 6H), 7.42 (d, 6H).
LRMS : m/z 318.4 (MH⁺)

### Preparation 64

### tert-Butyl benzyl (4-iodobutyl)carbamate

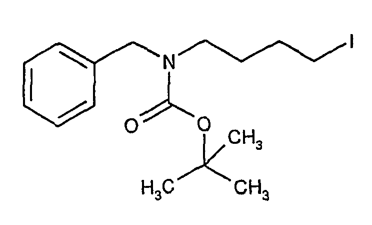

A mixture of the chloride from Preparation 65 (9.3g, 31.3mmol) and sodium iodide (14.9g, 100mmol) in acetone (200ml) was heated under reflux for 18 hours. The cooled reaction mixture was concentrated under reduced pressure, and the residue partitioned between ether and water. The layers were separated and the aqueous phase extracted with ether. The combined organic extracts were then dried (Na₂SO₄), filtered, and concentrated under reduced pressure to afford the title compound as a yellow oil, 10.5g, 87% yield.
¹H-NMR (CDCl₃, 300MHz) δ: 1.40-1.65 (m, 11H), 1.79 (m, 2H), 3.19 (m, 4H), 4.42 (s, 2H), 7.20-7.38 (m, 5H).
LRMS : m/z 390 (MH⁺)

### Preparation 65

### tert-Butyl benzyl (4-chlorobutyl)carbamate

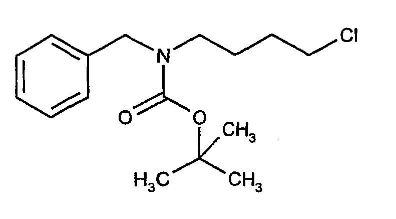

*Tert*-butyl benzylcarbamate (J. Org. Chem. 1993, 58, 56) (9.1g, 44mmol) was added to a solution of sodium hydride (2.14g, 53mmol) in tetrahydrofuran (160ml), and the solution stirred at room temperature for 20 minutes. 1-Bromo-4-chlorobutane (5.07ml, 44mmol) was then added and the reaction heated under reflux for 18 hours. The cooled reaction was quenched by the addition of aqueous ammonium chloride solution, and the mixture extracted with ethyl acetate (2x). The combined organic extracts were dried (Na₂SO₄), filtered, and concentrated under reduced pressure. The crude product was purified by column chromatography on silica gel, eluting with acetate:pentane (95:5), to afford the title compound as a clear oil, 6.1g, 47% yield.
¹H-NMR (CDCl₃, 300MHz) δ: 1.45 (s, 9H), 1.58-1.80 (m, 4H), 3.14-3.30 (m, 2H), 3.52 (t, 2H), 4.42 (s, 2H), 7.25 (m, 5H).
LRMS : m/z 298.0 (MH⁺)

### Preparation 66

### 1-Propyl-1H-imidazole-4-carboxaldehyde

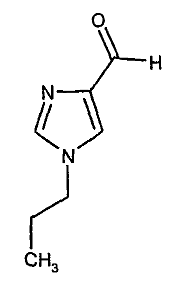

Imidazole-4-carboxaldehyde (30g, 0.31 mol) was added portionwise to a solution of sodium hydride (13.9g, 60% dispersion in mineral oil, 0.348mol) in tetrahydrofuran (450ml), and the solution stirred for 45 minutes. n-Propyl bromide (31.2ml, 0.344mol) was then added portionwise, followed by 18-crown-6 (150mg), and the reaction heated under reflux for 18 hours. Aqueous ammonium chloride solution was added to the cooled reaction, and the mixture extracted with ethyl acetate (2x) and dichloromethane (2x). The combined organic extracts were dried (MgSO₄), filtered, and concentrated under reduced pressure. The crude product was purified by column chromatography on silica gel, eluting with ethyl acetate : pentane (40:60), to give the title compound, 20.2g, 47% yield.
¹H-NMR (DMSO-d₆, 400MHz) δ : 0.80 (t, 3H), 1.76 (m, 2H), 3.98 (t, 2H), 7.84 (s, 1H), 8.04 (s, 1H), 9.70 (s, 1H).
LRMS : m/z 277.3 (2M+H)⁺

### Preparation 67

### 1-n-Butyl-1H-imidazole-4-carboxaldehyde

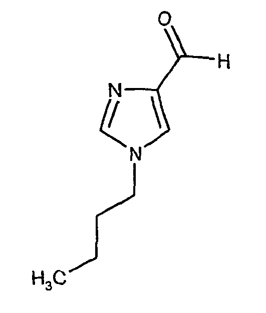

Imidazole-4-carboxaldehyde (10g, 104mmol) was added portionwise to a solution of sodium hydride (4.56g, 60% dispersion in mineral oil, 114mmol) in tetrahydrofuran (150ml), and the solution stirred for 30 minutes. n-Butyl bromide (15.7g, 114mmol) was added portionwise, followed by 18-crown-6 (50mg), and the reaction heated under reflux for 18 hours. Aqueous ammonium chloride solution was added to the cooled reaction and the mixture extracted with ethyl acetate (2x) and dichloromethane (2x). The combined organic extracts were then dried (MgSO₄), filtered, and concentrated under reduced pressure. The residue was purified by column chromatography on silica gel, eluting with a solvent gradient of pentane : ethyl acetate (50:50 to 25:75), to give the title compound, 4.45g, 28% yield.
¹H-NMR (CDCl₃, 300MHz) δ: 0.97 (t, 3H), 1.37 (m, 2H), 1.80 (m, 2H), 4.00 (t, 2H), 7.55 (s, 1H), 7.62 (s, 1H), 9.88 (s, 1H).
LRMS : m/z 153.3 (MH⁺)

### Preparation 68

### 1-{[2-(Trimethylsilyl)ethoxy]methyl}-1H-imidazole-4-carboxaldehyde

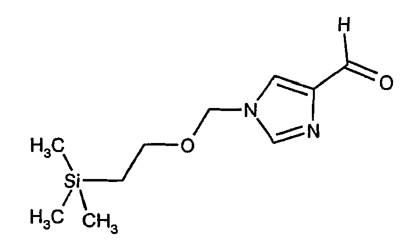

Imidazole-4-carboxaldehyde (1g, 10.4mmol) was added portionwise to a solution of sodium hydride (463mg, 60% dispersion in mineral oil, 11.4mmol) in *N,N-*dimethylformamide (15ml), and the solution stirred for 30 minutes at room temperature. 2-(Trimethylsilyl)ethoxymethyl chloride (2.03ml, 11.4mmol) was added and the reaction stirred at room temperature for 18 hours. The reaction was quenched by the addition of aqueous ammonium chloride solution, and the mixture extracted with ethyl acetate (2x). The combined organic extracts were dried (Na₂SO₄), filtered, and concentrated under reduced pressure. The residue was purified by column chromatography on silica gel, eluting with methanol:ethyl acetate (3:97), to give the title compound, 1.8g, 77% yield.
¹H-NMR (CDCl₃, 300MHz) δ: -0.02 (s, 9H), 0.92 (t, 2H), 3.52 (t, 2H), 5.33 (s, 2H), 7.68 (s, 1H), 7.72 (s, 1H), 9.92 (s, 1H).

### Preparations 69 and 70

### 4-Propyl-1-{[2-(trimethylsilyl)ethoxy]methyl}-1H-imidazole-2-carboxaldehyde and 5-Proayl-1-{[2-(trimethylsilyl)ethoxy]methyl}-1H-imidazole-2-carboxaldehyde

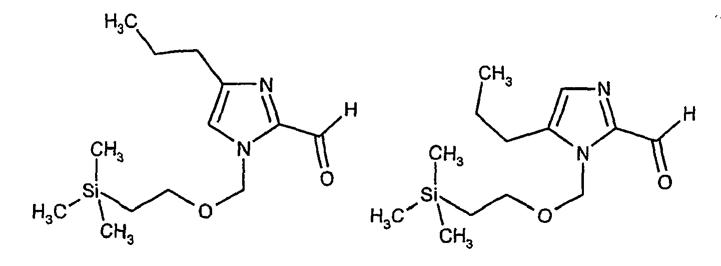

n-Butyl lithium (11.9ml, 1.6M in hexanes, 19.14mmol) was added dropwise to a cooled (-40°C) solution of the imidazoles from Preparations 71 and 72 (4.6g, 19.14mmol) in tetrahydrofuran (75ml) and, once addition was complete, the resulting red solution was stirred for 20 minutes. *N,N*-Dimethylformamide (1.36ml, 19.14mmol) was added dropwise over 15 minutes, and the reaction then allowed to warm to room temperature and stirred for 18 hours. The reaction was quenched by the addition of aqueous ammonium chloride, extracted with ether and the combined organic extracts were concentrated under reduced pressure. The crude product was purified by column chromatography on silica gel, eluting with hexane:ethyl acetate (75:25), to give the title compounds of Preparations 69 and 70 respectively in a 3:1 regioisomeric mixture, 3.4g, 66% yield.
¹H-NMR (CDCl₃, 300MHz) δ: -0.02 (s, 9H), 0.84-1.02 (m, 3H), 1.74 (m, 4H), 2.61 (m, 2H), 3.57 (m, 2H), 5.75 (s, 1.5H), 5.80 (s, 0.5H), 6.98 (s, 0.25H), 7.10 (s, 0.75H), 9.75 (s, 0.25H), 9.77 (s, 0.75H).
LRMS : m/z 269.0 (MH⁺)

### Preparations 71 and 72

### 4-n-Propyl-1-{[2-(trimethylsilyl)ethoxy]methyl}-1H-imidazole and 5-n-ProPyl-1-{[2-(trimethylsilyl)ethoxy]methyl}-1H-imidazole

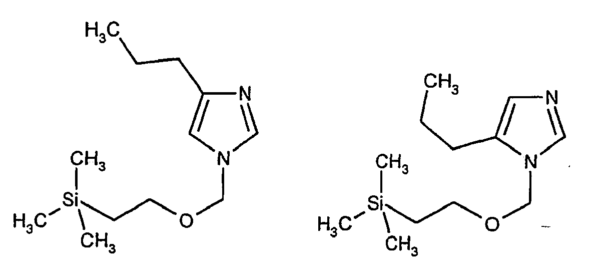

A solution of the imidazole from Preparation 76 (4.9g, 44.6mmol) in tetrahydrofuran (20ml) was added dropwise to a solution of sodium hydride (1.96g, 60% dispersion in mineral oil, 49.1mmol) in tetrahydrofuran (20ml) and, once addition was complete, the solution was stirred for an hour. The solution was cooled to 0°C and 2-(trimethylsilyl)ethoxymethyl chloride (8.28ml, 46.8mmol) was added dropwise over 20 minutes. The reaction mixture was stirred at room temperature for 18 hours, then concentrated under reduced pressure. The residue was partitioned between ether and water, the layers separated, and the aqueous phase extracted with ether. The combined organic extracts were washed with brine, dried (MgSO₄), filtered, and concentrated under reduced pressure. The residual brown oil was purified by column chromatography on silica gel, eluting with dichloromethane : methanol (95:5), to afford the title compounds of Preparation 71 and 72 respectively in a regioisomeric mixture of 3:1, 7g, 65% yield.
¹H-NMR (CDCl₃, 300MHz) δ: 0.0 (s, 9H), 0.90 (m, 3H), 1.65 (m, 4H), 2.58 (m, 2H), 3.45 (m, 2H), 5.20 (s, 2H), 6.74 (s, 0.75H), 6.80 (s, 0.25H), 7.28 (s, 1H).
LRMS : m/z 241.1 (MH⁺)

### Preparation 73

### 2-(Chloromethyl)-1-{[2-(trimethylsilyl)ethoxy]methyl}-1H-imidazole

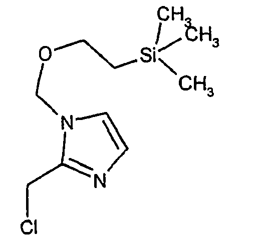

A solution of the alcohol (150mg, 0.66mmol) from Preparation 74 in dichloromethane (3.7ml) was treated with triethylamine (0.138ml, 0.99mmol). Methanesulfonyl chloride (0.061ml, 1.79mmol) was then added and the reaction mixture was stirred for 1 hour. The reaction was then diluted with water and extracted with dichloromethane (2x). The combined organic extracts were dried (Na₂SO₄) and filtered. A small aliquot of the resultant solution was concentrated under reduced pressure to provide a sample of the title compound for characterisation. The remaining organic solution was concentrated to a small volume (0.5ml) and diluted with tetrahydrofuran (5ml). This organic solution was used directly in Preparation 58.
¹H-NMR (CDCl₃, 400MHz) δ: 0.00 (s, 9H), 0.94 (t, 2H), 3.52 (t, 2H), 4.72 (s, 2H), 5.37 (s, 2H), 7.01 (s, 2H).
LRMS : m/z 247 (MH⁺)

### Preparation 74

### (1-{[2-(Trimethylsilyl)ethoxy]methyl}-1H-imidazol-2-yl)methanol

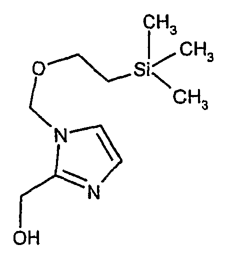

A solution of the aldehyde (2.3g, 10.2mmol) from Preparation 75 in methanol (30ml) was cooled to -20°C. Sodium borohydride (462mg, 12.2mmol) was added portionwise to the stirred solution and the reaction was allowed to warm to room temperature over 1 hour. The reaction was quenched by the addition of aqueous ammonium chloride solution and the resultant mixture was extracted with dichloromethane (2x). The combined organic extracts were dried (Na₂SO₄), filtered, and concentrated under reduced pressure to give the title compound as a beige solid, 2.15g, 93% yield.
¹H-NMR (CDCl₃, 400MHz) δ: -0.03 (s, 9H), 0.90 (t, 2H), 3.52 (t, 2H), 4.71 (s, 2H), 5.35 (s, 2H), 6.94 (s, 1H), 6.97 (s, 1H).

### Preparation 75

### 1-{[2-(Trimethylsilyl)ethoxy]methyl}-1H-imidazole-2-carboxaldehyde

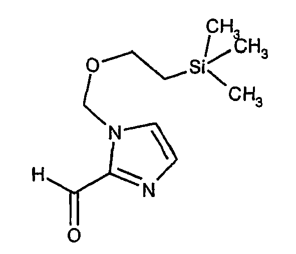

Sodium hydride (463mg, 60% dispersion in mineral oil, 11.4mmol) was washed with hexane under an atmosphere of dry nitrogen. *N,N*-Dimethylformamide (15 ml) was added, the resultant mixture was stirred at room temperature and imidazole-2-carboxaldehyde (1g, 10.4mmol) was added portionwise. The reaction was then stirred for 1.5 hours, 2-(trimethylsilyl)ethoxy methyl chloride (2.03ml, 11.4mmol) was added, and the resultant mixture was then stirred at room temperature for 18 hours. The reaction was quenched by the addition of aqueous ammonium chloride solution and the resultant mixture then extracted with ethyl acetate (2x). The combined organic extracts were dried (Na₂SO₄), filtered, concentrated under reduced pressure and then azeotroped with xylene to give the title compound, 2.3g, 98% yield.
¹H-NMR (CDCl₃, 400MHz) δ: -0.03 (s, 9H), 0.90 (t, 2H), 3.55 (t, 2H), 5.77 (s, 2H), 7.32 (s, 1H), 7.35 (s, 1H), 9.84 (s, 1H).

### Preparation 76

### 4-Propyl-1H-imidazole

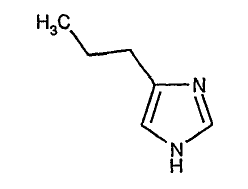

A mixture of 2-bromopentanal (15g, 91mmol) (Bull. Chim. Soc. Fr. 1973, 1465) and formamide (32ml, 806mmol) were heated at 180°C for 8 hours, then allowed to cool. Excess formamide was removed by vacuum distillation, and the residue partitioned between aqueous sodium bicarbonate solution and ethyl acetate. The layers were separated, and the organic phase was concentrated under reduced pressure. The crude product was purified by column chromatography on silica gel, eluting with a solvent gradient of dichloromethane : methanol (93:7 to 90:10), to give the title compound, 9g, 90% yield.
¹H-NMR (CDCl₃, 300MHz) δ: 0.98 (t, 3H), 1.67 (m, 2H), 2.60 (t, 2H), 6.79 (s, 1H), 7.25 (s, 1H), 7.58 (s, 1H).
LRMS : m/z 221 (2M+H)⁺

### Preparation 77

### tert-Butyl N-(2-oxobutyl)carbamate

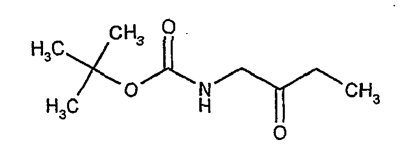

Ethyl magnesium bromide (1M solution in tetrahydrofuran, 13.7ml, 13.7mmol)) was added to a stirred solution of *tert*-butyl 2-[methoxy(methyl)amino]-2-oxoethylcarbamate (Synth. Commun. 1988, 18, 2273) (1g, 4.58mmol) in tetrahydrofuran (25ml) at 0°C then stirred at 0°C for 15 minutes. The solution was allowed to warm to room temperature and was stirred for 45 minutes. Ethyl acetate (5ml) was added, followed by saturated ammonium chloride solution. The aqueous phase was extracted with ethyl acetate. The combined organic extracts were washed with saturated aqueous sodium hydrogen carbonate solution and brine. The organic phase was then dried (Na₂SO₄), filtered, and concentrated under reduced pressure. The residue was purified by column chromatography on silica gel, eluting with a solvent gradient of hexane:ethyl acetate (85:15 to 70:30), to afford the title compound as a colourless oil, 730mg, 84% yield.
¹H-NMR (CDCl₃, 300 MHz) δ: 1.10 (t, 3H), 1.43 (s, 9H), 2.45 (q, 2H), 4.01 (m, 2H), 5.22 (br s, 1H).
LRMS : m/z 187.9 (MH⁺), 204.9 (MNH₄⁺)
TLC: hexane : ethyl acetate (70:30) Rf = 0.41

### Preparations 78 and 79

The compounds of the following tabulated Preparations of the general formula: 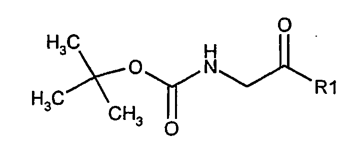 were prepared by a similar method to that of Preparation 77 using *tert*-butyl 2-[methoxy(methyl)amino]-2-oxoethylcarbamate (Synth. Commun. 1988, 18, 2273 and the appropriate Grignard starting materials.

| Preparation | R¹ | Yield (%) | Analytical Data |
|---|---|---|---|
| 78 | 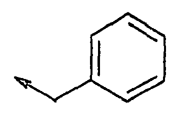 | 12 | ¹H-NMR (CDCl₃, 300 MHz) δ: 1.41 (s, 9H), 3.72 (s, 2H), 4.05 (d, 2H), 5.15 (d, 1H), 7.17-7.40 (m, 5H). |
| 79 | 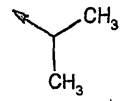 | 58 | ¹H-NMR (CDCl₃, 300 MHz) δ: 1.15 (d, 6H), 1.43 (s, 9H), 2.82 (d, 1H), 4.07 (d, 2H), 5.25 (br s, 1H). |

### Preparation 80

### tert-Butyl (1S)-1-methyl-2-oxopropylcarbamate

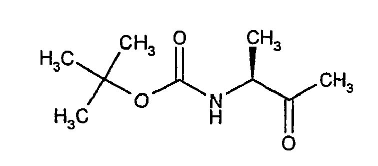

Methyl magnesium bromide (3.0M solution in diethyl ether, 4.3ml, 12.9mmol) was added to a stirred solution of *tert*-butyl (1*S*)-2-[methoxy(methyl)amino]-1-methyl-2-oxoethylcarbamate (Tetrahedron: Asymmetry 1996, 7, 985) (1g, 4.3mmol) in anhydrous tetrahydrofuran (20ml) at -60 °C under a nitrogen atmosphere. The mixture was allowed to warm to 0°C and to then room temperature and was stirred at room temperature for 1 hour. Aqueous saturated ammonium chloride was added and aqueous phase was extracted with diethyl ether (2x 76ml). The combined organic extracts were then washed with saturated aqueous ammonium chloride solution and brine. The organic phase was then dried (MgSO₄), filtered, and concentrated under reduced pressure. The residue was purified by column chromatography on silica gel, eluting with a solvent gradient of dichloromethane : methanol (99:1 to 98:2), to afford the title compound as a colourless solid, 412mg, 51 % yield.
¹H-NMR (CDCl₃, 300 MHz) δ: 1.35 (d, 3H), 1.45 (s, 9H), 2.20 (s, 3H), 4.30 (m, 1H), 5.22 (br s, 1H).

### Preparation 81

### (±)-2-Methoxy-1-methylethyl 4-methylbenzenesulfonate

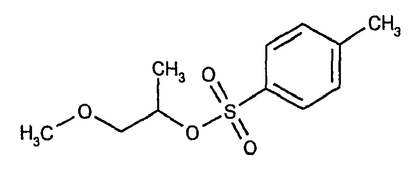

A solution of 1-methoxy-2-propanol in dichloromethane (2.3g, 25.5mmol) (25ml) and pyridine (5ml) was cooled to between -5 and 0°C. 4-Methylbenzenesulfonyl chloride (5.35g, 28.1mmol) was added dropwise and the mixture was stirred at 0°C for 15 minutes. The mixture was then stirred at room temperature for 18 hours. Ice was added and the mixture was stirred for 1 hour. The organic phase was separated, washed with 10% aqueous sulfuric acid (4x) and water (1x), and then dried (MgSO₄) and filtered. The filtrate was purified by column chromatography on silica gel eluting with dichloromethane. The solution obtained was dried (MgSO₄), filtered, and concentrated under reduced pressure to afford the title compound as a colourless oil, 4.3g, 69% yield.
¹H-NMR (CDCl₃, 400 MHz) δ: 1.27 (d, 3H), 2.43 (s, 3H), 3.23 (s, 3H), 3.37 (m, 2H), 4.70 (m, 1H), 7.32 (d, 2H), 7.80 (d, 2H).
LRMS : m/z 262.0 (MNH₄⁺)

### Preparation 82

### Methyl (2S)2-[(tert-butoxycarbonyl)aminol-3-[1-(4,4,4-trifluorobutyl)-1H-imidazol-4-yl]propanoate

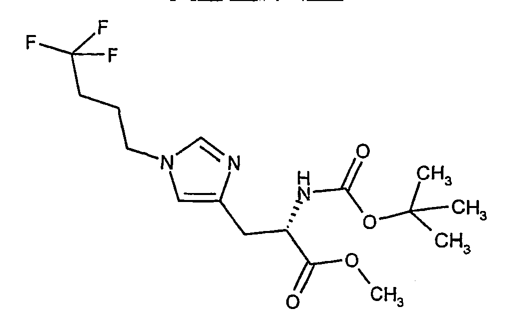

Cesium carbonate (1.95g, 6mmol) and 1-bromo-4,4,4-trifluorobutane (954mg, 5mmol) were added to a solution of methyl (2*S*)-2-[(*tert*-butoxycarbonyl)amino]-3-(1*H*-imidazol-4-yl)propanoate (1.08g, 4mmol) in N,N-dimethylformamide (5ml), and the reaction stirred at 70°C for 3 hours. The cooled mixture was concentrated under reduced pressure and the residue partitioned between ethyl acetate (150ml) and water (50ml). The layers were separated, the organic phase dried (MgSO₄), filtered, and concentrated under reduced pressure. The crude product was purified by column chromatography on silica gel using an elution gradient of cyclohexane:ethyl acetate (100:0 to 0:100) to afford the title compound as an oil, 840mg, 55% yield.
¹H-NMR (CDCl₃, 400MHz) δ: 1.41 (s, 9H), 2.01 (m, 4H), 3.01 (m, 2H), 3.68 (s, 3H), 3.98 (t, 2H), 4.57 (m, 1H), 5.84 (m, 1H), 6.66 (s, 1H), 7.38 (s, 1H).
LRMS : m/z 380.3 (MH⁺)
[α]_{D} = -0.81 (*c* 0.148, methanol)

### Preparation 83

### Methyl (2S)-2-[(tert-butoxycarbonyl)amino]-3-[1-(1,3-thiazol-5-ylmethyl)-1H-imidazol-4-yl]propanoate

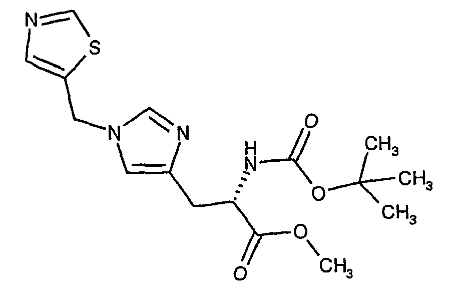

The title compound was obtained as an oil in 20% yield, from methyl (2*S*)-2-[(*tert*-butoxycarbonyl)amino]-3-(1*H*-imidazol-4-yl)propanoate and 5-(chloromethyl)-1,3-thiazole hydrochloride (EP 373891), following a similar procedure to that described in preparation 82, except methanol:ethyl acetate (10:90) was used as the column eluant.
¹H-NMR (CDCl₃, 400MHz) δ: 1.41 (s, 9H), 3.03 (m, 2H), 3.65 (s, 3H), 4.55 (m, 1H), 5.22 (s, 2H), 5.86 (m, 1H), 6.78 (s, 1H), 7.01 (s, 1H), 7.50 (s, 1H), 8.80 (s, 1H).
LRMS : m/z 367.1 (MH⁺)

### Preparation 84

### Methyl (2S)-2-[(tert-butoxycarbonyl)amino]-3-{1-[2-(2-pyridinyl)ethyl]-1H-imidazol-4-yl}propanoate

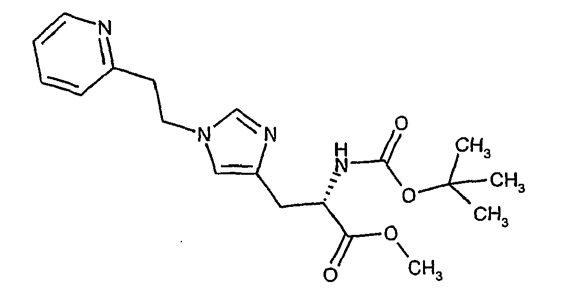

The title compound was obtained in 16% yield, from methyl (2*S*)-2-[(*tert-*butoxycarbonyl)amino]-3-(1*H*-imidazol-4-yl)propanoate and 2-(2-bromoethyl)pyridine hydrobromide (J. Het. Chem. 1973, 10, 39) following a similar procedure to that described in preparation 82, except methanol:ethyl acetate was used as the column eluant.
¹H-NMR (CDCl₃, 400MHz) δ: 1.41 (s, 9H), 2.95 (m, 1H), 3.03 (m, 1H), 3.18 (t, 2H), 3.65 (s, 3H), 4.32 (t, 2H), 4.50 (m, 1 H), 5.80 (m, 1 H), 6.58 (s, 1 H), 6.95 (d, 1H), 7.15 (m, 1 H), 7.20 (s, 1 H), 7.58 (m, 1H), 8.58 (d, 1H).
LRMS: m/z 375.2 (MH⁺)

### Preparation 85

### Methyl (2S)-2-[(tert-butoxycarbonyl)amino]-3-(1-phenyl-1H-imidazol-4-yl)propanoate

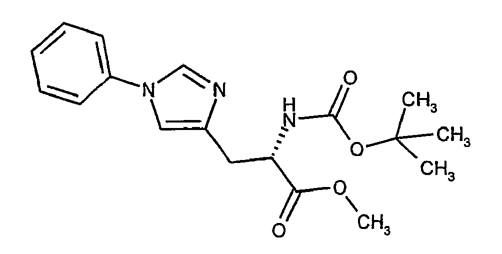

Phenylboronic acid (2.44g, 20mmol), copper acetate (2.72g, 15mmol), 4Å molecular sieves (3g) and pyridine (1.62ml, 20mmol) were added to a solution of methyl (2*S*)-2-[(*tert*-butoxycarbonyl)amino]-3-(1*H*-imidazol-4-yl)propanoate (2.69g, 10mmol) in dichloromethane (60ml), and the reaction mixture stirred at room temperature whilst bubbling through compressed air, for 2 days. A solution of ethylenediaminetetraacetic acid (5g, 17mmol) in saturated sodium bicarbonate solution (200ml) was added and the mixture stirred at room temperature for 20 minutes. The phases were separated, the aqueous layer extracted with dichloromethane (2x100ml), and the combined organic extracts dried (MgSO₄), filtered, and concentrated under reduced pressure. The residue was azeotroped with toluene (300ml), and then purified by column chromatography on silica gel using an elution gradient of pentane:ethyl acetate (100:0 to 40:60), to afford the title compound as a yellow gum, 1.87g, 52% yield.
¹H-NMR (CDCl₃, 400MHz) δ: 1.42 (s, 9H), 3.05-3.19 (m, 2H), 3.72 (s, 3H), 4.60 (m, 1H), 5.84 (m, 1 H), 7.04 (s, 1 H), 7.36 (m, 3H), 7.46 (m, 2H), 7.78 (s, 1 H).
LRMS: m/z 346.1 (MH⁺)
Anal. Found: C, 60.59; H, 6.56; N, 11.57. C₁₈H₂₃N₃O₄•0.75H₂O requires C, 60.24; H, 6.88; N, 11.71%.
[α]_{D} = +10.64 (*c* 0.126, methanol)

### Preparation 86

### Methyl (2S)-2-amino-3-[1-(4,4,4-trifluorobutyl)-1H-imidazol-4-yl]propanoate dihydrochloride

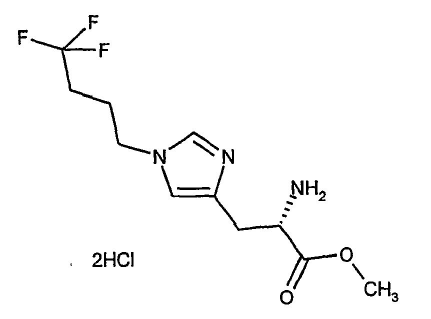

4M Hydrochloric acid in dioxan (5ml) was added to the protected amine from preparation 82 (830mg, 2.19mmol), in an ice-cooled flask. The solution was allowed to warm to room temperature, and stirred for 3 hours. The mixture was concentrated under reduced pressure, the residue azeotroped with ethyl acetate (3x100ml), then dried *in vacuo*, to afford the title compound as a white foam in quantitative yield.
¹H-NMR (D₂O, 400MHz) δ: 2.00-2.19 (m, 4H), 3.28 (m, 2H), 3.70 (s, 3H), 4.17 (t, 2H), 4.37 (t, 1 H), 7.40 (s, 1 H), 8.62 (s, 1 H).
LRMS : m/z 280.1 (MH⁺)
[α]_{D} = +14.60 (*c* 0.1, methanol)

### Preparation 87

### Methyl (2S)-2-amino-3-[1-phenyl-1H-imidazol-4-yl]propanoate dihydrochloride

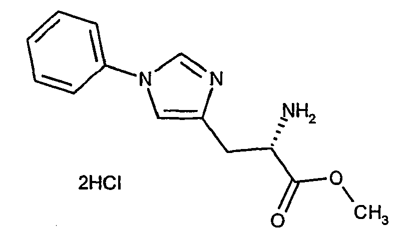

The title compound was obtained in 90% yield as a yellow solid, after trituration from diethyl ether, from the protected amine from preparation 85, following a similar procedure to that described in preparation 86.
¹H-NMR (D₂O, 400MHz) δ: 3.40 (m, 2H), 3.77 (s, 3H), 4.42 (t, 1H), 7.50 (m, 5H), 7.77 (s, 1H), 9.00 (s, 1 H).
LRMS : m/z 246 (MH⁺)
Anal. Found: C, 47.86; H, 5.51; N, 12.61. C₁₃H₁₇N₃O₂Cl₂•1.0H₂O requires C, 47.72; H, 5.54; N, 12.84%.
[α]_{D} = +12.55 (*c* 0_{.}11, methanol)

### Preparation 88

### Methyl (2S)-2-amino-3-[1-(1.3-thiazol-5-ylmethyl)-1H-imidazol-4-yl]propanoate dihydrochloride

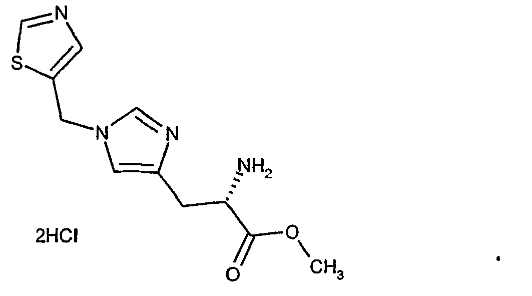

4M Hydrochloric acid in dioxan (6ml) was added to the protected amine from preparation 83 (1.3g, 3.5mmol) in an ice-cooled flask. Water (5ml) followed by concentrated hydrochloric acid were then added, and the solution stirred at room temperature for 18 hours. The mixture was concentrated under reduced pressure and azeotroped with ethanol to afford the title compound, 1.2g, 100% yield.
¹H-NMR (CD₃OD, 400MHz) δ: 3.30-3.46 (m, 2H), 3.81 (s, 3H), 4.43 (m, 1 H), 5.62 (s, 2H), 7.63 (s, 1 H), 7.95 (s, 1H), 9.10 (s, 1H), 9.18 (s, 1H).
LRMS: m/z 267.0 (MH⁺)
[α]_{D} = +14.60 (*c* 0.1, methanol)

### Preparation 89

### Methyl (2S)-2-amino-3-{1-[2-(2-pyridinyl)ethyl]-1H-imidazol-4-yl}propanoate dihydrochloride

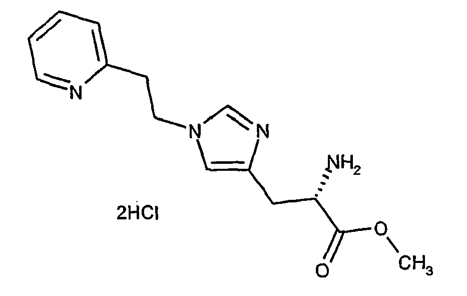

The title compound was obtained as a gum in 95% yield, from the protected amine from preparation 84, following the procedure described in preparation 88. ¹H-NMR (D₂O 400MHz) δ: 3.30 (m, 2H), 3.58 (m, 4H), 3.70 (s, 3H), 4.36 (m, 1H), 7.40 (s, 1 H), 7.78 (d, 1 H), 7.85 (dd, 1 H), 8.41 (dd, 1 H), 8.61 (m, 2H). LRMS : m/z 275.1 (MH⁺)

### Preparation 90

### Methyl(2S)-2-({2-[(tert-butoxycarbonyl)amino]ethyl}amino)-3-(1-methyl-1H imidazol-4-yl)propanoate

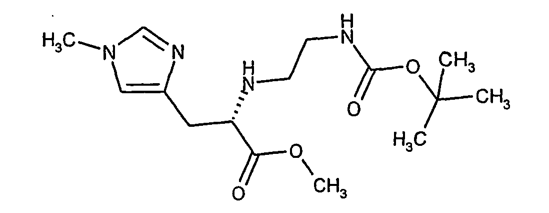

Methyl (2*S*)-2-amino-3-(1-methyl-1*H*-imidazol-4-yl)propanoate dihydrochloride (1.06g, 4mmol), sodium acetate (1.3g, 16mmol) and 4Å molecular sieves (500mg) were added to a solution of *tert*-butyl N-(2-oxoethyl)carbamate (637mg, 4mmol) in methanol (10ml), and the solution stirred for 10 minutes. Sodium cyanoborohydride (1.3g, 16mmol) was then added, and the reaction stirred at room temperature for 72 hours. 2M Hydrochloric acid (2ml) and water (50ml) were added, and the solution then basified using saturated sodium bicarbonate solution. The mixture was extracted with ethyl acetate (5x100ml), the combined organic extracts dried (MgSO₄), filtered, and concentrated under reduced pressure. The crude product was purified by column chromatography on silica gel using an elution gradient of ethyl acetate:methanol:diethylamine (100:0:0 to 96:2:2) to afford the title compound as a colourless oil, 220mg, 17% yield.
¹H-NMR (CDCl₃, 400MHz) δ: 1.41 (s, 9H), 2.62 (m, 1H), 2.77-2.86 (m, 2H), 2.98 (dd, 1H), 3.18 (m, 2H), 3.60 (m, 4H), 3.70 (s, 3H), 5.38 (m, 1H), 6.63 (s, 1H), 7.34 (s, 1 H).
LRMS : m/z 327.2 (MH⁺)
[α]_{D} = -1.48 (*c* 0.108, methanol)

### Preparation 91

### Methyl (2S)-2-({2-[(tert-butoxycarbonyl)amino]ethyl}amino)-3-[1-(4,4,4-trifluorobutyl)-1H-imidazol-4-yl]propanoate

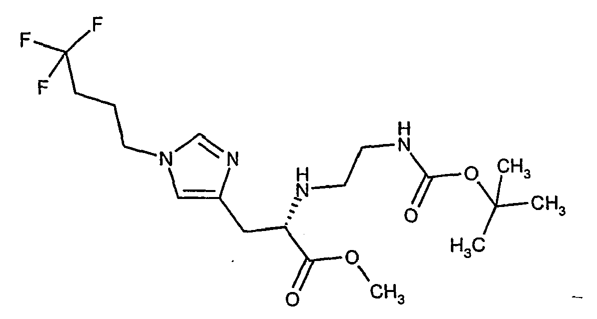

4Å Molecular sieves (500mg) and *tert*-butyl N-(2-oxoethyl)carbamate (350mg, 2.2mmol) were added to a solution of the amine from preparation 86 (780mg, 2.2mmol) in methanol (5ml), and the mixture stirred for 20 minutes. Sodium cyanoborohydride (276mg, 4.4mmol) was added, and the reaction stirred at room temperature for 18 hours. 2M Hydrochloric acid (5ml) was added, the mixture then neutralised using sodium bicarbonate solution, and filtered through Arbocel®. The filtrate was concentrated under reduced pressure and the residue partitioned between ethyl acetate (100ml) and water (20ml). The layers were separated and the organic layer was dried (MgSO₄), filtered, and concentrated under reduced pressure. The crude product was purified by column chromatography on silica gel using an elution gradient of ethyl acetate:methanol (100:0 to 90:10) to afford the title compound as a colourless oil, 300mg, 32% yield.
¹H-NMR (CDCl₃, 400MHz) δ: 1.42 (s, 9H), 2.02 (m, 4H), 2.62 (m, 1 H), 2.78-2.92 (m, 2H), 2.98 (dd, 1 H), 3.18 (m, 2H), 3.60 (t, 1H), 3.68 (s, 3H), 3.98 (t, 2H), 5.40 (m, 1 H), 6.70 (s, 1H), 7.38 (s, 1H).
LRMS : m/z 423.2 (MH⁺)
[α]_{D} = +2.0 (*c* 0.1, methanol)

### Preparations 92 to 94

The following compounds of general structure: 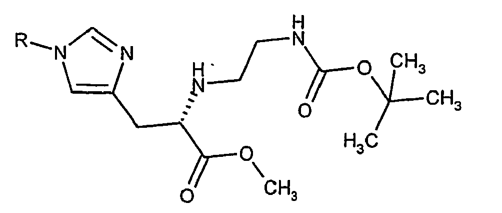 were prepared from the appropriate amines (preparations 87-89) and *tert*-butyl N-(2-oxoethyl)carbamate, following a similar procedure to that described in preparation 91.

| Prep. | R | Yield (%) | Data |
|---|---|---|---|
| 92¹ | 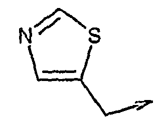 | 12 oil | ¹H-NMR (CDCl₃, 400MHz) δ: 1.38 (s, 9H), 2.58 (m, 1H), 2.70-2.84 (m, 2H), 2.92 (dd, 1H), 3.10 (m, 2H), 3.58 (dd, 1H), 3.62 (s, 3H), 5.19 (s, 2H), 5.38 (m, 1H), 6.75 (s, 1H), 7.00 (s, 1H), 7.44 (s, 1H), 8.77 (s, 1H). |
| | | | LRMS : m/z 410.0 (MH⁺) |
| 93 | 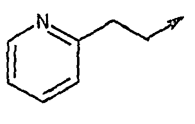 | 35 oil | ¹H-NMR (CDCl₃, 400MHz) δ: 1.42 (s, 9H), 2.60 (m, 1H), 2.75-2.84 (m, 2H), 2.94 (dd, 1H), 3.18 (m, 4H), 3.58 (t, 1H), 3.68 (s, 3H), 4.35 (t, 2H), 5.41 (m, 1 H), 6.61 (s, 1H), 6.98 (d, 1 H), 7.18 (m, 1 H), 7.22 (s, 1H), 7.58 (m, 1H), 8.58 (d, 1H). |
| | | | LRMS : m/z 418.2 (MH⁺) |
| | | | [α]_{D} = +2.52 (*c* 0.103, methanol) |
| 94² | 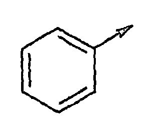 | 10 gum | ¹H-NMR (CDCl₃, 400MHz) δ: 1.41 (s, 9H), 2.65 (m, 1H), 2.81 (m, 1H), 2.96 (dd, 1H), 3.03 (dd, 1 H), 3.19 (m, 2H), 3.70 (m, 4H), 5.38 (m, 1 H), 7.08 (s, 1H), 7.37 (m, 3H), 7.45 (m, 2H), 7.78 (s, 1H). |
| | | | LRMS: m/z 389.2 (MH⁺) |

| | | | |
|---|---|---|---|
| 1 = the product was further purified by column chromatography on silica gel, using ethyl acetate:methanol:diethylamine (90:5:5) as eluant 2 = the product was additionally purified by column chromatography on reverse phase polystyrene gel using water:methanol (100:0 to 0:100) as eluant | | | |

### Preparation 95

### (7S)-2-Benzyl-6-{2-[(tert-butoxycarbonyl)amino]ethyl}-7-(methoxycarbonyl)-5-oxo-5,6,7,8-tetrahydroimidazo[1,5-c]pyrimidin-2-ium bromide

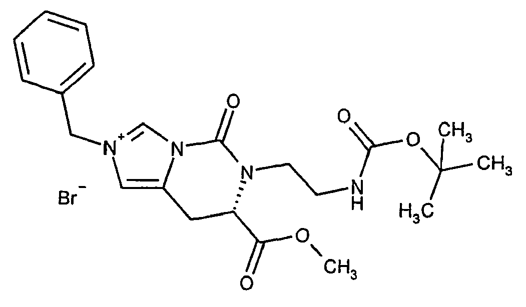

Benzyl bromide (119µl, 1mmol) was added to a solution of the compound from preparation 48 (270mg, 0.8mmol) in acetonitrile (5ml), and the mixture heated at 60°C for 18 hours. The cooled mixture was concentrated under reduced pressure and the residue purified by column chromatography on silica gel using an elution gradient of dichloromethane:methanol (100:0 to 90:10) to afford the title compound, 299mg, 59% yield.
¹H-NMR (DMSOd₆, 400MHz) δ: 1.28 (s, 9H), 3.18 (m, 3H), 3.42 (m, 2H), 3.61 (s, 3H), 3.95 (m, 1H), 4.85 (m, 1H), 5.42 (dd, 2H), 6.94 (m, 1H), 7.38-7.48 (m, 5H), 7.64 (s, 1 H), 10.08 (s, 1 H).
LRMS : m/z 430 (M⁺)
[α]_{D} = +42.09 (*c* 0.096, methanol)

### Preparation 96

### 1-lsopentyl-1H-imidazole-4-carboxaldehyde

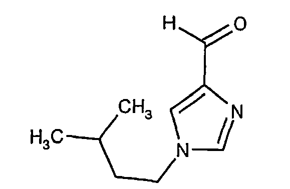

A mixture of sodium hydride (20g, 60% dispersion in mineral oil, 0.5mol) in tetrahydrofuran (300ml) was cooled to 0°C, and 2-imidazolecarboxaldehyde (45g, 0.47mol) was added portionwise over 30 minutes. Once addition was complete, the reaction was stirred at 0°C for 30 minutes, then allowed to warm to room temperature. 1-Bromo-3-methylbutane (60.8ml, 0.5mol) and 18-crown-6 (140mg) were added, and the reaction was heated at reflux for 18 hours. The cooled reaction was quenched by the addition of water (400ml), and the resulting mixture extracted with dichloromethane (800ml in total). The combined organic extracts were dried (MgSO₄) and evaporated under reduced pressure. The residual orange oil was purified by column chromatography on silica gel using an elution gradient of ethyl acetate:pentane:methanol (40:60:0 to 100:0:0 to 98:0:2) to afford the title compound, 19.6g.
Further purification of impure fractions using a Biotage® silica gel column, and ethyl acetate:cyclohexane (40:60) as eluant afforded a further 11.4g of the title compound.

Combination of the two batches provided 31 g of the title compound, 41 % yield.

¹H-NMR (CDCl₃, 400MHz) δ: 0.90 (d, 6H), 1.52 (m, 1H), 1.63 (dt, 2H), 3.97 (t, 2H), 7.47 (s, 1 H), 7.58 (s, 1 H), 9.80 (s, 1H).
LRMS : m/z 189 (MNa⁺)
Anal. Found: C, 63.73; H, 8.43; N, 16.36. C₉H₁₄N₂O;0.2H₂O requires C, 63.65; , 8.55; N, 16.50%.

### Preparation 97

### tert-Butyl 3-[hydroxy(1-isopentyl-1H-imidazol-4-yl)methyl]-2-oxo-1-piperidinecarboxylate

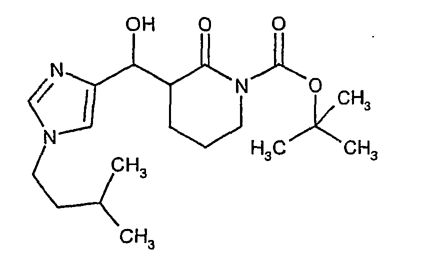

Lithium diisopropylamide (6.5ml, 2M in heptane/tetrahydrofuran/ethylbenzene, 13mmol) was added dropwise over 5 minutes to a cooled (-78°C) solution of *tert-*butyl 2-oxo-1-piperidinecarboxylate (J. Org. Chem. 1983, 48, 2424; J. Chem. Soc. I, 1989, 721) (2.6g, 13mmol) in tetrahydrofuran (25ml), so as to maintain a temperature below -70°C. Once addition was complete, the solution was stirred for 30 minutes, then allowed to warm to -10°C, and stirred for a further 30 minutes, before recooling to -78°C. A solution of the aldehyde from preparation 96 (1.66g, 10mmol) in tetrahydrofuran (5ml) was added dropwise so as to maintain the temperature below -70°C, and once addition was complete, the reaction was stirred for 30 minutes. Saturated ammonium chloride solution (30ml) was added, the mixture allowed to warm to room temperature and then partitioned between water and ethyl acetate. The layers were separated, the aqueous phase extracted with ethyl acetate, and the combined organic extracts dried (MgSO₄), filtered and concentrated under reduced pressure. The resulting yellow oil was purified by column chromatography on silica gel using an elution gradient of ethyl acetate:diethylamine:methanol (100:0:0 to 88:6:6) to afford the title compound, 1.1 g, 30% yield.
¹H-NMR (CDCl₃, 400MHz) (mixture of diastereoisomers) δ: 0.90 (d, 6H), 1.46-1.64 (m, 13H), 1.76 (m, 3H), 2.98 (m, 1 H), 3.52 (m, 1 H), 3.74 (m, 1 H), 3.84 (t, 2H), 4.08, 4.90 (2xm, 1 H), 4.58, 5.34 (2xm, 1 H), 6.85 (2xs, 1 H), 7.35 (2xs, 1 H).
LRMS : m/z 388 (MNa⁺)

### Preparation 98

### 3-[Hydroxy(1-isopentyl-1H-imidazol-4-yl)methyl]-1-methyl-2-piperidinone

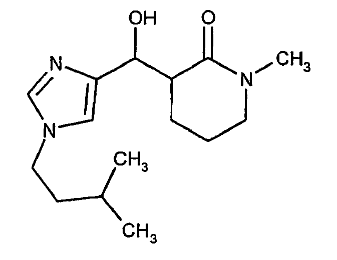

The title compound was obtained in 67% yield from the aldehyde from preparation 96 and 1-methyl-2-piperidinone, following the procedure described in preparation 97. ¹H-NMR (CDCl₃, 400MHz) (mixture of diastereoisomers) δ: 0.88 (2xd, 6H), 1.35-1.82 (m, 7H), 2.67, 2.81 (m, 1 H), 2.88, 2.94 (2xs, 3H), 3.18, 3.22 (m, 2H), 3.84 (t, 2H), 4.78 (m, 1 H), 5.04 (m, 1H), 6.83 (2xs, 1 H), 7.32 (2xs, 1 H).
LRMS : m/z 302 (MNa⁺)

### Preparation 99

### tert-Buty 3-[hydroxy(1-propyl-1H-imidazol-4-yl)methyl]-2-oxo-1-piperidinecarboxyate

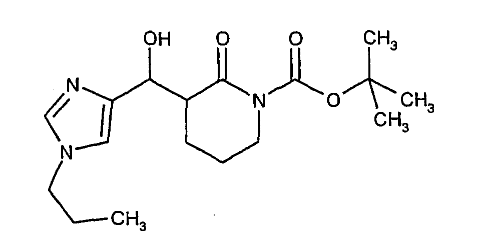

Lithium bis(trimethylsilyl)amide (244ml, 1 M in tetrahydrofuran, 244mmol) was added dropwise over an hour to a cooled (-75°C) solution of *tert-*butyl 2-oxo-1-piperidinecarboxylate (J. Org. Chem. 1983, 48, 2424; J. Chem. Soc. I, 1989, 721) (48.7g, 244mmol) in tetrahydrofuran (200ml) under nitrogen, so as to maintain the temperature below -70°C. The mixture was warmed to 0°C, stirred for 90 minutes, then re-cooled to -75°C. A solution of the imidazole from preparation 66 (26.0g, 188mmol) in tetrahydrofuran (86ml) was added dropwise over 30 minutes, and once addition was complete, the reaction was stirred for 2 hours at -75°C. The mixture was poured into 15% aqueous citric acid solution (650ml), and extracted with ethyl acetate (3x250ml). The aqueous solution was basified to pH 8 using 10% sodium hydroxide, and extracted with dichloromethane (3x250ml). These organic extracts were dried and concentrated under reduced pressure to give the title compound as a pale yellow solid, 54.1g.
The ethyl acetate extracts from above were combined, evaporated under reduced pressure and the residue re-suspended in 10% aqueous citric acid solution (100ml). This was extracted with ethyl acetate (3x50ml), and the aqueous basified to pH 8 using 10% sodium hydroxide solution. The aqueous solution was extracted with dichloromethane (3x50ml), and these organic extracts dried and evaporated under reduced pressure to give additional product as a pale yellow solid, 22.4g. Overall yield of the title compound was thus 76.5g, 93% yield.
¹H-NMR (CDCl₃, 300MHz) (mixture of diastereoisomers) δ: 0.88 (t, 3H), 1.52 (s, 9H), 1.78 (m, 6H), 3.00 (m, 1H), 3.58 (m, 2H), 3.74 (m, 1 H), 3.82 (t, 2H), 5.38 (d, 1 H), 6.87 (s, 1H), 7.38 (s, 1 H).

### Preparation 100

### tert-Butyl 3-[hydroxy(1-trityl-1H-imidazol-4-yl)methyl]-2-oxo-1-piperidinecarboxylate

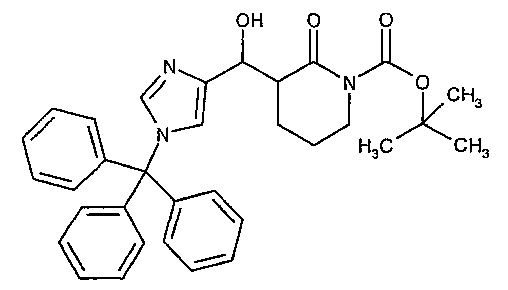

Lithium diisopropylamide (8ml, 1.5M in cyclohexane, 12mmol) was added dropwise over 5 minutes to a cooled (-78°C) solution of *tert-*butyl 2-oxo-1-piperidinecarboxylate (J. Org. Chem. 1983, 48, 2424; J. Chem. Soc. I, 1989, 721) (1.99g, 10mmol) in tetrahydrofuran (40ml), so as to maintain a temperature below -70°C. Once addition was complete, the solution was stirred for 20 minutes. A solution of 1-tritylimidazole-4-carboxaldehyde (J. Med. Chem. 1977, 20, 721) (4.06g, 12mmol) in tetrahydrofuran (60ml) was added slowly, and once addition was complete, the reaction was stirred at -78°C for 2 hours. Saturated aqueous ammonium chloride solution (50ml) was added, the mixture allowed to warm to room temperature and then partitioned between water (50ml) and ethyl acetate (300ml). The phases were separated, the organic layer dried (MgSO₄), filtered, and concentrated under reduced pressure to give the title compound, 5.3g, 99% yield.
¹H-NMR (CDCl₃, 400MHz) (mixture of diastereoisomers) δ: 1.50 (2xs, 9H), 1.60-1.81 (m, 4H), 3.00 (m, 1 H), 3.58 (m, 1 H), 3.74 (m, 1 H), 4.10, 4.90 (2xm, 1 H), 4.62, 5.40 (2xm, 1 H), 6.80 (2xs, 1 H), 7.14 (m, 6H), 7.25-7.40 (m, 10H).
LRMS : m/z 538 (MH⁺)

### Preparation 101

### tert-Butyl (3E)-3-[(1-isopentyl-1H-imidazol-4-yl)methylene]-2-oxo-1-piperidinecarboxylate

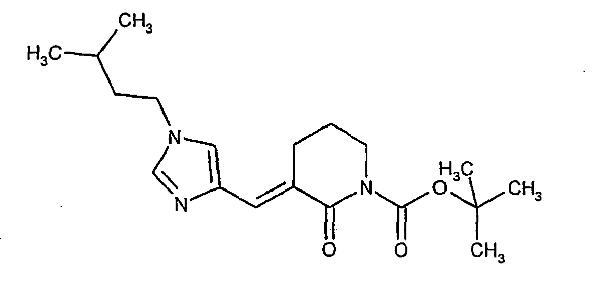

Triethylamine (1.25ml, 9.0mmol) and methanesulphonyl chloride (256µl, 3.3mmol) were added to a solution of the compound from preparation 97 (1.1g, 3.0mmol) in dichloromethane (15ml), and the reaction stirred at room temperature for 18 hours. The solution was poured into water (200ml), and extracted with ethyl acetate (300ml). The organic extract was dried (MgSO₄), filtered, and concentrated under reduced pressure. The crude product was purified by column chromatography on silica gel using an elution gradient of pentane:ethyl acetate (25:75 to 0:100) to afford the title compound as a white solid, 430mg, 41% yield.
¹H-NMR (CDCl₃, 400MHz) δ: 0.92 (d, 6H), 1.52 (s, 9H), 1.56 (m, 1H), 1.64 (m, 2H), 1.88 (m, 2H), 3.03 (t, 2H), 3.73 (dd, 2H), 3.92 (t, 2H), 7.05 (s, 1 H), 7.45 (s, 1 H), 7.62 (s, 1 H).
LRMS : m/z 348.1 (MH⁺)
Anal. Found: C, 65.47; H, 8.49; N, 12.05. C₁₉H₂₉N₃O₃ requires C, 65.68; H, 8.41; N, 12.09%.

### Preparation 102

### (3E)-3-[(1-lsopentyl-1H-imidazol-4-yl)methylene]-1-methyl-2-piperidinone and (3Z)-3-[(1-Isopentyl-1H-imidazol-4-yl)methylene]-1-methyl-2-piperidinone

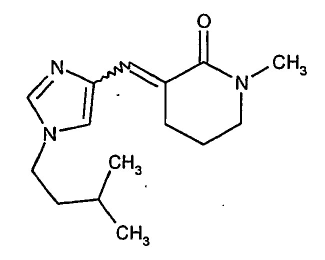

The title compound was obtained as a yellow solid in 46% yield, from the compound from preparation 98, following a similar procedure to that described in preparation 101, except ethyl acetate:diethylamine:methanol (100:0:0 to 96:2:2) was used as the column eluant.
¹H-NMR (CDCl₃, 400MHz) δ (mixture of isomers): 0.94 (d, 6H), 1.58 (m, 1H), 1.70 (m, 2H), 1.92 (m, 2H), 3.03 (s, 3H), 3.12 (m, 2H), 3.40 (t, 2H), 3.97 (t, 2H), 7.02 (s, 1 H), 7.48 (s, 1 H), 7.58 (s, 1 H).
LRMS : m/z 262 (MH⁺)

### Preparation 103

### tert-Butyl (3E)-2-oxo-3-[(1-trityl-1H-imidazol-4-yl)methylene]-1-piperidinecarboxylate and tert-Butyl (3Z)-2-oxo-3-[(1-trityl-1H-imidazol-4-yl)methylene]-1-piperidinecarboxylate

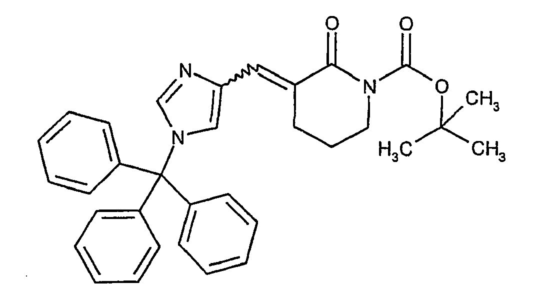

Triethylamine (2.78ml, 20.0mmol) and methanesulphonyl chloride (773µl, 10.0mmol) were added to an ice-cooled solution of the compound from preparation 100 (5.3g, 10.0mmol) in dichloromethane (50ml), and the reaction stirred at room temperature for 18 hours, and a further 4 hours at reflux. The cooled solution was concentrated under reduced pressure and the residue purified by column chromatography on silica gel using an elution gradient of toluene:ethyl acetate (100:0 to 20:80) to afford the title compound, 2.6g, 50% yield.
¹H-NMR (CDCl₃, 400MHz) δ (mixture of isomers): 1.54 (2xs, 9H), 1.85 (m, 2H), 3.00 (t, 2H), 3.68 (t, 2H), 6.99 (s, 1 H), 7.10 (m, 6H), 7.30 (m, 9H), 7.44 (s, 1 H), 7.58 (s, 1H).
LRMS : m/z 520.1 (MH⁺)
Anal. Found: C, 76.40; H, 6.51; N, 7.85. C₃₃H₃₃N₃O₃ reqires C, 76.28; H, 6.40; N, 8.09%.

### Preparation 104

### (2E)-2-{3[(tert-Butoxycarbonyl)amino]propyl}-3-(1-propyl-1H-imidazol-4-yl)-2-propenoic acid

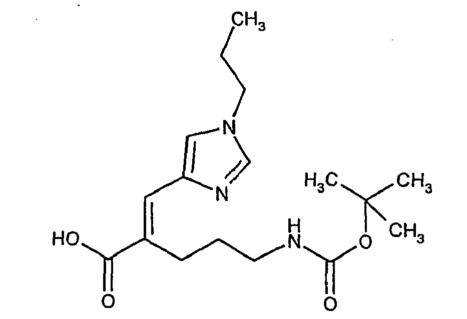

A solution of sodium hydroxide (171.3g, 4.28M) in water (4.55L) was added to a solution of the compound from preparation 53 (455g, 1.42M) in tetrahydrofuran (2.275L), and the reaction stirred at room temperature for 18 hours. The mixture was concentrated under reduced pressure to remove the tetrahydrofuran and the remaining aqueous solution was adjusted to pH 5 using glacial acetic acid. The resulting precipitate was granulated in an ice-bath for 1 hour, then filtered, washed with water and dried *in vacuo.* This solid was recrystallised from isopropanol and water to afford the title compound as a white solid, 304g, 63% yield.
¹H-NMR (DMSOd₆, 400MHz) δ: 0.81 (t, 3H), 1.38 (s, 9H), 1.56 (m, 2H), 1.74 (m, 2H), 2.75 (t, 2H), 2.93 (m, 2H), 3.95 (t, 2H), 6.97 (bs, 1 H), 7.37 (s, 1H), 7.52 (s, 1H), 7.76 (s, 1 H), 12.02 (bs, 1 H).

### Preparation 105

### (±)-tert-Butyl 3-[(1-isopentyl-1H-imidazol-4-yl)methyl]-2-oxo-1-piperidinecarboxylate

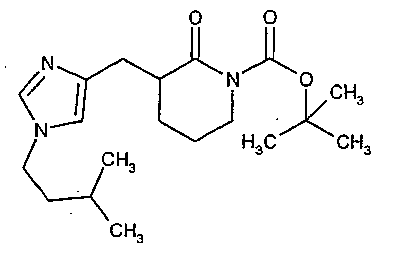

The alkene from preparation 101 (430mg, 1.25mmol), and 10% palladium on charcoal (Degussa® 101) (100mg) in ethanol (10ml) was hydrogenated at 60psi and room temperature for 18 hours. The mixture was filtered through Arbocel®, washing through with ethanol. The filtrate was concentrated under reduced pressure to give the title compound as a colourless oil, 420mg, 97% yield.
¹H-NMR (CDCl₃, 400MHz) δ: 0.89 (d, 6H), 1.50 (m, 10H), 1.62 (m, 4H), 1.78 (m, 1H), 1.98 (m, 1H), 2.63 (dd, 1H), 2.77 (m, 1H), 3.15 (dd, 1 H), 3.54 (m, 1H), 3.70 (m, 1H), 3.81 (t, 2H), 6.68 (s, 1H), 7.30 (s, 1H).
LRMS : m/z 350 (MH⁺)

### Preparation 106

### (±)-3-[(1-lsopentyl-1H-imidazol-4-yl)methyl]-1-methyl-2-piperidinone

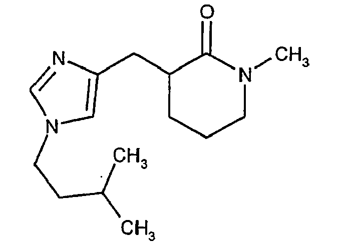

The title compound was obtained as a colourless oil in 24% yield, from the alkenes from preparation 102, following a similar procedure to that described in preparation 105, except the product was additionally purified by column chromatography on silica gel using an elution gradient of ethyl acetate:diethylamine:methanol (100:0:0 to 90:5:5).
¹H-NMR (CDCl₃, 400MHz) δ: 0.94 (d, 6H), 1.55 (m, 1H), 1.62 (m, 3H), 1.75 (m, 2H), 1.86 (m, 1 H), 2.60 (m, 1 H), 2.73 (dd, 1 H), 2.94 (s, 3H), 3.22 (m, 3H), 3.85 (t, 2H), 6.69 (s, 1H), 7.35 (s, 1H).
LRMS : m/z 264 (MH⁺)

### Preparation 107

### (±)-tert-Butyl 3-(1H-imidazol-4-ylmethyl)-2-oxo-1-piperidinecarboxylate

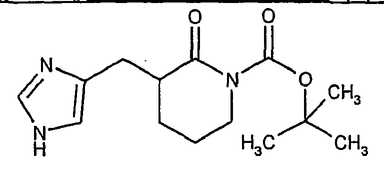

A mixture of the alkenes from preparation 103 (2.4g, 4.6mmol) and 10% palladium on charcoal (Degussa® 101) (200mg) in ethanol (400ml) was hydrogenated at 50°C and 60 psi for 18 hours. TLC analysis showed starting material remaining, so additional 10% palladium on charcoal (Degussa® 101) (100mg) was added, and the mixture hydrogenated for a further 72 hours. The mixture was filtered through Arbocel®, and the filtrate concentrated under reduced pressure. The crude product was purified by column chromatography on silica gel using an elution gradient of dichloromethane:ethyl acetate:methanol (100:0:0 to 0:100:0 to 0:90:10) to afford the title compound as a solid, 1.2g, 93% yield.
¹H-NMR (CDCl₃, 400MHz) δ: 1.46-1.62 (m, 10H), 1.81 (m, 2H), 1.98 (m, 1H), 2.66 (m, 1 H), 2.95 (m, 2H), 3.55 (m, 1 H), 3.78 (m, 1 H), 6.80 (s, 1 H), 7.24 (s, 1 H), 7.50 (s, 1 H).
LRMS : m/z 280 (MH⁺)

### Preparation 108

### (±)-tert-Butyl 2-oxo-3-[(1-phenyl-1H-imidazol-4-yl)methyl]-1-piperidinecarboxylate

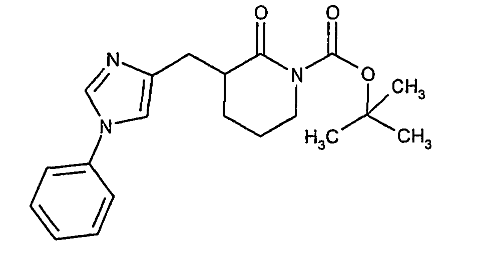

Phenylboronic acid (366mg, 3mmol), 4Å molecular sieves (1g), copper acetate (408mg, 2.25mmol) and pyridine (243µl, 3mmol) were added to a solution of the imidazole from preparation 107 (419mg, 1.5mmol) in dichloromethane (10ml), and the reaction mixture stirred at room temperature for 4 hours in the presence of a slow stream of compressed air. The air flow was then stopped, and the reaction was stirred for a further 18 hours at room temperature. A solution of ethylenediaminetetraacetic acid (2g) in aqueous sodium bicarbonate solution (10ml) was added, the mixture stirred for 10 minutes, then diluted with dichloromethane (100ml). The layers were separated, the organic phase dried (MgSO₄) and concentrated under reduced pressure. The residue was purified by column chromatography on silica gel using an elution gradient of ethyl acetate:pentane (50:50 to 80:20) to afford the title compound as a gum, 253mg, 47% yield.
¹H-NMR (CDCl₃, 400MHz) δ: 1.52 (s, 9H), 1.81 (m, 2H), 2.05 (m, 1H), 2.78-2.90 (m, 2H), 3.22 (dd, 1 H), 3.58 (m, 1 H), 3.77 (m, 2H), 7.11 (s, 1 H), 7.36 (m, 3H), 7.42 (m, 2H), 7.77 (s, 1 H).
LRMS : m/z 356.1 (MH⁺)

### Preparation 109

### (±)-5-[(tert-Butoxycarbonyl)amino]-2-[(1-propyl-1H-imidazol-4-yl)methyl]pentanoic acid

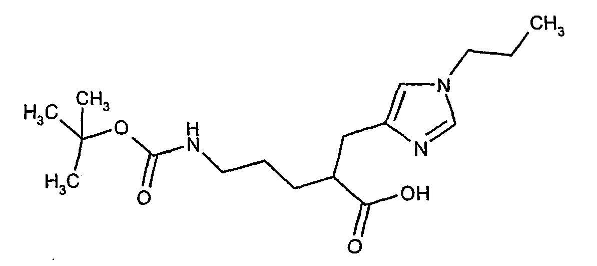

A mixture of the compound from preparation 104 (302g, 0.895M) and 5% palladium on charcoal (30g) in ethanol (3.0L) was hydrogenated at 60 psi and 60°C for 18 hours. The cooled reaction was filtered through Arbocel® and the filtrate evaporated under reduced pressure to give a colourless oil. This was crystallised from ethyl acetate and pentane, to afford the title compound as a white solid, 291.7g, 96% yield.
¹H-NMR (CDCl₃, 300MHz) δ: 0.90 (t, 3H), 1.42 (m, 10H), 1.58 (m, 2H), 1.66-1.86 (m, 3H), 2.70 (m, 1 H), 2.83 (d, 2H), 3.10 (m, 2H), 3.84 (t, 2H), 4.63 (bs, 1 H), 6.68 (s, 1 H), 7.49 (s, 1 H).

### Preparation 110

### (2S)-5-[(tert-Butoxvcarbonyl)amino]-2-[(1-propyl-1H-imidazol-4-yl)methyl]pentanoic acid with quinidine

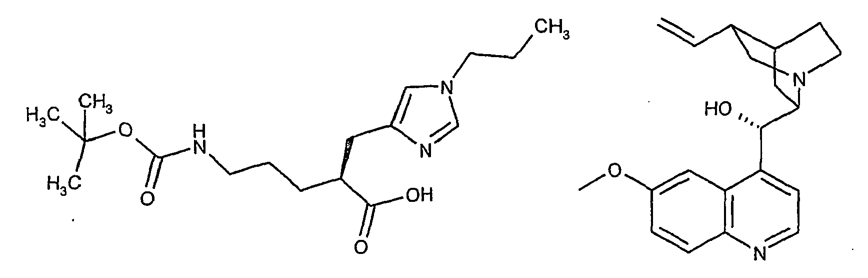

A mixture of the acid from preparation 104 (20g, 59mmol), quinidine (19.23g, 59mmol) and methanol (160ml) in a pressure vessel was purged with nitrogen, and then hydrogen to a pressure of 3 psi. The vessel was heated to 60°C, a solution of

[(*R*)-iPrFerroLANE Rh(COD)]BF₄ (Chirotech Technology Limited) (9.8mg, 0.012mmol) in deoxygenated methanol (1ml) was added, and the reaction mixture hydrogenated at 145 psi for 40 hours. The cooled solution was concentrated under reduced pressure and the crude product dissolved in ethyl acetate, with warming to 60°C. On cooling to room temperature with stirring, precipitation occurred, and the solid was filtered and dried *in vacuo* to afford the title compound, 29.8g, 76% yield (94% ee determined by CE).

### Alternative method of synthesis for title compound in preparation 110

A mixture of the acid from preparation 109 (50g, 147mmol) and quinidine (47.8g, 147mmol) in ethyl acetate (1.75L) was heated at 50°C on a steam bath, until a solution was obtained. The solution was warmed to 60°C, the heat removed and the solution allowed to cool, then stirred at room temperature for 18 hours. The resulting precipitate was filtered, washed with ethyl acetate and dried at 80°C *in vacuo* to afford the title compound as a white solid, 45.1g, 46% yield.
¹H-NMR (CD₃OD, 400MHz) δ: 0.83 (t, 3H), 1.10-1.20 (m, 1H), 1.40 (s, 9H), 1.45-1.62 (m, 5H), 1.65-1.80 (m, 4H), 1.88 (m, 1 H), 2.37 (m, 1H), 2.50-2.64 (m, 3H), 2.84 (m, 1H), 3.00-3.14 (m, 3H), 2.21 (m, 1H), 3.39 (m, 1H), 3.80 (m, 2H), 3.96 (m, 4H), 5.17-5.25 (m, 2H), 5.91 (m, 1H), 6.07-6.18 (m, 1 H), 6.89 (s, 1 H), 7.38 (d, 1 H), 7.43 (dd, 1H), 7.57 (s, 1 H), 7.76 (d, 1 H), 7.98 (d, 1 H), 8.72 (d, 1H).
LRMS : m/z 340 (MH⁺), 325 (quinidineH⁺)
Anal. Found: C, 65.82; H, 8.17; N, 10.32. C₃₇H₅₃N₅O₆•0.5H₂O requires 66.05; H, 8.09; N, 10.41%.
[α]_{D} = +121.36 (*c* 0.15, methanol)

## Claims

1. Compounds of formula (I) 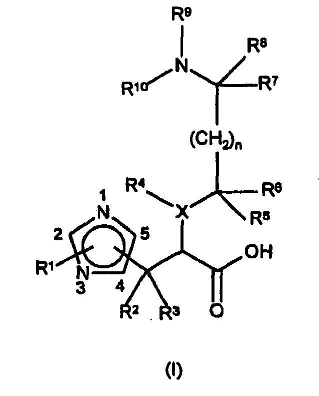 Where:
X is N or CH
n is 0 to 3
R¹ is:
a) C₁₋₆ alkyl, straight chain or branched chain,
b) C₁₋₆ alkenyl, straight chain or branched chain,
c) C₁₋₆ alkynyl, straight chain or branched chain,
d) Heterocycle,
e) Aromatic heterocycle,
f) Aryl;
g) hydrogen;
said groups (a), (b) and (c) optionally further substituted by: C₃₋₇ cycloalkyl, aryl, aromatic heterocycle, heterocycle, OR¹¹, NR¹¹R¹², S(O)ₚR¹¹, OC(O)R¹¹, CO₂R¹¹, CONR¹¹R¹², SO₂NR¹¹R¹², halo and NHSO₂R¹¹,
where R¹ may be attached at any position on the imidazole ring,
R² and R³ are each independently selected from hydrogen, C₁₋₆ alkyl, optionally further substituted by OR¹¹, halo; or
wherein R² and R³ may be joined to form a link, said link is C₂₋₆ alkylene,
R⁴ is hydrogen, C₁₋₆ alkyl, optionally further substituted by C₃₋₇ cycloalkyl, aryl, OR¹¹, halo and R¹¹; or
wherein R⁴ and R¹⁰ may be joined to form a link, wherein said link is C₁₋₄ alkylene, optionally further substituted by OR¹¹, halo and R¹¹,
R⁵ and R⁶ are selected from:
hydrogen, aryl, C₁₋₆ alkyl, said alkyl optionally further substituted by C₃₋₇ cycloalkyl, aromatic heterocycle, heterocycle, aryl, OR¹¹, R¹¹ and halo; or wherein R¹⁰ and either of R⁵ or R⁶ may be joined to form a link, wherein said link is a C₁₋₃ alkylene, optionally further substituted by OR¹¹, halo, R¹¹ and aryl; or
wherein R⁵ and R⁶ may be joined to form a link, wherein said link is C₂₋₆ alkylene,
R⁷ and R⁸ are independently selected from:
hydrogen, C₁₋₆ alkyl, optionally further substituted by OR¹¹, halo, aryl and R¹¹; or wherein R⁷ and R⁸ may be joined to form a link, wherein said link is C₂₋₆ alkylene,
R⁹ and R¹⁰ are independently selected from:
Hydrogen, C(NR¹¹)NR¹¹R¹², C₁₋₆ alkyl, said alkyl optionally substituted by OR¹¹, halo, aryl and R¹¹; or
wherein R⁹ and R¹⁰ may be joined to form a link, wherein said link is C₂₋₆ alkylene,
R¹¹ and R¹² are each independently selected from hydrogen or C₁₋₆ alkyl; or when forming a NR¹¹R¹² moiety, R¹¹ and R¹² may also be joined to form a link wherein said link is C₂₋₆ alkylene,
p is 0, 1 or 2
Wherein:
Aryl is defined as a 6-14 membered aromatic carbocycle, optionally further substituted by R¹¹, halo, OR¹¹, NR¹¹R¹², NR¹¹CO₂R¹², CO₂R¹¹, NR¹¹SO₂R¹², CN, haloalkyl, O(haloalkyl), S(O)ₚR¹¹, OC(O)R¹¹, SO₂NR¹¹R¹², C(O)NR¹¹R¹²,
Aromatic heterocycle is defined as a 5 to 7 membered ring, containing from 1 to 3 heteroatoms, each independently selected from O, S and N, said heterocycle group optionally substituted by OR¹¹, NR¹¹R¹², CO₂R¹¹, NR¹¹CO₂R¹², R¹¹, halo, CN, haloalkyl, O(haloalkyl), S(O)ₚR¹¹, OC(O)R¹¹, NR¹¹SO₂R¹², SO₂NR¹¹R¹², C(O)NR¹¹R¹²,
Heterocycle is defined as a 3-8 membered ring containing from 1-3 heteroatoms, each independently selected from O, S and N, said ring being saturated or partially saturated, said heterocycle group optionally substituted by OR¹¹, NR¹¹R¹², CO₂R¹¹, NR¹¹CO₂R¹², R¹¹, halo, CN, haloalkyl, O(haloalkyl), S(O)ₚR¹¹, OC(O)R¹¹, NR¹¹SO₂R¹², SO₂NR¹¹R¹², C(O)NR¹¹R¹²,
Or a pharmaceutically acceptable salt or solvate thereof.

2. A compound of formula (I) as claimed in claim 1 wherein the compound possesses the stereochemistry of a compound of formulae (IA) or (IB)

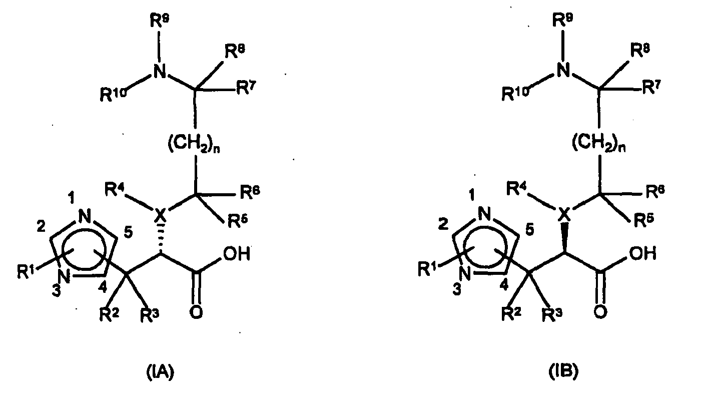

3. A compound of formula (IA) as claimed in claim 2.

4. A compound of formula (I) as claimed in any one of claims 1-3 wherein the imidazole is 1,4 disubstituted wherein the R¹ group is attached to N1.

5. A compound of formula (I) as claimed in any one of claims 1-3 wherein the imidazole is 2,4 disubstituted wherein the R¹ group is attached to C4.

6. A compound of formula (I) as claimed in any one of claims 1-5 wherein R¹ is an aryl group, C₁₋₆ alkenyl group or a C₁₋₆ alkyl group, wherein said alkyl group is optionally substituted by one or more groups selected from CO₂R¹¹, OR¹¹, aryl, C₃₋₇ cycloalkyl, NHSO₂R¹¹, halo and aromatic heterocycle.

7. A compound of formula (I) as claimed in any one of claims 1-6 wherein R¹ is C₁₋₃ alkyl.

8. A compound of formula (I) as claimed in any one of claims 1-7 wherein R² and R³ are hydrogen.

9. A compound of formula (I) as claimed in any one of claims 1-8 wherein R⁴ is independently selected from hydrogen and C₁₋₃ alkyl; or wherein R⁴ and R¹⁰ may be joined to form a link, said link is C₂₋₃ alkylene.

10. A compound of formula (I) as claimed in any one of claims 1-9 wherein R⁴ is hydrogen.

11. A compound of formula (I) as claimed in any one of claims 1-10 wherein R⁵ and R⁶ are preferably independently selected from hydrogen and C₁₋₆ alkyl, said alkyl group optionally substituted by phenyl; or wherein R⁵ and R¹⁰ may be joined to form a link, said link is C₁₋₃ alkylene.

12. A compound of formula (I) as claimed in any one of claims 1-11 wherein R⁵ and R⁶ are hydrogen.

13. A compound of formula (I) as claimed in any one of claims 1-12 wherein R⁷ and R⁸ are independently selected from hydrogen and C₁₋₆ alkyl.

14. A compound of formula (I) as claimed in any one of claims 1-13 wherein R⁷ and R⁸ are hydrogen.

15. A compound of formula (I) as claimed in any one of claims 1-14 wherein R⁹ and R¹⁰ are independently selected from hydrogen and C₁₋₃ alkyl; or wherein R¹⁰ and R⁴ may be joined to form a link, said link is C₂₋₃ alkylene.

16. A compound of formula (I) as claimed in any one of claims 1-15 wherein R⁹ and R¹⁰ are hydrogen.

17. A compound of formula (I) as claimed in any one of claims 1-16 wherein R¹¹ and R¹² are independently selected from hydrogen and C₁₋₆ alkyl.

18. A compound of formula (I) as claimed in any one of claims 1-17 wherein R¹¹ and R¹² are independently selected from hydrogen and CH₃.

19. A compound of formula (I) as claimed in any one of claims 1-18 wherein X is CH.

20. A compound of formula (I) as claimed in any one of claims 1-19 wherein n is 0.

21. A compound of formula (I) as claimed in any one of claims 1-20 wherein aryl is phenyl.

22. A compound of formula (I) as claimed in any one of claims 1-21 wherein Aromatic heterocycle is defined as a 5 to 6 membered ring, containing from 1 to 2 heteroatoms, each independently selected from O, S and N.

23. A compound as claimed in claim 1 which is (+)-(2*S*)-5-Amino-2-[(1-*n*-propyl-1*H*-imidazol-4-yl)methyl]pentanoic acid.

24. Compounds of formula (II) and (III) 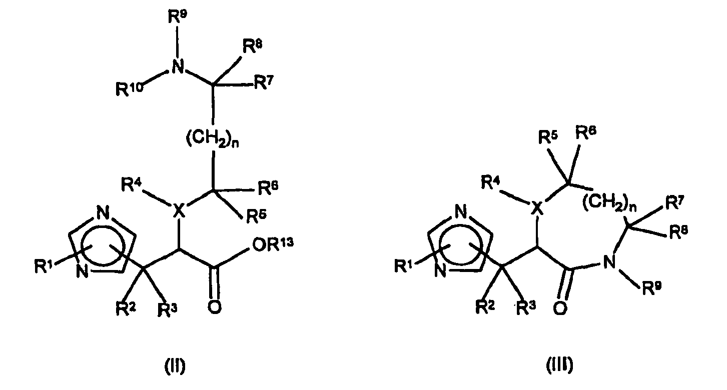 Wherein R¹, R², R³, R⁴, R⁵. R⁶, R⁷, R⁸ and X are as described in any one of claims 1-22, R⁹ and R¹⁰ are as described in any one of claims 1-22 or additionally one or both groups may be a suitable nitrogen protecting group and R¹³ is an appropriate oxygen protecting group.

25. Compounds of formula (XXIII) and (XXIV) 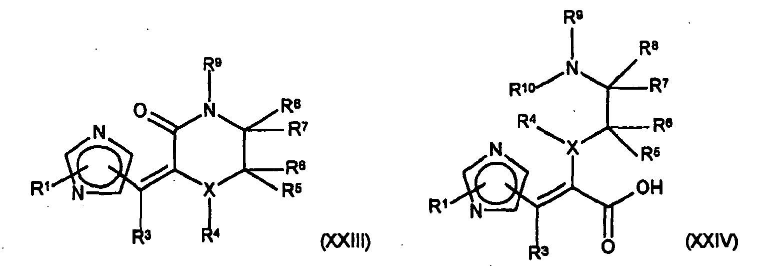 Where R¹, R³, R⁵, R⁶, R⁷, R⁸, R¹⁰ and Z are as described in any one of claims 1-22, R⁴ is hydrogen, X is CH and R⁹ is as described in any one of claims 1-22, or is an appropriate nitrogen protecting group.

26. A process for the preparation of a compound of formulae (IA) or (IB) according to any one of claims 2-22 which comprises the steps of
a) hydrolysis of a compound of formula (XXIII) 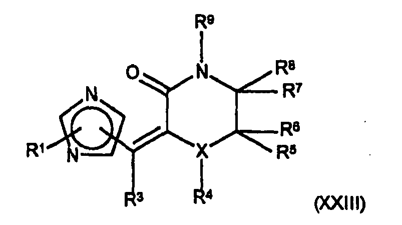 Where R¹, R³, R⁵, R⁶, R⁷ and R⁸ are as described in any one of claims 1-22, R⁴ is hydrogen, n is 0, X is CH and R⁹ is as described in any one of claims 1-22, or is an appropriate nitrogen protecting group;
To give a compound of formula (XXIV) 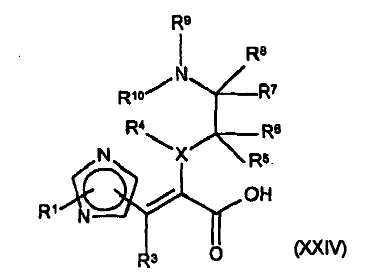 Wherein R¹, R³, R⁴, R⁵, R⁶, R⁷, R⁸, n, X and R⁹ are as hereinbefore defined and R¹⁰ is hydrogen;
b) hydrogenating the compound of formula (XXIV) so obtained; then
c) resolving the enantiomeric mix to give compounds of formulae (IA) and (IB); then
d) optionally removing the nitrogen protecting group when R⁹ is a nitrogen protecting group; and
e) optionally converting said compound of formulae (IA) or (IB) to a pharmaceutically acceptable salt thereof.

27. The process as claimed in claim 26 wherein said hydrogenation is an asymmetric hydrogenation.

28. A composition comprising a compound of formula (I) or a pharmaceutically acceptable salt or solvate thereof as claimed in any one of claims 1-23 and a pharmaceutically acceptable diluent or carrier.

29. A compound of formula (I) or a pharmaceutically acceptable salt or solvate thereof as claimed in any one of claims 1-23 for use as a medicament.

30. The use of a compound of formula (I) or a pharmaceutically acceptable salt or solvate thereof as claimed in any of claims 1-23 in the preparation of a medicament for the treatment or prevention of a condition selected from thrombosis, atherosclerosis, adhesions, dermal scarring, cancer, fibrotic conditions, inflammatory diseases and those conditions which benefit from maintaining or enhancing bradykinin levels in the body.

31. The use as claimed in claim 30 where the condition is a thrombotic condition selected from myocardial infarction, deep vein thrombosis, stroke, young stroke, cerebral infarction, cerebral thrombosis, cerebral embolism, peripheral vascular disease, angina and other forms of acute coronary syndromes, disseminating intravascular coagulation, sepsis, pulmonary embolism, embolic events secondary to cardiac arrhythmias and the prevention of cardiovascular events following surgical revascularisation or intervention.

32. The use as claimed in claim 30 wherein the condition is atherosclerosis.

33. The use as claimed in claim 30 wherein the condition is adhesions or dermal scarring.

34. The use as claimed in claim 30 wherein the condition is cancer.

35. The use as claimed in claim 30 wherein the condition is a fibrotic condition selected from cystic fibrosis, pulmonary fibrotic diseases, chronic obstructive pulmonary disease (COPD), adult respiratory distress syndrome (ARDS), fibromuscular dysplasia, fibrotic lung disease, fibrin deposits in the eye during opthalmic surgery and arthritis.

36. The use as claimed in claim 30 wherein the condition is an inflammatory disease selected from asthma, endometriosis, inflammatory bowel diseases, psoriasis and atopic dermatitis and neurodegenerative diseases, Alzheimers and Parkinsons.

37. The use as claimed in claim 30 wherein the condition is one which benefits from maintaining or enhancing bradykinin levels in the body selected from hypertension, angina, heart failure, pulmonary hypertension, renal failure and organ failure.

38. The use of a compound of formula (I) or a pharmaceutically acceptable salt or solvate thereof as claimed in any of claims 1-23 in the preparation of a medicament in combination with a antithrombotic for the treatment of thrombosis.

39. The use as claimed in claim 38 where the antithrombotic is a profibrinolytic.

40. The use as claimed in any one of claims 38 and 39 where the antithrombotic is recombinant tissue plasminogen activator (tPA).

41. The use of a compound of formula (I) or a pharmaceutically acceptable salt or solvate thereof as claimed in any one of claims 1-23 as a coating on intravascular devices.

42. An intravascular device control with a compound of formula (I) as claimed in any one of claims 1-23.

43. A kit comprising:
a) a composition comprising a compound of formula (I) or a pharmaceutically acceptable salt or solvate thereof as claimed in any one of claims 1-23 and a pharmaceutically acceptable diluent or carrier;
b) a composition comprising an antithrombotic and a pharmaceutically acceptable diluent or carrier; and
c) a container.

## Patentansprüche

1. Verbindungen der Formel (I) 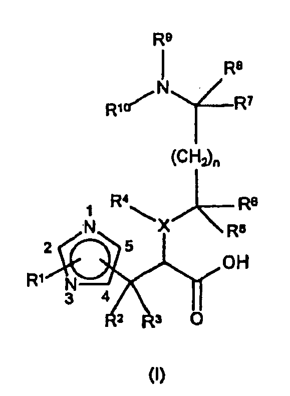 worin:
X N oder CH ist;
n 0 bis 3 ist;
R¹ ist:
a) C₁₋₆-Alkyl, geradkettig oder verzweigtkettig,
b) C₁₋₆-Alkenyl, geradkettig oder verzweigtkettig,
c) C₁₋₆-Alkinyl, geradkettig oder verzweigtkettig,
d) Heterocyclyl,
e) aromatisches Heterocyclyl,
f) Aryl,
g) Wasserstoff,
wobei die genannten Gruppen (a), (b) und (c) gegebenenfalls außerdem substituiert sind mit: C₃₋₇-Cycloalkyl, Aryl, aromatischem Heterocyclyl, Heterocyclyl, OR¹¹, NR¹¹R¹², S(O)ₚR¹¹, OC(O)R¹¹, CO₂R¹¹, CONR¹¹R¹², SO₂NR¹¹R¹², Halogen und NHSO₂R¹¹,
worin R¹ an einer beliebigen Position am Imidazolring gebunden sein kann,
R² und R³ unabhängig ausgewählt sind aus Wasserstoff, C₁₋₆-Alkyl, gegebenenfalls außerdem substituiert mit OR¹¹, Halogen; oder
worin R² und R³ unter Bildung einer Verknüpfung verbunden sein können, wobei die Verknüpfung C₂₋₆-Alkylen ist,
R⁴ Wasserstoff, C₁₋₆-Alkyl, gegebenenfalls außerdem substituiert mit C₃₋₇-Cycloalkyl, Aryl, OR¹¹, Halogen und R¹¹ ist oder worin R⁴ und R¹⁰ unter Bildung einer Verknüpfung verbunden sein können, wobei die Verknüpfung C₁₋₄-Alkylen, gegebenenfalls außerdem substituiert mit OR¹¹, Halogen und R¹¹, ist;
R⁵ und R⁶ ausgewählt sind aus:
Wasserstoff, Aryl, C₁₋₆-Alkyl, wobei das Alkyl gegebenenfalls außerdem substituiert ist mit C₃₋₇-Cycloalkyl, aromatischem Heterocyclyl, Heterocyclyl, Aryl, OR¹¹, R¹¹ und Halogen oder
wobei R¹⁰ und entweder R⁵ oder R⁶ unter Bildung einer Verknüpfung verbunden sein können, wobei die Verknüpfung ein C₁₋₃-Alkylen ist, das gegebenenfalls außerdem mit OR¹¹, Halogen, R¹¹ und Aryl substituiert ist, oder wobei R⁵ und R⁶ unter Bildung einer Verknüpfung verbunden sein können, wobei die Verknüpfung C₂₋₆-Alkylen ist;
R⁷ und R⁸ unabhängig ausgewählt sind aus:
Wasserstoff, C₁₋₆-Alkyl, gegebenenfalls weiter substituiert mit OR¹¹, Halogen, Aryl und R¹¹; oder wobei R⁷ und R⁸ unter Bildung einer Verknüpfung verbunden sein können, wobei die Verknüpfung C₂₋₆-Alkylen ist;
R⁹ und R¹⁰ unabhängig ausgewählt sind aus:
Wasserstoff, C(NR¹¹)NR¹¹R¹² C₁₋₆-Alkyl, wobei das Alkyl gegebenenfalls substituiert ist mit OR¹¹, Halogen, Aryl und R¹¹, oder
wobei R⁹ und R¹⁰ unter Bildung einer Verknüpfung verbunden sein können, wobei die Verknüpfung C₂₋₆-Alkylen ist;
R¹¹ und R¹² jeweils unabhängig ausgewählt sind aus Wasserstoff und C₁₋₆-Alkyl, oder, wenn sie eine NR¹¹R¹²-Gruppierung bilden, R¹¹ und R¹² auch unter Bildung einer Verknüpfung verbunden sein können, wobei die Verknüpfung C₂₋₆-Alkylen ist;
p 0, 1 oder 2 ist;
wobei:
Aryl als ein 6-14-gliedriger aromatischer Carbocyclus definiert ist, der gegebenenfalls weiter substituiert ist mit R¹¹, Halogen, OR¹¹, NR¹¹R¹², NR¹¹CO2R¹², CO₂R¹¹, NR¹¹SO₂R¹², CN, Halogenalkyl, O(Halogenalkyl), S(O)ₚR¹¹, OC(O)R¹¹, SO₂NR¹¹R¹², C(O)NR¹¹R¹²;
aromatischer Heterocyclus als ein 5-7-gliedriger Ring, der 1 bis 3 Heteroatome enthält, jedes unabhängig ausgewählt aus O, S und N, definiert ist, wobei die Heterocyclusgruppe bzw. Heterocyclylgruppe gegebenenfalls substituiert ist mit OR¹¹, NR¹¹R¹², CO₂R¹¹, NR¹¹COR¹², R¹¹, Halogen, CN, Halogenalkyl, O(Halogenalkyl), S(O)ₚR¹¹, OC(O)R¹¹, NR¹¹SO₂R¹², SO₂NR¹¹R¹², C(O)NR¹¹R¹²;
Heterocyclus als 3-8-gliedriger Ring, der 1 bis 3 Heteroatome, jeweils unabhängig ausgewählt aus 0, S und N, enthält, definiert ist, wobei der Ring gesättigt oder teilweise gesättigt ist, wobei die Heterocyclusgruppe bzw. Heterocyclylgruppe gegebenenfalls substituiert ist mit OR¹¹, NR¹¹R¹², CO₂R¹¹, NR¹¹CO₂R¹², R¹¹, Halogen, CN, Halogenalkyl, O(Halogenalkyl), S(O)ₚR¹¹, OC(O)R¹¹, NR¹¹SO₂R¹², SO₂NR¹¹R¹², C(O)NR¹¹R¹²;
oder ein pharmazeutisch verträgliches Salz oder Solvat davon.

2. Verbindung der Formel (I), wie sie in Anspruch 1 beansprucht ist, wobei die Verbindung die Stereochemie einer Verbindung der Formel (IA) oder (IB) besitzt:

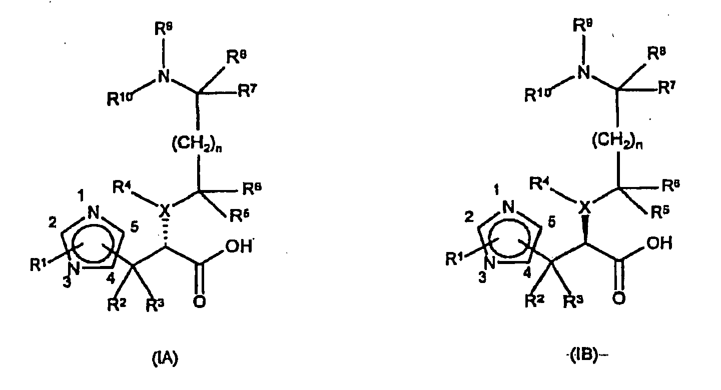

3. Verbindung der Formel (IA), wie sie in Anspruch 2 beansprucht ist.

4. Verbindung der Formel (I), wie sie in einem der Ansprüche 1-3 beansprucht ist, wobei das Imidazol 1,4-disubstituiert ist, wobei die R¹-Gruppe an N1 gebunden ist.

5. Verbindung der Formel (I), wie sie in einem der Ansprüche 1-3 beansprucht ist, wobei das Imidazol 2,4-disubstituiert ist, wobei die R¹-Gruppe an C4 gebunden ist.

6. Verbindung der Formel (I), wie sie in einem der Ansprüche 1-5 beansprucht ist, wobei R¹ eine Arylgruppe, C₁₋₆-Alkenylgruppe oder eine C₁₋₆-Alkylgruppe ist, wobei die Alkylgruppe gegebenenfalls mit einer oder mehr Gruppe(n), ausgewählt aus CO₂R¹¹, OR¹¹, Aryl, C₃₋₇-Cycloalkyl, NHSO₂R¹¹, Halogen und aromatischem Heterocyclyl, substituiert ist.

7. Verbindung der Formel (I), wie sie in einem der Ansprüche 1-6 beansprucht ist, wobei R¹ C₁₋₃-Alkyl ist.

8. Verbindung der Formel (I), wie sie in einem der Ansprüche 1-7 beansprucht ist, wobei R² und R³ Wasserstoff sind.

9. Verbindung der Formel (I), wie sie in einem der Ansprüche 1-8 beansprucht ist, wobei R⁴ unabhängig ausgewählt ist aus Wasserstoff und C₁₋₃-Alkyl oder wobei R⁴ und R¹⁰ unter Bildung einer Verknüpfung verbunden sein können, wobei die Verknüpfung C₂₋₃-Alkylen ist.

10. Verbindung der Formel (I), wie sie in einem der Ansprüche 1-9 beansprucht ist, wobei R⁴ Wasserstoff ist.

11. Verbindung der Formel (I), wie sie in einem der Ansprüche 1-10 beansprucht ist, wobei R⁵ und R⁶ vorzugsweise unabhängig ausgewählt sind aus Wasserstoff und C₁₋₆-Alkyl, wobei die Alkylgruppe gegebenenfalls mit Phenyl substituiert ist, oder wobei R⁵ und R¹⁰ unter Bildung einer Verknüpfung verbunden sein können, wobei die Verknüpfung C₁₋₃-Alkylen ist.

12. Verbindung der Formel (I), wie sie in einem der Ansprüche 1-11 beansprucht ist, wobei R⁵ und R⁶ Wasserstoff sind.

13. Verbindung der Formel (I), wie sie in einem der Ansprüche 1-12 beansprucht ist, wobei R⁷ und R⁸ unabhängig aus Wasserstoff und C₁₋₆-Alkyl ausgewählt sind.

14. Verbindung der Formel (I), wie sie in einem der Ansprüche 1-13 beansprucht ist, wobei R⁷ und R⁸ Wasserstoff sind.

15. Verbindung der Formel (I), wie sie in einem der Ansprüche 1-14 beansprucht ist, wobei R⁹ und R¹⁰ unabhängig ausgewählt sind aus Wasserstoff und C₁₋₃-Alkyl oder wobei R¹⁰ und R⁴ unter Bildung einer Verknüpfung verbunden sein können, wobei die Verknüpfung C₂₋₃-Alkylen ist.

16. Verbindung der Formel (I), wie sie in einem der Ansprüche 1-15 beansprucht ist, wobei R⁹ und R¹⁰ Wasserstoff sind.

17. Verbindung der Formel (I), wie sie in einem der Ansprüche 1-16 beansprucht ist, wobei R¹¹ und R¹² unabhängig ausgewählt sind aus Wasserstoff und C₁₋₆-Alkyl.

18. Verbindung der Formel (I), wie sie in einem der Ansprüche 1-17 beansprucht ist, wobei R¹¹ und R¹² unabhängig ausgewählt sind aus Wasserstoff und CH₃.

19. Verbindung der Formel (I), wie sie in einem der Ansprüche 1-18 beansprucht ist, wobei X CH ist.

20. Verbindung der Formel (I), wie sie in einem der Ansprüche 1-19 beansprucht ist, wobei n 0 ist.

21. Verbindung der Formel (I), wie sie in einem der Ansprüche 1-20 beansprucht ist, wobei Aryl Phenyl ist.

22. Verbindung der Formel (I), wie sie in einem der Ansprüche 1-21 beansprucht ist, wobei aromatischer Heterocyclus als ein 5-6-gliedriger Ring definiert ist, der 1 bis 2 Heteroatome, jeweils unabhängig ausgewählt aus 0, S und N, enthält, definiert ist.

23. Verbindung, wie sie in Anspruch 1 beansprucht ist, nämlich (+)-(2*S*)-5-Amino-2-[(1-*n*-propyl-1*H*-imidazol-4-yl)methyl]pentansäure.

24. Verbindungen der Formel (II) und (III): 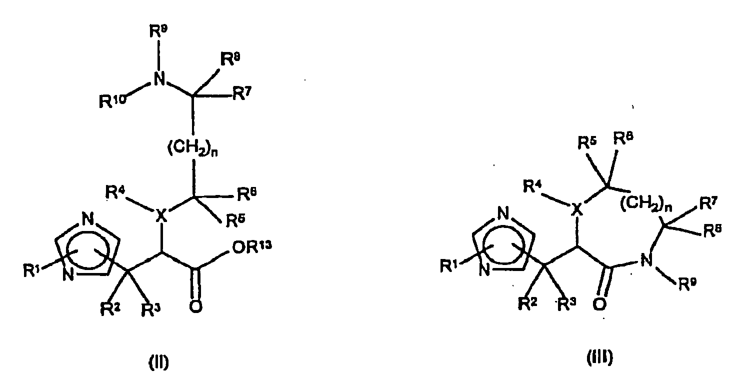 worin R¹, R², R³, R⁴, R⁵, R⁶, R⁷, R⁸ und X wie in einem der Ansprüche 1-22 beschrieben sind, R⁹ und R¹⁰ wie in einem der Ansprüche 1-22 beschrieben sind oder zusätzlich eine oder beide Gruppen eine geeignete Stickstoff-Schutzgruppe sein können und R¹³ eine geeignete Sauerstoff-Schutzgruppe ist.

25. Verbindungen der Formel (XXIII) und (XXIV): 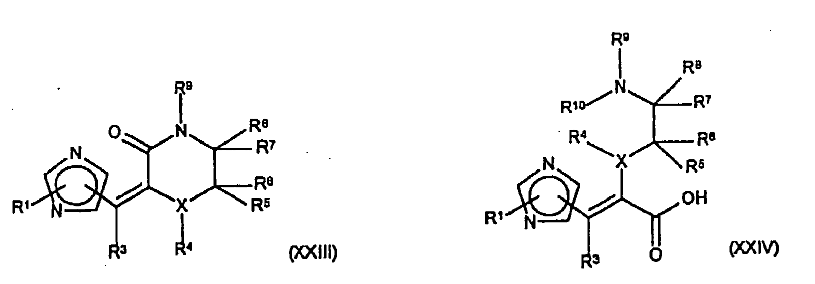 worin R¹, R³, R⁵, R⁶, R⁷, R⁸, R¹⁰ und Z wie in einem der Ansprüche 1-22 beschrieben sind, R⁴ Wasserstoff ist, X CH ist und R⁹ wie in einem der Ansprüche 1-22 beschrieben ist oder eine geeignete Stickstoff-Schutzgruppe ist.

26. Verfahren zur Herstellung einer Verbindung der Formel (IA) oder (IB) gemäß einem der Ansprüche 2-22, welches die Schritte:
a) Hydrolyse einer Verbindung der Formel (XXIII) 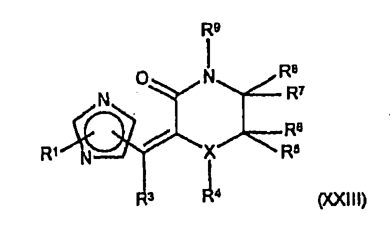 worin R¹, R³, R⁵, R⁶, R⁷ und R⁸ wie in einem der Ansprüche 1-22 beschrieben sind, R⁴ Wasserstoff ist, n 0 ist, X CH ist und R⁹ wie in einem der Ansprüche 1-22 beschrieben ist oder eine geeignete Stickstoff-Schutzgruppe ist;
unter Erhalt einer Verbindung der Formel (XXIV): 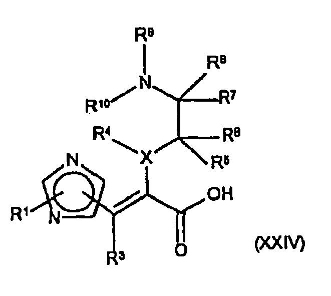 worin R¹, R³, R⁴, R⁵, R⁶, R⁷, R⁸, n, X und R⁹ wie vorher definiert sind und R¹⁰ Wasserstoff ist;
b) Hydrieren der so erhaltenen Verbindung der Formel (XXIV), danach
c) Trennen des Enantiomerengemisches unter Erhalt von Verbindungen der Formeln (IA) und (IB), danach
d) gegebenenfalls Entfernen der Stickstoff-Schutzgruppe, wenn R⁹ eine Stickstoff-Schutzgruppe ist, und
e) gegebenenfalls Umwandeln der Verbindung der Formel (IA) oder (IB) in ein pharmazeutisch verträgliches Salz davon, umfasst.

27. Verfahren, wie es in Anspruch 26 beansprucht ist, wobei die Hydrierung eine asymmetrische Hydrierung ist.

28. Zusammensetzung, umfassend eine Verbindung der Formel (I) oder ein pharmazeutisch verträgliches Salz oder Solvat davon, wie in einem der Ansprüche 1-23 beansprucht, und ein pharmazeutisch verträgliches Verdünnungsmittel oder einen pharmazeutisch verträglichen Träger.

29. Verbindung der Formel (I) oder ein pharmazeutisch verträgliches Salz oder Solvat davon, wie in einem der Ansprüche 1-23 beansprucht, zur Verwendung als Medikament.

30. Verwendung einer Verbindung der Formel (I) oder eines pharmazeutisch verträglichen Salzes oder Solvats davon, wie in einem der Ansprüche 1-23 beansprucht, bei der Herstellung eines Medikaments zur Behandlung oder Prävention eines Zustandes, ausgewählt aus Thrombose, Atherosklerose, Adhäsionen, Narbenbildung der Haut, Krebs, fibrotischen Zuständen, Entzündungserkrankungen und solchen Zuständen, für die eine Aufrechterhaltung oder Erhöhung der Bradykinin-Level im Körper von Nutzen ist.

31. Verwendung, wie sie in Anspruch 30 beansprucht ist, wobei der Zustand ein thrombotischer Zustand ist, ausgewählt aus Myokardinfarkt, Thrombose der tiefen Venen, Schlaganfall, Schlaganfall bei jungen Menschen, Hirninfarkt, cerebraler Thrombose, cerebraler Embolie, Erkrankung der peripheren Gefäße, Angina und anderen Formen akuter Koronarsyndrome, disseminierender intravaskulärer Koagulation, Sepsis, Lungenembolie, Embolieereignisse nach Herzrhythmusstörungen sowie die Prävention von kardiovaskulären Ereignissen nach chirurgischer Revaskularisierung oder chirurgischem Eingriff ist.

32. Verwendung, wie sie in Anspruch 30 beansprucht ist, wobei der Zustand Atherosklerose ist.

33. Verwendung, wie sie in Anspruch 30 beansprucht ist, wobei der Zustand Adhäsionen oder Narbenbildung der Haut ist.

34. Verwendung, wie sie in Anspruch 30 beansprucht ist, wobei der Zustand Krebs ist.

35. Verwendung, wie sie in Anspruch 30 beansprucht ist, wobei der Zustand ein fibrotischer Zustand ist, ausgewählt aus cystischer Fibrose, pulmonalen fibrotischen Erkrankungen, chronisch-obstruktiver Lungenerkrankung (COPD), Atemnotsyndrom bei Erwachsenen (ARDS), fibromuskulärer Dysplasie, fibrotischer Lungenerkrankung, Fibrinablagerungen im Auge während einer Augenoperation und Arthritis.

36. Verwendung, wie sie in Anspruch 30 beansprucht ist, wobei der Zustand eine inflammatorische Erkrankung ist, ausgewählt aus Asthma, Endometriose, entzündlichen Darmerkrankungen, Psoriasis und atopischer Dermatitis und neurodegenerativen Erkrankungen, Alzheimer'scher Erkrankung und Parkinson' scher Erkrankung.

37. Verwendung, wie sie in Anspruch 30 beansprucht ist, wobei der Zustand einer ist, der aus einer Aufrechterhaltung oder Erhöhung der Bradykinin-Level im Körper Nutzen zieht, ausgewählt aus Bluthochdruck, Angina, Herzversagen bzw. Herzinsuffizienz, Lungenhypertonie, Nierenversagen und Organversagen.

38. Verwendung einer Verbindung der Formel (I) oder eines pharmazeutisch verträglichen Salzes oder Solvats davon, wie in einem der Ansprüche 1-23 beansprucht, bei der Herstellung eines Medikaments in Kombination mit einem Antithrombotikum für die Behandlung von Thrombose.

39. Verwendung, wie sie in Anspruch 38 beansprucht ist, wobei das Antithrombotikum ein Profibrinolytikum ist.

40. Verwendung, wie sie in einem der Ansprüche 38 und 39 beansprucht ist, wobei das Antithrombotikum ein rekombinanter Gewebeplasminaktivator (tPA) ist.

41. Verwendung einer Verbindung der Formel (I) oder eines pharmazeutisch verträglichen Salzes oder Solvats davon als eine Beschichtung auf intravaskulären Vorrichtungen.

42. Intravaskuläre Vorrichtung, beschichtet mit einer Verbindung der Formel (I), wie sie in einem der Ansprüche 1-23 beansprucht ist.

43. Kit, umfassend:
a) eine Zusammensetzung, umfassend eine Verbindung der Formel (I) oder ein pharmazeutisch verträgliches Salz oder Solvat davon, wie in einem der Ansprüche 1-23 beansprucht, und ein pharmazeutisch verträgliches Verdünnungsmittel oder einen pharmazeutisch verträglichen Träger;
b) eine Zusammensetzung, umfassend ein Antithrombotikum und ein pharmazeutisch verträgliches Verdünnungsmittel oder einen pharmazeutisch verträglichen Träger und
c) einen Behälter.

## Revendications

1. Composés de formule (I) 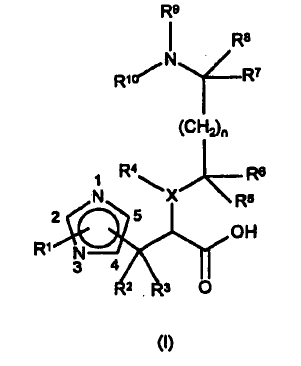 dans laquelle :
X représente N ou un groupe CH
n a une valeur de 0 à 3
R¹ représente :
a) un groupe alkyle en C₁ à C₆, à chaîne droite ou à chaîne ramifiée,
b) un groupe alcényle en C₁ à C₆, à chaîne droite ou à chaîne ramifiée,
c) un groupe alcényle en C₁ à C₆, à chaîne droite ou à chaîne ramifiée,
d) un hétérocycle,
e) un hétérocycle aromatique,
f) un groupe aryle,
g) un atome d'hydrogène ;
lesdits groupes (a), (b) et (c) étant en outre facultativement substitués avec des substituants : cycloalkyle en C₃ à C₇, aryle, hétérocycle aromatique, hétérocycle, OR¹¹, NR¹¹R¹², S(O)ₚR¹¹, OC(O)R¹¹, CO₂R¹¹, CONR¹¹R¹², SO₂NR¹¹R¹², halogéno et NHSO₂R¹¹,
où R¹ peut être fixé à n'importe quelle position sur le noyau imidazole,
R² et R³ sont chacun choisis indépendamment entre un atome d'hydrogène, un groupe alkyle en C₁ à C₆, en outre facultativement substitué avec un substituant OR¹¹, un groupe halogéno ; ou bien
R² et R³ peuvent être joints pour former une liaison, ladite liaison étant une liaison alkylène en C₂ à C₆,
R⁴ représente un atome d'hydrogène, un groupe alkyle en C₁ à C₆ en outre facultativement substitué avec un substituant cycloalkyle en C₃ à C₇, un groupe aryle, OR¹¹, halogéno ou R¹¹ ; ou
R⁴ et R¹⁰ peuvent être joints pour former une liaison, ladite liaison étant une liaison alkylène en C₁ à C₄, facultativement substituée en outre avec un substituant OR¹¹, halogéno ou R¹¹,
R⁵ et R⁶ sont choisis entre :
un atome d'hydrogène, des groupes aryle, alkyle en C₁ à C₆, lesdits groupes alkyle étant en outre facultativement substitués avec un substituant cycloalkyle en C₃ à C₇, hétérocycle aromatique, hétérocycle, aryle, OR¹¹, R¹¹ ou halogéno ; ou bien
R¹⁰ et R⁵ ou bien R⁶ peuvent être joints pour former une liaison, ladite liaison étant une liaison alkylène en C₁ à C₃, en outre facultativement substituée avec un substituant OR¹¹, halogéno, R¹¹ ou aryle ; ou bien
R⁵ et R⁶ peuvent être joints pour former une liaison, ladite liaison étant une liaison alkylène en C₂ à C₆,
R⁷ et R⁸ sont choisis indépendamment entre :
un atome d'hydrogène, des groupes alkyle en C₁ à C₆, en outre facultativement substitués avec un substituant OR¹¹, halogéno, aryle ou R¹¹ ; ou bien
R⁷ et R⁸ peuvent être joints pour former une liaison, ladite liaison étant une liaison alkylène en C₂ à C₆,
R⁹ et R¹⁰ sont choisis indépendamment entre :
un atome d'hydrogène, des groupes C(NR¹¹)NR¹¹R¹², alkyle en C₁ à C₆, ledit groupe alkyle étant facultativement substitué avec un substituant OR¹¹, halogéno, aryle ou R¹¹ ; ou bien
R⁹ et R¹⁰ peuvent être joints pour former une liaison, ladite liaison étant une liaison alkylène en C₂ à C₆,
R¹¹ et R¹² sont choisis chacun indépendamment entre un atome d'hydrogène et un groupe alkyle en C₁ à C₆ ; ou bien, lorsqu'ils forment un groupement NR¹¹R¹², R¹¹ et R¹² peuvent également être joints pour former une liaison, ladite liaison étant une liaison alkylène en C₂ à C₆,
p est égal à 0, 1 ou 2
où :
le terme "aryle" désigne un carbocycle aromatique de 6 à 14 membres, en outre facultativement substitué avec un substituant R¹¹, halogéno, OR¹¹, NR¹¹R¹², NR¹¹CO₂R¹², CO₂R¹¹, NR¹¹SO₂R¹², CN, halogénalkyle, O(halogénalkyle), S(O)ₚR¹¹, OC(O)R¹¹, SO₂NR¹¹R¹²_{,} C(O)NR¹¹R¹²,
l'hétérocycle aromatique est défini en tant qu'un noyau penta- à heptagonal, contenant 1 à 3 hétéroatomes, choisis chacun indépendamment entre O, S et N, ledit groupe hétérocyclique étant facultativement substitué avec un substituant OR¹¹, NR¹¹R¹², CO²R¹¹, NR¹¹CO₂R¹², R¹¹, halogéno, CN, halogénalkyle, O(halogénalkyle), S(O)ₚR¹¹, OC(O)R¹¹, NR¹¹SO₂R¹², SO₂NR¹¹R¹², C(O)NR¹¹R¹²,
le terme "hétérocycle" désigne un noyau tri- à octogonal contenant 1 à 3 hétéroatomes, choisis chacun indépendamment entre O, S et N, ledit noyau étant saturé ou partiellement insaturé, ledit groupe hétérocyclique étant facultativement substitué avec un substituant OR¹¹, NR¹¹R¹², CO₂R¹¹_{,} NR¹¹CO₂R¹², R¹¹, halogéno, CN, halogénalkyle, O (halogénalkyle), S(O)ₚR¹¹, OC(O)R¹¹, NR¹¹SO₂R¹², SO₂NR¹¹R¹², C(O)NR¹¹R¹²,
ou un de leurs sels ou produits de solvatation pharmaceutiquement acceptables.

2. Composé de formule (I) suivant la revendication 1, le composé possédant la stéréochimie d'un composé de formule (IA) ou (IB)

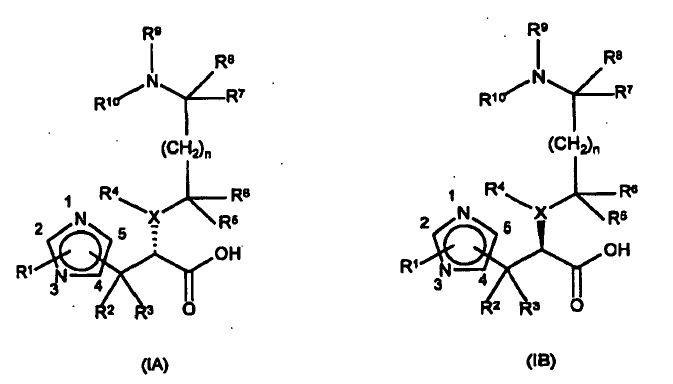

3. Composé de formule (IA), suivant la revendication 2.

4. Composé de formule (I) suivant l'une quelconque des revendications 1 à 3, dans lequel l'imidazole est 1,4-disubstitué, le groupe R¹ étant fixé à N1.

5. Composé de formule (I) suivant l'une quelconque des revendications 1 à 3, dans lequel l'imidazole est 1,4-disubstitué, le groupe R¹ étant fixé à C4.

6. Composé de formule (I) suivant l'une quelconque des revendications 1 à 5, dans lequel R¹ représente un groupe aryle, un groupe alcényle en C₁ à C₆ ou un groupe alkyle en C₁ à C₆, ledit groupe alkyle étant facultativement substitué avec un ou plusieurs groupes choisis entre des groupes CO₂R¹¹, OR¹¹, aryle, cycloalkyle en C₃ à C₇, NHSO₂R¹¹, halogéno et hétérocyclique aromatique.

7. Composé de formule (I) suivant l'une quelconque des revendications 1 à 6, dans lequel R¹ représente un groupe alkyle en C₁ à C₃.

8. Composé de formule (I) suivant l'une quelconque des revendications 1 à 7, dans lequel R² et R³ représentent des atomes d'hydrogène.

9. Composé de formule (I) suivant l'une quelconque des revendications 1 à 8, dans lequel R⁴ est choisi indépendamment entre un atome d'hydrogène et un groupe alkyle en C₁ à C₃ ; ou bien dans lequel R⁴ et R¹⁰ peuvent être joints pour former une liaison, ladite liaison étant une liaison alkylène en C₂ ou C₃.

10. Composé de formule (I) suivant l'une quelconque des revendications 1 à 9, dans lequel R⁴ représente un atome d'hydrogène.

11. Composé de formule (I) suivant l'une quelconque des revendications 1 à 10, dans lequel R⁵ et R⁶ sont choisis de préférence indépendamment entre un atome d'hydrogène et un groupe alkyle en C₁ à C₆, ledit groupe alkyle étant facultativement substitué avec un substituant phényle ; ou dans lequel R⁵ et R¹⁰ peuvent être joints pour former une liaison, ladite liaison étant une liaison alkylène en C₂ ou C₃.

12. Composé de formule (I) suivant l'une quelconque des revendications 1 à 11, dans lequel R⁵ et R⁶ représentent des atomes d'hydrogène.

13. Composé de formule (I) suivant l'une quelconque des revendications 1 à 12, dans lequel R⁷ et R⁸ sont choisis indépendamment entre un atome d'hydrogène et un groupe alkyle en C₁ à C₆.

14. Composé de formule (I) suivant l'une quelconque des revendications 1 à 13, dans lequel R⁷ et R⁸ représentent des atomes d'hydrogène.

15. Composé de formule (I) suivant l'une quelconque des revendications 1 à 14, dans lequel R⁹ et R¹⁰ sont choisis indépendamment entre un atome d'hydrogène et un groupe alkyle en C₁ à C₃ ; ou bien dans lequel R¹⁰ et R⁴ peuvent être joints pour former une liaison, ladite liaison étant une liaison alkylène en C₂ ou C₃.

16. Composé de formule (I) suivant l'une quelconque des revendications 1 à 15, dans lequel R⁹ et R¹⁰ représentent des atomes d'hydrogène.

17. Composé de formule (I) suivant l'une quelconque des revendications 1 à 16, dans lequel R¹¹ et R¹² sont choisis indépendamment entre un atome d'hydrogène et un groupe alkyle en C₁ à C₆.

18. Composé de formule (I) suivant l'une quelconque des revendications 1 à 17, dans lequel R¹¹ et R¹² sont choisis indépendamment entre un atome d'hydrogène et un groupe CH₃.

19. Composé de formule (I) suivant l'une quelconque des revendications 1 à 18, dans lequel X représente un groupe CH.

20. Composé de formule (I) suivant l'une quelconque des revendications 1 à 19, dans lequel n est égal à 0.

21. Composé de formule (I) suivant l'une quelconque des revendications 1 à 20, dans lequel le groupe aryle est un groupe phényle.

22. Composé de formule (I) suivant l'une quelconque des revendications 1 à 21, dans lequel l'hétérocycle aromatique est défini en tant que noyau penta- ou hexagonal, contenant 1 ou 2 hétéroatomes, choisis chacun indépendamment entre O, S et N.

23. Composé suivant la revendication 1, qui est l'acide (+)-(2S)-5-amino-2-[(1-*n*-propyl-1*H*-imidazole-4-yl)-méthyl]pentanoïque.

24. Composés de formules (II) et (III) 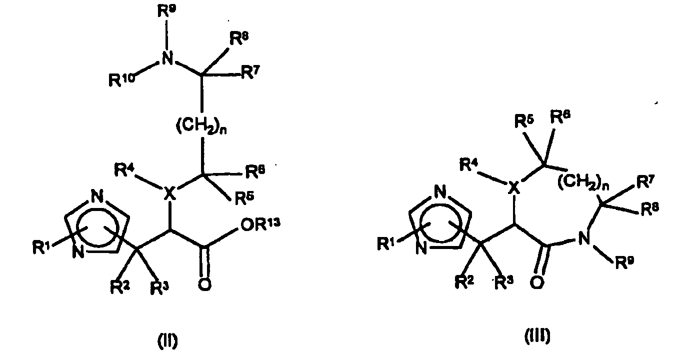 dans lesquelles R¹, R², R³, R⁴, R⁵, R⁶, R⁷, R⁸ et X sont tels que définis dans l'une quelconque des revendications 1 à 22, R⁹ et R¹⁰ sont tels que définis dans l'une quelconque des revendications 1 à 22, ou bien, en outre, l'un des ou les deux groupes peuvent représenter un groupe convenable protecteur de l'atome d'azote et R¹³ représente un groupe approprié protecteur de l'atome d'oxygène.

25. Composés de formule (XXIII) et (XXIV) 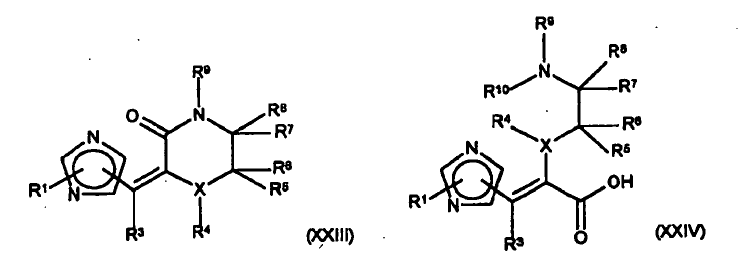 dans lesquelles R¹, R³, R⁵, R⁶, R⁷, R⁸, R¹⁰ et Z sont tels que définis dans l'une quelconque des revendications 1 à 22, R⁴ représente un atome d'hydrogène, X représente un groupe CH et R⁹ est tel que défini dans l'une quelconque des revendications 1 à 22, ou bien représente un groupe approprié protecteur de l'atome d'azote.

26. Procédé pour la préparation d'un composé de formule (IA) ou (IB) suivant l'une quelconque des revendications 2 à 22, qui comprend les étapes suivantes
a) hydrolyse d'un composé de formule (XXIII) 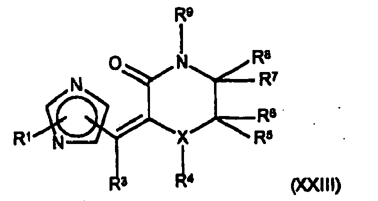 dans laquelle R¹, R³, R⁵, R⁶, R⁷ et R⁸ sont tels que définis dans l'une quelconque des revendications 1 à 22, R⁴ représente un atome d'hydrogène, n est égal à 0, X représente un groupe CH et R⁹ est tel que défini dans l'une quelconque des revendications 1 à 22, ou bien représente un groupe approprié protecteur de l'atome d'azote ;
pour donner un composé de formule (XXIV) 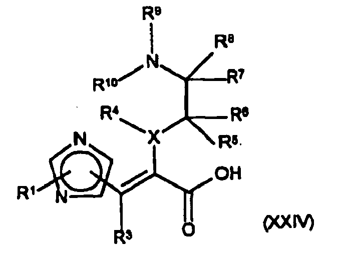 dans laquelle R¹, R³, R⁴, R⁵, R⁶, R⁷, R⁸, n, X et R⁹ répondent aux définitions précitées et R¹⁰ représente un atome d'hydrogène ;
b) hydrogénation du composé de formule (XXIV) ainsi obtenu ; puis
c) résolution du mélange énantiomère, pour donner des composés de formules (IA) et (IB) ; puis
d) facultativement élimination du groupe protecteur de l'atome d'azote lorsque R⁹ représente un groupe protecteur de l'atome d'azote ; et
e) facultativement conversion dudit composé de formule (IA) ou (IB) en un de ses sels pharmaceutiquement acceptables.

27. Procédé suivant la revendication 26, dans lequel ladite hydrogénation est une hydrogénation asymétrique.

28. Composition comprenant un composé de formule (I) ou un de ses sels ou produits de solvatation pharmaceutiquement acceptables suivant l'une quelconque des revendications 1 à 23 et un diluant ou support pharmaceutiquement acceptable.

29. Composé de formule (I) ou un de ses sels ou produits de solvatation pharmaceutiquement acceptables suivant l'une quelconque des revendications 1 à 23, destiné à être utilisé comme médicament.

30. Utilisation d'un composé de formule (I) ou d'un de ses sels ou produits de solvatation pharmaceutiquement acceptables suivant l'une quelconque des revendications 1 à 23 dans la préparation d'un médicament destiné au traitement ou à la prévention d'une affection choisie entre la thrombose, l'athérosclérose, des adhérences, la cicatrisation dermique, le cancer, des états fibrotiques, des maladies inflammatoires et les affections qui bénéficient du maintien ou de l'augmentation des taux de bradykinine dans l'organisme.

31. Utilisation suivant la revendication 30, dans laquelle l'affection est une affection thrombotique choisie entre l'infarctus du myocarde, la thrombose veineuse profonde, un ictus, un ictus jeune, l'infarctus cérébral, la thrombose cérébrale, l'embolie cérébrale, une maladie vasculaire périphérique, l'angine de poitrine et d'autres formes de syndromes coronariens aigus, la coagulation intravasculaire disséminée, la septicémie, l'embolie pulmonaire, des accidents emboliques secondaires à des arythmies cardiaques et la prévention d'accidents cardiovasculaires après une revascularisation ou intervention chirurgicale.

32. Utilisation suivant la revendication 30, dans laquelle l'affection est l'athérosclérose.

33. Utilisation suivant la revendication 30, dans laquelle l'affection consiste en des adhérences ou la cicatrisation dermique.

34. Utilisation suivant la revendication 30, dans laquelle l'affection est le cancer.

35. Utilisation suivant la revendication 30, dans laquelle l'affection est une affection fibrotique choisie entre la fibrose kystique, des maladies fibrotiques pulmonaires, la maladie pulmonaire obstructive chronique (COPD), le syndrome de détresse respiratoire de l'adulte (ARDS), la dysplasie fibromusculaire, une maladie pulmonaire fibrotique, les dépôts de fibrine dans l'oeil au cours d'une intervention chirurgicale ophtalmique et l'arthrite.

36. Utilisation suivant la revendication 30, dans laquelle l'affection est une maladie inflammatoire choisie entre l'asthme, l'endométriose, des maladies intestinales inflammatoires, le psoriasis et la dermatite atopique et des maladies neurodégénératives, la maladie d'Alzheimer et la maladie de Parkinson.

37. Utilisation suivant la revendication 30, dans laquelle l'affection est une affection qui bénéficie du maintien ou de l'augmentation des taux de bradykinine dans l'organisme, choisie entre l'hypertension, l'angine de poitrine, l'insuffisance cardiaque, l'hypertension pulmonaire, l'insuffisance rénale et une insuffisance d'organe.

38. Utilisation d'un composé de formule (I) ou d'un de ses sels ou produits de solvatation pharmaceutiquement acceptables suivant l'une quelconque des revendications 1 à 23, dans la préparation d'un médicament en association avec un agent antithrombotique pour le traitement de la thrombose.

39. Utilisation suivant la revendication 38, dans laquelle l'agent antithrombotique est un agent profibrinolytique.

40. Utilisation suivant l'une quelconque des revendications 38 et 39, dans laquelle l'agent antithrombotique est l'activateur de plasminogène tissulaire (tPA) recombinant.

41. Utilisation d'un composé de formule (I) ou d'un de ses sels ou produits de solvatation pharmaceutiquement acceptables suivant l'une quelconque des revendications 1 à 23 comme revêtement sur des dispositifs intravasculaires.

42. Dispositif intravasculaire revêtu d'un composé de formule (I) suivant l'une quelconque des revendications 1 à 23.

43. Kit comprenant :
a) une composition comprenant un composé de formule (I) ou d'un de ses sels ou produits de solvatation pharmaceutiquement acceptables suivant l'une quelconque des revendications 1 à 23 et un diluant ou support pharmaceutiquement acceptable ;
b) une composition comprenant un agent antithrombotique et un diluant ou support pharmaceutiquement acceptable ; et
c) un récipient.
